# EUROPEAN PATENT SPECIFICATION

(11) **EP 4 591 782 B1**
(45) Date of publication and mention of the grant of the patent: **18.11.2026**
(21) Application number: 25181301.0
(22) Date of filing: 14.07.2022
(51) Int. Cl.: A61M 25/01, A61B 1/005

(54) **STEERABLE INSTRUMENT FOR ENDOSCOPIC OR INVASIVE APPLICATIONS**
LENKBARES INSTRUMENT FÜR ENDOSKOPISCHE ODER INVASIVE ANWENDUNGEN
INSTRUMENT ORIENTABLE POUR APPLICATIONS ENDOSCOPIQUES OU INVASIVES

(30) Priority: 15.07.2021 NL 2028747
(43) Date of publication of application: 30.07.2025
(62) Document number(s) of the earlier application(s) in accordance with Art. 76 EPC: 22744323.1 / 4 370 187
(73) Proprietor: Fortimedix Assets II B.V., 6167 RD Geleen (NL)
(72) Inventor: THISSEN, Mattheus Hendrik Louis, 6071 NC Swalmen (NL); VERBEEK, Marcel Antonius Elisabeth, 6367 HA Voerendaal (NL)
(74) Representative: Nederlandsch Octrooibureau

(56) References cited:
- US-A1- 2010 228 191
- US-A1- 2012 116 163
- US-A1- 2012 323 077
- US-A1- 2014 018 620
- US-A1- 2015 107 396
- US-A1- 2020 129 166

## Description

### Field of the invention

The present invention relates to a steerable instrument for endoscopic and/or invasive type of applications, such as in surgery. The steerable instrument according to the invention can be used in both medical and non-medical applications. Examples of the latter include inspection and/or repair of mechanical and/or electronic hardware at locations that are difficult to reach. Hence, terms used in the following description such as endoscopic application or invasive instrument, must be interpreted in a broad manner.

### Background art

Transformation of surgical interventions that require large incisions for exposing a target area into minimal invasive surgical interventions, i.e. requiring only natural orifices or small incisions for establishing access to the target area, is a well-known and ongoing process. In performing minimal invasive surgical interventions, an operator such as a physician, requires an access port that is arranged for introducing and locating invasive instruments into the human or animal body. In order to reduce scar tissue formation and pain to a human or animal patient, the access port is preferably provided by a single small incision in the skin and underlying tissue. In some applications, a natural orifice of the body can be used as an entrance.

Surgical invasive instruments and endoscopes are well-known in the art. Both the invasive instruments and endoscopes can comprise a steerable tube that enhances its navigation and steering capabilities. Such a steerable tube may comprise a proximal end part including at least one flexible zone, a distal end part including at least one flexible zone, and an intermediate part, wherein the steerable tube further comprises a steering arrangement that is adapted for translating a deflection of at least a part of the proximal end part relative to the intermediate part into a related deflection of at least a part of the distal end part. Alternatively, the distal flexible zone may be steered by a robotic instrument arranged at the proximal end of the steerable instrument.

Steerable invasive instruments may comprise a handle that is arranged at the proximal end part of the steerable tube for steering the tube and/or for manipulating a tool that is arranged at the distal end part of the steerable tube. Such a tool can for example be a camera, a manual manipulator, e.g. a pair of scissors, forceps, or manipulators using an energy source, e.g. an electrical, ultrasonic or optical energy source.

Furthermore, such a steerable tube may comprise a number of co-axially arranged cylindrical elements including an outer cylindrical element, an inner cylindrical element and one or more intermediate cylindrical elements merely depending on the number of flexible zones in the proximal and distal end parts of the tube and the desired implementation of the steering members of the steering arrangement, i.e. all steering members can be arranged in a single intermediate cylindrical element or the steering members are divided in different sets and each set of steering members is arranged, at least in part, in a different or the same intermediate cylindrical element. In most prior art devices, the steering arrangement comprises conventional steering cables with, for instance, sub 1 mm diameters as steering members, wherein the steering cables are arranged between related flexible zones at the proximal and distal end parts of the tube. Other steering units at the proximal end, like ball shaped steering units or robot driven steering units, may be applied instead.

However, as steering cables have many well-known disadvantages, for some applications one may want to avoid them and to implement the steering members by one or more sets of steering wires that form integral parts of the one or more intermediate cylindrical elements. Each of the intermediate cylindrical elements including the steering wires can be fabricated either by using a suitable material addition technique, such as injection molding or plating, or by starting from a tube and then using a suitable material removal technique, such as laser cutting, photochemical etching, deep pressing, conventional chipping techniques such as drilling or milling or high-pressure water jet cutting systems. Steering wires manufactured in that way are, then, implemented as longitudinal strips resulting from the tube material, and can be used as pulling/pushing wires. Of the aforementioned material removal techniques, laser cutting is very advantageous as it allows a very accurate and clean removal of material under reasonable economic conditions. The embodiments of the present invention may be manufactured using this technique.

The inner and outer cylindrical elements may be manufactured from tubes too. These tubes should be flexible at locations where the distal end, and possibly the proximal end too, of the instrument is bendable. Also at other locations where the instrument should be flexible, the inner and outer cylindrical elements should be flexible. This can be implemented by providing the inner and outer cylindrical elements with hinges at these flexible locations. Such hinges may result from (laser) cutting predetermined patterns in the tube. Many different patterns are known from the prior art. Which pattern to use depends on design requirements at the location concerned including but not limited to the required bending angle, bending flexibility, longitudinal stiffness, and radial stiffness.

Further details regarding the design and fabrication of the abovementioned steerable tube and the steering arrangement thereof have been described for example in WO 2009/112060 A1, WO 2009/127236 A1, US 13/160,949, and US 13/548,935 of the applicant.

As is known from for example a flexible endoscopic instrument with a steerable tip, flexible invasive steerable instruments can show performance flaws with respect to steerable tip control. When such a flexible instrument is inserted into a body through a curved channel, either an endoscope or a natural body lumen, bending of the instrument causes displacement of the longitudinal tip steering elements. Because in conventionally built instruments the steering elements, e.g. wires, are fixed to a steering device, like a handle, at the proximal side and to the steerable tip at the distal side, movement of the steering wires will result in deflection of the steering device and or deflection of the steerable tip. This causes the problem that when the instrument is advanced through a narrow curved channel, and when one holds the steering device in a fixed position, the tip will deflect uncontrollable during advancement and can either lock up in, for example, a narrow endoscope working channel or it can damage tissue in for example a soft tissue natural body lumen like the lung bronchi or the esophagus.

Another problem is that when the instrument passed the entrance channel and the instrument tip reached the targeted operation site, the tip deflection does not match the steering device deflection anymore. So a neutral position of the steering device does not result in a neutral position of the steerable tip. This offset does adversely affect eye-hand coordination of the user.

A partial solution to this problem that addresses the problem of unwanted tip steering due to bending of the instrument body is described in WO2014/011049. This solution describes an instrument in which the steering wires can be de-coupled from the steering device and the ends of these steering wires and hence the instrument tip can move freely when the instrument is advanced through a curved entrance path. Once the instrument tip passed the entrance channel and is at the targeted operation site, the steering wires are re-coupled to the steering device and the instrument tip can now be steered. The disadvantages of this solution are that the instrument is mechanically more complex and requires more parts to build. Another disadvantage is that the operator has to follow a certain procedure for passing the curved entrance channel with which he can make mistakes or which he might forget to perform.

Prior art solutions have in common that they are built from specially fabricated, usually plastic tubings, usually metal coils and machined parts and that assembly of such instruments is usually a time consuming and difficult process. Also tolerances of the separate parts add up in the assembly and can be the cause of a wide spread in for example instrument performance, often requiring an individual calibration of each instrument.

US2010/0228191 relates to a support structure for elongated instruments, such as catheters, and in particular to controllably and independently lockable and unlockable coupling interfaces. The instrument can navigate small pathways and selectively lock into and maintain a desired shape. The instrument may comprise several adjacent tubular structures having interfaces there between, whereby the adjacent structures may be, individually or as a set, spatially locked and unlocked relative to each other with application of a load. The tubular structures may comprise one or more spring members configured to deflect with application of a load greater than a preconfigured threshold, thereby causing a locking state of the interface to change from a first locking state to a second locking state. Embodiments are described wherein such load may be a tensile and/or compressive load. Disclosed examples of load applying members are pushrods, push cables, push coils, or coil tubes.

US2011/0004157 discloses a steerable tube wherein steering wires to deflect a distal bendable zone of the instrument are manufactured from the tube itself. In an embodiment, the tube comprises a braking mechanism to prevent slidable movements by the steering wires relative to an outer tube or inner tube. The object is to fix a position of the distal bendable zone. The only disclosed example of such a brake is a compressible annular ring having an inner diameter that varies according to the degree of compression. The inner circumference of the ring applies pressure to the steering wires when the ring is compressed along its central axis.

US2015/0107396 discloses a steerable instrument wherein steering wires to deflect a distal bendable zone of the instrument are manufactured from a tube. The instrument comprises a curving motion restricting member which is configured to be movable in the axial direction of the instrument, and which is configured to restrict curving of at least a portion of the instrument. Thus, the instrument is capable of preventing a portion to curve but is not capable of locking/unlocking a curved state of a hinge or locking/unlocking steering wires.
US2020/129166 A1 discloses a steerable instrument comprising radial spacers between coaxial cylindrical elements. The steerable instrument has an actuator at a proximal side and a bendable zone at a distal side. The actuator controls bending of the flexible zone by means of one or more longitudinal elements. The instrument has a first cylindrical element, a second cylindrical element and a third cylindrical element. The second cylindrical element has a zone with a longitudinal element portion with a first height in a radial direction of the instrument. Radial spacers are located at mutual sides of the longitudinal element portion, which have, at least locally, a second height in the radial direction larger than the first height.
US2012/323077 A1 discloses a steerable tune, an endoscopic instrument and an endoscope comprising such a tube, and an assembly. The steerable tube for endoscopic applications includes a first, a second and a third part. The first part includes a first movable portion and the third part includes a second movable portion. The steerable tube includes longitudinal elements that are constructed and arranged such that a change in orientation of the first movable portion of the first part with respect to the second part results in a change in orientation of the second movable portion of the third part with respect to the second part. The longitudinal elements are constructed and arranged such that they are twisted and/or that they are twistable. An endoscopic instrument and an endoscope including the steerable tube, and an assembly including an endoscopic instrument and the endoscope are also described.

### Summary of the invention

It is an object of the invention to provide a steerable instrument for endoscopic and/or invasive type of applications where at least one of the above mentioned problems are solved or at least reduced.

The invention is defined in the independent claim whereas dependent claims relate to advantageous embodiments.

### Summary of the disclosure

Thus, all relevant elements of the disclosure are made by making suitable slot patterns in several tubes inserted into each other, e.g., by (laser) cutting such patterns. Other techniques may be used for cutting such patterns as explained further below. Cutting such patterns is easy and cheap. After the slot patterns are made, some of the cut elements in different tubes need be attached to one another to arrive at the desired functionality. This can easily be done by (laser) welding or gluing, etc. Also this is a cheap process. By only using elements cut in several tubes, the cross section dimension of the invasive instrument can be reduced drastically which meets current market demands for those instruments. At the same time, these instruments are reliable and provide the operator with high precision as to maneuvering tools to desired operating sites, e.g. in a (human) body.

The disclosure may have several different applications. For instance, the invention can be used to compensate path length differences of steering wires due to bending of flexible portions of the instrument once the instrument is inserted into a curved channel, to lock or unlock individual hinges in the instrument, to limit bending of bendable portions of the instrument, to couple steering wires to other steering wires, to provide steering wires with a preload before using the instrument, etc.

The switching element may be configured for a switching function, a clamping function, a coupling function, a friction function, a braking function.

In one aspect the instrument is provided with at least one switching element with which an operator or robot can uncouple steering wire(s) from the steering unit, then the instrument can be navigated through a curved path without affecting the direction of the distal tip due to the body of the instrument taking the shape of the curved path. During navigation through the curved path, the tip of the instrument can move freely, because the steering wire(s) can move freely and the tip would follow the curvature of the curved path without being forced in another direction when the flexible body is bent. Once the operation site is reached, the switching element is operated such as to couple the steering wire(s) to the steering unit. In that way, the orientation of the tip can always be kept straight. Moreover, in an instrument in which the tip is deflected by a deflectable steering unit at the proximal end of the instrument, the operator can set the position of the steering unit to a corresponding position of the tip.

Prior art only describes a coupling that couples the steering wires to a fixed proximal end of the instrument and the described coupling only can act as an 'on-off' coupling. Also, the activation mechanism is composed of several separate parts.

In this application, the terms "proximal" and "distal" are defined with respect to an operator, e.g. a robot or physician that operates the instrument or endoscope. For example, a proximal end part is to be construed as a part that is located near the robot or physician and a distal end part as a part located at a distance from the robot or physician, i.e., in the area of operation.

All the necessary elements to construct a steerable instrument according to the disclosure are integrally manufactured, in a largely pre-assembled state, from a number of tubes. The only remaining assembly steps consist of sliding the tubes into each other and attach the tubes to each other in the required places. The preassembled parts can be made in a tube wall by material deposition processes like 3D printing or plating techniques. Preferably the preassembled parts can be made by material removal processes from a solid wall plastic or metal tube (stainless steel, cobalt chromium alloys, super-elastic alloys like nitinol, etc.). The material removal processes that can be used are for example conventional chipping processes, water jet cutting, etching and preferably laser cutting processes.

Therefore, those embodiments of this disclosure enable a significant reduction of manufacturing costs of such instruments and therefor the costs of an intervention in which these instruments are used. It even becomes commercially viable to use these instruments only once, and then throw them away. This increases the safety of an intervention because one can now use new instruments instead of pre-used and re-sterilized instruments that are known to have a 10% risk of post procedural complication due to contaminating or infecting the patient with not properly cleaned or re-sterilized pre-used instruments.

Another advantage of such an instrument is that by using this integrated way of producing parts in a pre-assembled state, they always fit to each other and that minimal play between the parts can be achieved. This is especially true when a laser cutting process is used. The minimal achievable play between two integrally manufactured parts is as low as the width of the used laser beam, which can be as small as 0.01mm. Typically a play of 0.01 to 0.05mm can be obtained easily. The integral fabrication of parts according to the disclosure therefor is so accurate with respect to fitting of parts and the play between them, that an improved accuracy and repeatability of the instrument's functional performance is ensured.

### Brief description of the drawings

Further features and advantages of the invention will become apparent from the description of the invention by way of non-limiting and non-exclusive embodiments. These embodiments are not to be construed as limiting the scope of protection. The person skilled in the art will realize that other alternatives and embodiments of the invention can be conceived and reduced to practice without departing from the scope of the present invention. Embodiments of the invention will be described with reference to the figures of the accompanying drawings, in which like or same reference symbols denote like, same or corresponding parts, and in which:
Figure 1 shows a schematic cross sectional view of an invasive instrument assembly having one bendable distal end portion and one proximal end portion which controls the bending of the bendable distal end portion by means of strips cut out in a cylindrical element.
Figure 2 shows a schematic overview of three cylindrical elements from which the instrument of Figure 1 may be manufactured.
Figure 3a shows a portion of an intermediate cylindrical element of the instrument of Figures 1 and 2.
Figure 3b shows an alternative example of an intermediate cylindrical element of such an instrument.
Figure 4 shows an example intermediate cylindrical element and an inner cylindrical element inserted in the intermediate cylindrical element.
Figure 5 shows an outside view of a steerable invasive instrument assemble having two steerable bendable distal end portions and two proximal flexible control portions.
Figure 6 shows an enlarged view of the distal tip of the instrument shown in Figure 5.
Figure 7 shows a cross section view through the invasive instrument shown in Figure 5.
Figures 8 and 9 show examples of how the invasive instrument of Figures 5 and 7 can bend.
Figure 10 shows an alternative embodiment of the invasive instrument shown in Figures 5-9, wherein at least a portion of an intermediate section between the distal end and the proximal end is flexible too.
Figures 11 and 12 show schematic examples of using an invasive instrument as an endoscopic surgical instrument in which the intermediate section between the distal end and the proximal end is flexible too such that the invasive instrument can be inserted in a natural body canal like the intestinal canal, and the oesophagus.
Figures 13 - 18C schematically show how steering wires or control wires can be switched/clamped to other parts of the instrument in order to provide extra functionality to the instrument.
Figures 19A, 19B show a mechanism to freeze a deflection of a hinge.
Figure 20 shows a telescopic device.
Figure 21 shows a device with switching elements that can be used to freeze a hinge in a deflected orientation.
Figures 22A - 36 show some schematic examples of how steering wires can be clamped to other portions of the instrument, e.g. other steering wire portions or the "fixed" world of the instrument body.
Figures 37A - 40 show some schematic mechanisms that can be used to freeze adjacent hinge segments.
Figures 41A - 41C show an implementation with several tubes in which steering wires can be locked.
Figures 42A - 42C show an implementation with several tubes in which steering wires can be pre-loaded with a tension force.
Figures 43A - 43C show an implementation with several tubes in which steering wires can be locked.
Figures 44A - 44C and figures 45A - 45C show implementations with several tubes in which a hinge can be frozen in a current deflected orientation.
Figures 46A - 46E show an implementation with one or more tubes in which different portions of a steering wire can be connected to one another such that they can operate as a single steering wire.
Figures 47A, 47B show an example of how adjacent longitudinal elements can be coupled with a switching mechanism.

### Description of embodiments

For the purpose of the present document, the terms cylindrical element and tube may be used interchangeably, i.e., the term cylindrical element also refers to a physical entity. The disclosure will be explained with reference to steering wires which are cut from such cylindrical elements and are operative as push and/or pull steering wires to transfer movement of the steering wires at the proximal end of the instrument to the distal end to thereby control bending of one or more flexible distal end portions.

### Instruments in which the invention can be applied

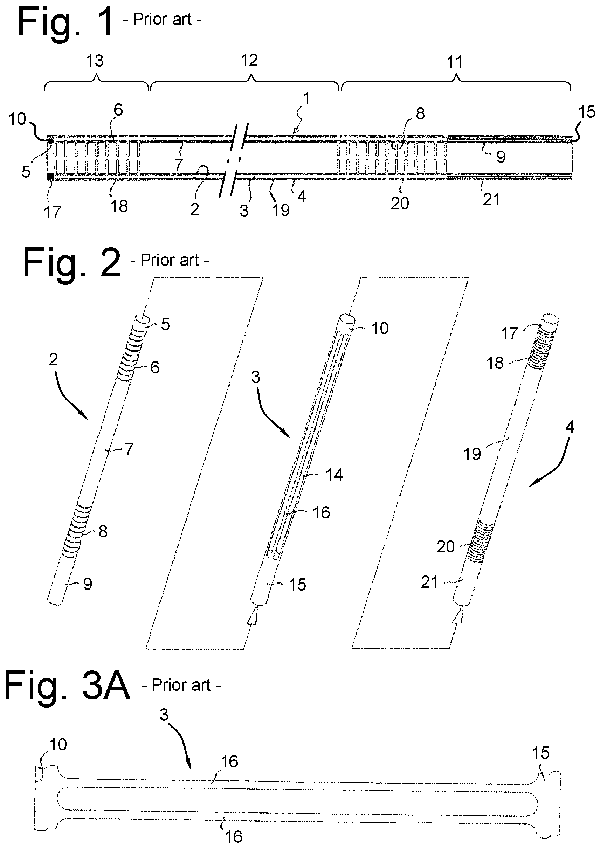

Figures 1, 2, 3a, and 3b are known from WO2009/112060. They are explained in detail because the present invention can be applied in this type of instruments.

Figure 1 shows a longitudinal cross-section of a prior art steerable instrument comprising three co-axially arranged cylindrical elements, i.e. inner cylindrical element 2, intermediate cylindrical element 3 and outer cylindrical element 4. Suitable materials to be used for making the cylindrical elements 2, 3, and 4 include stainless steel, cobalt-chromium, shape memory alloy such as Nitinol^{®}, plastic, polymer, composites or other materials that can be shaped by material removal processes like laser cutting or EDM. Alternatively, the cylindrical elements can be made by a 3D printing process or other known material deposition processes.

The inner cylindrical element 2 comprises a first rigid end part 5, which is located at a distal end part 13 of the instrument, a first flexible part 6, an intermediate rigid part 7 located at an intermediate part 12 of the instrument, a second flexible part 8 and a second rigid end part 9, which is located at a proximal end part 11 of the instrument.

The outer cylindrical element 4 also comprises a first rigid end part 17, a first flexible part 18, an intermediate rigid part 19, a second flexible part 20 and a second rigid end part 21. The lengths of the parts 5, 6, 7, 8, and 9, respectively, of the cylindrical element 2 and the parts 17, 18, 19, 20, and 21, respectively, of the cylindrical element 4 are, preferably, substantially the same so that when the inner cylindrical element 2 is inserted into the outer cylindrical element 4, these different respective parts are longitudinally aligned with each other.

The intermediate cylindrical element 3 also has a first rigid end part 10 and a second rigid end part 15 which in the assembled condition are located between the corresponding rigid parts 5, 17 and 9, 21 respectively of the two other cylindrical elements 2, 4. The intermediate part 14 of the intermediate cylindrical element 3 comprises one or more separate steering wires 16 which can have different forms and shapes as will be explained below. They are made from the cylindrical element 3 themselves and have the form of a longitudinal strip. In figure 3a, two such steering wires 16 are shown. After assembly of the three cylindrical elements 2, 3 and 4 whereby the element 2 is inserted in the element 3 and the two combined elements 2, 3 are inserted into the element 4 (any other order is possible), at least the first rigid end part 5 of the inner cylindrical element 2, the first rigid end part 10 of the intermediate cylindrical element 3 and the first rigid end part 17 of the outer cylindrical element 4 at the distal end of the instrument are attached to each other, e.g., by means of glue or one or more laser welding spots. In the embodiment shown in figures 1 and 2, also the second rigid end part 9 of the inner cylindrical element 2, the second rigid end part 15 of the intermediate cylindrical element 3 and the second rigid end part 21 of the outer cylindrical element 4 at the proximal end of the instrument are attached to each other, e.g. by means of glue or one or more laser welding spots, such that the three cylindrical elements 2, 3, 4 form one integral unit.

In the embodiment shown in figure 2 the intermediate part 14 of intermediate cylindrical element 3 comprises a number of steering wires 16 with a uniform cross-section so that the intermediate part 14 has the general shape and form as shown in the unrolled condition of the intermediate cylindrical element 3 in figure 3a. From figure 3a it also becomes clear that the intermediate part 14 is formed by a number of over the circumference of the intermediate cylindrical part 3, possibly equally, spaced parallel steering wires 16. Advantageously, the number of steering wires 16 is at least three, so that the instrument becomes fully controllable in any direction, but any higher number is possible as well. The number of steering wires 16 may, e.g., be six or eight.

It is observed that the steering wires 16 need not have a uniform cross section across their entire length. They may have a varying width along their length, possibly such that at one or more locations adjacent steering wires 16 are only separated by a small slot resulting from the laser cutting in the cylindrical element 3. These wider portions of the steering wires, then, operate as spacers to prevent adjacent steering wires 16 from buckling in a tangential direction in a pushed state. Spacers may, alternatively, be implemented in other ways.

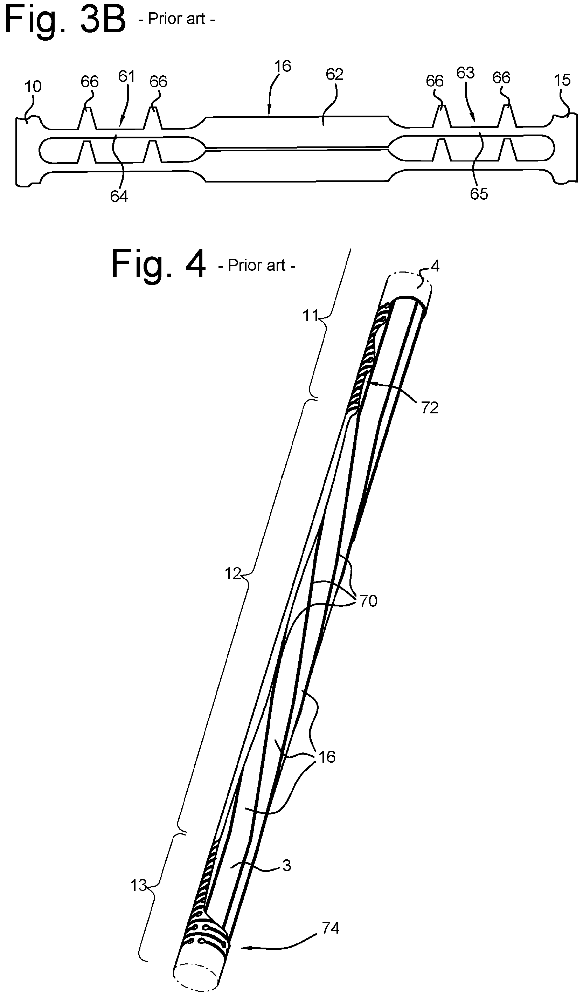

An embodiment with spacers is shown in figure 3b which shows two adjacent steering wires 16 in an unrolled condition. In the embodiment shown in figure 3b each steering wire 16 is composed of three portions 61, 62 and 63, co-existing with the first flexible part 6, 18 the intermediate rigid part 7, 19 and the second flexible part 8, 20 respectively. In the portion 62 coinciding with the intermediate rigid portion, each pair of adjacent steering wires 16 is almost touching each other in the tangential direction so that in fact only a narrow slot is present there between just sufficient to allow independent movement of each steering wire. The slot results from the manufacturing process and its width is, e.g., caused by the diameter of a laser beam cutting the slot.

In the other two portions 61 and 63 each steering wire consists of a relatively small and flexible part 64, 65 as seen in circumferential direction, so that there is a substantial gap between each pair of adjacent flexible parts, and each flexible part 64, 65 is provided with a number of spacers 66, extending in the tangential direction and almost bridging completely the gap to the adjacent flexible part 64, 65. Because of these spacers 66 the tendency of the steering wires 16 in the flexible portions of the instrument to shift in tangential direction is suppressed and tangential direction control is improved. The exact shape of these spacers 66 is not very critical, provided they do not compromise flexibility of flexible parts 64 and 65. The spacers 66 may form an integral part with the flexible parts 64, 65 and may result from a suitable laser cutting process too.

In the embodiment shown in figure 3b the spacers 66 are extending towards one tangential direction as seen from the flexible part 64, 65 to which they are attached. It is however also possible to have these spacers 66 extending to both circumferential directions starting from one flexible part 64, 65. By using this it is either possible to have alternating types of flexible parts 64, 65 as seen along the tangential direction, wherein a first type is provided at both sides with spacers 66 extending until the next flexible part, and a second intermediate set of flexible parts 64, 65 without spacers 66. Otherwise it is possible to have flexible parts with cams at both sides, where as seen along the longitudinal direction of the instrument the cams originating from one flexible part are alternating with spacers originating from the adjacent flexible parts. It is obvious that numerous alternatives are available.

The production of such an intermediate part is most conveniently done by injection moulding or plating techniques or starting from a cylindrical tube with the desired inner and outer diameters and removing parts of the wall of the cylindrical tube required e.g. by laser or water cutting to end up with the desired shape of the intermediate cylindrical element 3. However, alternatively, any 3D printing method can be used.

The removal of material can be done by means of different techniques such as laser cutting, photochemical etching, deep pressing, conventional chipping techniques such as drilling or milling, high pressure water jet cutting systems or any suitable material removing process available. Preferably, laser cutting is used as this allows for a very accurate and clean removal of material under reasonable economic conditions. The above mentioned processes are convenient ways as the cylindrical element 3 can be made so to say in one process, without requiring additional steps for connecting the different parts of the intermediate cylindrical element as required in the conventional instruments, where conventional steering cables must be connected in some way to the end parts. The same type of technology can be used for producing the inner and outer cylindrical elements 2 and 4 with their respective flexible parts 6, 8, 18 and 20. These flexible parts 6, 8, 18 and 20 can be manufactured as hinges resulting from cutting out any desired pattern from the cylindrical elements, e.g., by using any of the methods described in European patent application 08 004 373.0 filed on 10.03.2008, page 5, lines 15-26, but any other suitable process can be used to make flexible portions.

It is observed that the instruments shown in figures 4-10 are known from prior art WO2020/214027. Also in these instruments the present invention can be applied.

Figure 4 shows an exemplary embodiment of longitudinal (steering) elements 16 that have been obtained after providing longitudinal slots 70 to the wall of the intermediate cylindrical element 3 that interconnects proximal flexible zone 14 and distal flexible zone 16 as described above. Here, steering wires 16 are, at least in part, spiralling about a longitudinal axis of the instrument such that an end portion of a respective steering element 16 at the proximal portion of the instrument is arranged at another angular orientation about the longitudinal axis than an end portion of the same steering wire 16 at the distal portion of the instrument. Were the steering wires 16 arranged in a linear orientation, than a bending of the instrument at the proximal portion in a certain plane would result in a bending of the instrument at the distal portion in the same plane but in a 180 degrees opposite directions. This spiral construction of the steering wires 16 allows for the effect that bending of the instrument at the proximal portion in a certain plane may result in a bending of the instrument at the distal portion in another plane, or in the same plane in the same direction. A preferred spiral construction may be such that the end portion of a respective steering element 16 at the proximal portion of the instrument is arranged at an angularly shifted orientation of 180 degrees about the longitudinal axis relative to the end portion of the same steering wire 16 at the distal portion of the instrument. However, e.g. any other angularly shifted orientation, e.g. 90 degrees, is within the scope of this document. The slots 70 are dimensioned such that movement of a steering wire is guided by adjacent steering wires when provided in place in a steerable instrument. However, especially at the flexible zones 13, 14 of the instrument, the width of steering wires 16 may be less to provide the instrument with the required flexibility / bendability at those locations.

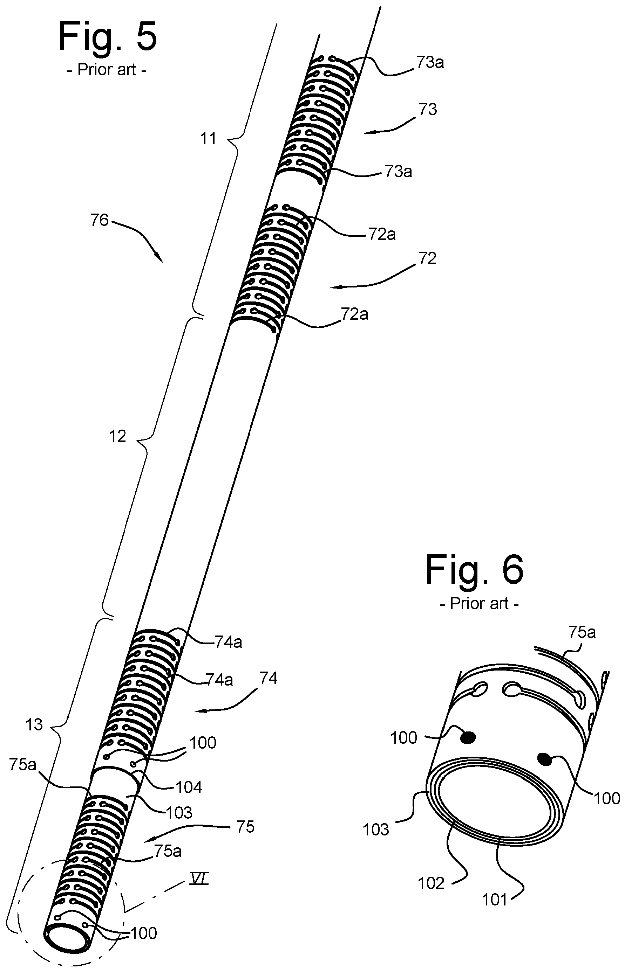

Figure 5 provides a detailed perspective view of the distal portion of an embodiment of an elongated tubular body 76 of a steerable instrument which has two steerable distal bendable zones 74, 75 which are operated by two bendable proximal zones 72, 73, respectively. Figure 5 shows that the elongated tubular body 76 comprises a number of co-axially arranged layers or cylindrical elements including an outer cylindrical element 104 that ends after a first distal flexible zone 74 at the distal end portion 13. The distal end portion 13 of the outer cylindrical element 104 is fixedly attached to a cylindrical element 103 located inside of and adjacent to the outer cylindrical element 104, e.g. by means of spot welding at welding spots 100. However, any other suitable attachment method can be used, including any mechanical snap fit connection or gluing by a suitable glue.

Figure 6 provides a more detailed view of the distal end part 13 and shows that, in this embodiment, it includes three co-axially arranged layers or cylindrical elements, i.e., an inner cylindrical element 101, a first intermediate cylindrical element 102 and a second intermediate cylindrical element 103. The distal ends of inner cylindrical element 101, first intermediate cylindrical element 102 and second intermediate cylindrical element 103 are all three fixedly attached to one another. This may be done by means of spot welding at welding spots 100. However, any other suitable attachment method can be used, including any mechanical snap fit connection or gluing by a suitable glue. The points of attachment may be at the end edges of inner cylindrical element 101, first intermediate cylindrical element 102 and second intermediate cylindrical element 103, as shown in the figures. However, these points of attachment may also be located some distance away from these edges, be it, preferably, between the end edges and the locations of the flexible zone 75.

It will be clear to the skilled person that the elongated tubular body 76 as shown in figure 5 comprises four cylindrical elements in total. The elongated tubular body 76 according to the embodiment shown in figure 5 comprises two intermediate cylindrical elements 102 and 103 in which the steering members of the steering arrangement are arranged. However, extra or less cylindrical elements may be provided if desired.

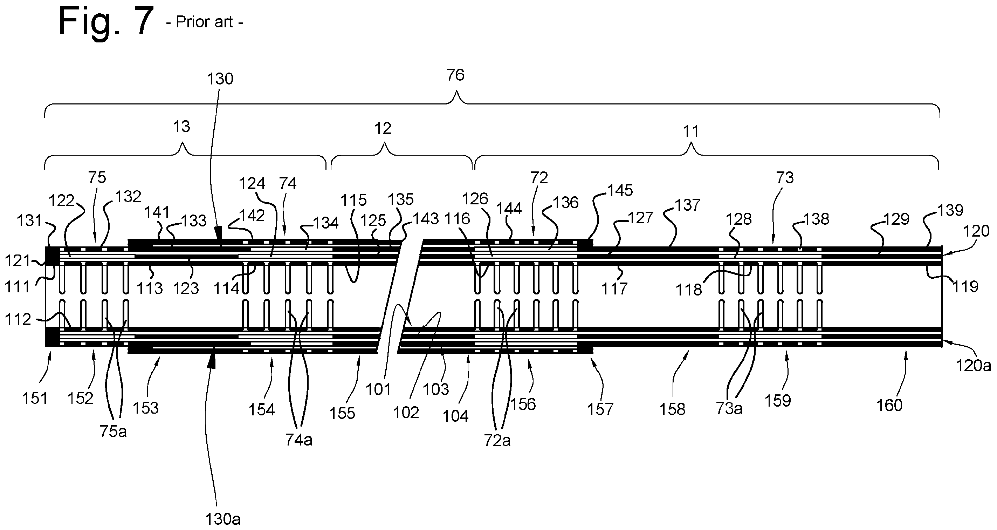

The steering arrangement in the exemplary embodiment of the elongated tubular body 76 as shown in figure 5 comprises the two flexible zones 72, 73 at the proximal end part 11 of the elongated tubular body 76, the two flexible zones 74, 75 at the distal end part 13 of the elongated tubular body 76 and the steering members that are arranged between related flexible zones at the proximal 11 and distal 13 end parts. An exemplary actual arrangement of the steering members is shown in figure 7, which provides a schematic longitudinal cross-sectional view of the exemplary embodiment of the elongated tubular body 76 as shown in figure 5.

Flexible zones 72, 73, 74, and 75 are, in this embodiment, implemented by providing the respective cylindrical elements with slits 72a, 73a, 74a, and 75a, respectively. Such slits 72a, 73a, 74a, and 75a may be arranged in any suitable pattern such that the flexible zones 72, 73, 74, and 75 have a desired flexibility in the longitudinal and tangential direction in accordance with a desired design.

Figure 7 shows a longitudinal cross section of the four layers or cylindrical elements mentioned above, i.e. the inner cylindrical element 101, the first intermediate cylindrical element 102, the second intermediate cylindrical element 103, and the outer cylindrical element 104.

The inner cylindrical element 101, as seen along its length from the distal end to the proximal end of the instrument, comprises a rigid ring 111, which is arranged at the distal end part 13 of the steerable instrument 10, a first flexible portion 112, a first intermediate rigid portion 113, a second flexible portion 114, a second intermediate rigid portion 115, a third flexible portion 116, a third intermediate rigid portion 117, a fourth flexible portion 118, and a rigid end portion 119, which is arranged at the proximal end portion 11 of the steerable instrument.

The first intermediate cylindrical element 102, as seen along its length from the distal end to the proximal end of the instrument, comprises a rigid ring 121, a first flexible portion 122, a first intermediate rigid portion 123, a second flexible portion 124, a second intermediate rigid portion 125, a third flexible portion 126, a third intermediate rigid portion 127, a fourth flexible portion 128, and a rigid end portion 129. The portions 122, 123, 124, 125, 126, 127 and 128 together form a steering wire 120 that can be moved in the longitudinal direction like a wire. The longitudinal dimensions of the rigid ring 121, the first flexible portion 122, the first intermediate rigid portion 123, the second flexible portion 124, the second intermediate rigid portion 125, the third flexible portion 126, the third intermediate rigid portion 127, the fourth flexible portion 128, and the rigid end portion 129 of the first intermediate element 102, respectively, are aligned with, and preferably approximately equal to the longitudinal dimensions of the rigid ring 111, the first flexible portion 112, the first intermediate rigid portion 113, the second flexible portion 114, the second intermediate rigid portion 115, the third flexible portion 116, the third intermediate rigid portion 117, the fourth flexible portion 118, and the rigid end portion 119 of the inner cylindrical element 101, respectively, and are coinciding with these portions as well. In this description "approximately equal" means that respective same dimensions are equal within a margin of less than 10%, preferably less than 5%.

Similarly, the first intermediate cylindrical element 102 comprises one or more other steering wires of which one is shown with reference number 120a.

The second intermediate cylindrical element 103, as seen along its length from the distal end to the proximal end of the instrument, comprises a first rigid ring 131, a first flexible portion 132, a second rigid ring 133, a second flexible portion 134, a first intermediate rigid portion 135, a first intermediate flexible portion 136, a second intermediate rigid portion 137, a second intermediate flexible portion 138, and a rigid end portion 139. The portions 133, 134, 135 and 136 together form a steering wire 130 that can be moved in the longitudinal direction like a wire. The longitudinal dimensions of the first rigid ring 131, the first flexible portion 132 together with the second rigid ring 133 and the second flexible portion 134, the first intermediate rigid portion 135, the first intermediate flexible portion 136, the second intermediate rigid portion 137, the second intermediate flexible portion 138, and the rigid end portion 139 of the second intermediate cylinder 103, respectively, are aligned with, and preferably approximately equal to the longitudinal dimensions of the rigid ring 111, the first flexible portion 112, the first intermediate rigid portion 113, the second flexible portion 114, the second intermediate rigid portion 115, the third flexible portion 116, the third intermediate rigid portion 117, the fourth flexible portion 118, and the rigid end portion 119 of the first intermediate element 102, respectively, and are coinciding with these portions as well.

Similarly, the second intermediate cylindrical element 103 comprises one or more other steering wires of which one is shown with reference number 130a.

The outer cylindrical element 104, as seen along its length from the distal end to the proximal end of the instrument, comprises a first rigid ring 141, a first flexible portion 142, a first intermediate rigid portion 143, a second flexible portion 144, and a second rigid ring 145. The longitudinal dimensions of the first flexible portion 142, the first intermediate rigid portion 143 and the second flexible portion 144 of the outer cylindrical element 104, respectively, are aligned with, and preferably approximately equal to the longitudinal dimension of the second flexible portion 134, the first intermediate rigid portion 135 and the first intermediate flexible portion 136 of the second intermediate element 103, respectively, and are coinciding with these portions as well. The rigid ring 141 has approximately the same length as the rigid ring 133 and is fixedly attached thereto, e.g. by spot welding or gluing. Preferably, the rigid ring 145 overlaps with the second intermediate rigid portion 137 only over a length that is required to make an adequate fixed attachment between the rigid ring 145 and the second intermediate rigid portion 137, respectively, e.g. by spot welding or gluing. The rigid rings 111, 121 and 131 are attached to each other, e.g., by spot welding or gluing. This may be done at the end edges thereof but also at a distance of these end edges.

In an embodiment, the same may apply to the rigid end portions 119, 129 and 139, which can be attached to one another as well in a comparable manner. However, the construction may be such that the diameter of the cylindrical elements at the proximal portion is larger, or smaller, with respect to the diameter at the distal portion. In such embodiment the construction at the proximal portion differs from the one shown in figure 7. As a result of the increase or decrease in diameter an amplification or attenuation is achieved, i.e., the bending angle of a flexible zone at the distal portion will be larger or smaller than the bending angle of a corresponding flexible portion at the proximal portion.

The inner and outer diameters of the cylindrical elements 101, 102, 103, and 104 are chosen in such a way at a same location along the elongated tubular body 76 that the outer diameter of inner cylindrical element 101 is slightly less than the inner diameter of the first intermediate cylindrical element 102, the outer diameter of the first intermediate cylindrical element 102 is slightly less than the inner diameter of the second intermediate cylindrical element 103 and the outer diameter of the second intermediate cylindrical element 103 is slightly less than the inner diameter of the outer cylindrical element 104, in such a way that a sliding movement of the adjacent cylindrical elements with respect to each other is possible. The dimensioning should be such that a sliding fit is provided between adjacent elements. A clearance between adjacent elements may generally be in the order of 0.02 to 0.1 mm, but depends on the specific application and material used. The clearance may be smaller than a wall thickness of the steering wires to prevent an overlapping configuration thereof. Restricting the clearance to about 30% to 40% of the wall thickness of the steering wires is generally sufficient.

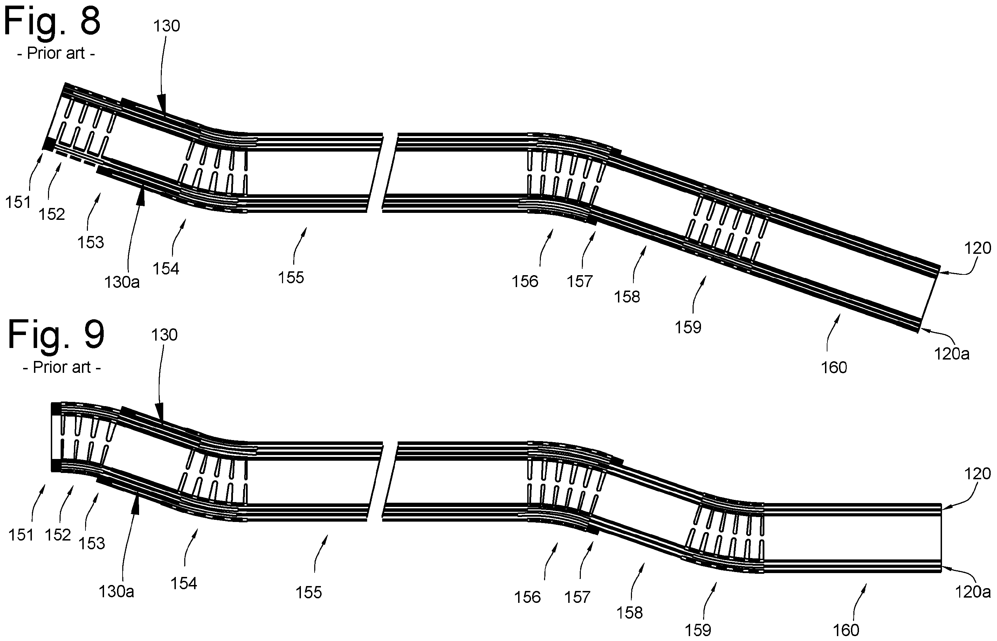

As can be seen in figure 7, flexible zone 72 of the proximal end part 11 is connected to the flexible zone 74 of the distal end part 13 by portions 134, 135 and 136, of the second intermediate cylindrical element 103, which form a first set of steering wires of the steering arrangement of the steerable instrument. Furthermore, flexible zone 73 of the proximal end part 11 is connected to the flexible zone 75 of the distal end part 13 by portions 122, 123, 124, 125, 126, 127, and 128 of the first intermediate cylindrical element 102, which form a second set of steering wires of the steering arrangement. The use of the construction as described above allows the steerable instrument 10 to be used for double bending. The working principle of this construction will be explained with respect to the examples shown in figures 8 and 9.

For the sake of convenience, as shown in figures 7, 8 and 9, the different portions of the cylindrical elements 101, 102, 103, and 104 have been grouped into zones 151 - 160 that are defined as follows. Zone 151 comprises the rigid rings 111, 121, and 131. Zone 152 comprises the portions 112, 122, and 132. Zone 153 comprises the rigid rings 133 and 141 and the portions 113 and 123. Zone 154 comprises the portions 114, 124, 134 and 142. Zone 155 comprises the portions 115, 125, 135 and 143. Zone 156 comprises the portions 116, 126, 136 and 144. Zone 157 comprises the rigid ring 145 and the parts of the portions 117, 127, and 137 coinciding therewith. Zone 158 comprises the parts of the portions 117, 127, and 137 outside zone 157. Zone 159 comprises the portions 118, 128 and 138. Finally, zone 160 comprises the rigid end portions 119, 129 and 139.

In order to deflect at least a part of the distal end part 13 of the steerable instrument, it is possible to apply a bending force, in any radial direction, to zone 158. According to the examples shown in figures 8 and 9, zone 158 is bent downwards with respect to zone 155. Consequently, zone 156 is bent downwards. Because of the first set of steering wires comprising portions 134, 135, and 136 of the second intermediate cylindrical element 103 that are arranged between the second intermediate rigid portion 137 and the second rigid ring 133, the downward bending of zone 156 is transferred by a longitudinal displacement of the first set of steering wires into an upward bending of zone 154 with respect to zone 155. This is shown in both figures 8 and 9.

It is to be noted that the exemplary downward bending of zone 156, only results in the upward bending of zone 154 at the distal end of the instrument as shown in figure 8. Bending of zone 152 as a result of the bending of zone 156 is prevented by zone 153 that is arranged between zones 152 and 154. When subsequently a bending force, in any radial direction, is applied to the zone 160, zone 159 is also bent. As shown in figure 9, zone 160 is bent in an upward direction with respect to its position shown in figure 8. Consequently, zone 159 is bent in an upward direction. Because of the second set of steering wires comprising portions 122, 123, 124, 125, 126, 127 and 128 of the first intermediate cylindrical element 102 that are arranged between the rigid ring 121 and the rigid end portion 129, the upward bending of zone 159 is transferred by a longitudinal displacement of the second set of steering wires into a downward bending of zone 152 with respect to its position shown in figure 8.

Figure 9 further shows that the initial bending of the instrument in zone 154 as shown in figure 8 will be maintained because this bending is only governed by the bending of zone 156, whereas the bending of zone 152 is only governed by the bending of zone 159 as described above. Due to the fact that zones 152 and 154 are bendable independently with respect to each other, it is possible to give the distal end part 13 of the steerable instrument a position and longitudinal axis direction that are independent from each other. In particular the distal end part 13 can assume an advantageous S-like shape. The skilled person will appreciate that the capability to independently bend zones 152 and 154 with respect to each other, significantly enhances the maneuverability of the distal end part 13 and therefore of the steerable instrument as a whole.

Obviously, it is possible to vary the lengths of the flexible portions shown in figures 7 to 9 as to accommodate specific requirements with regard to bending radii and total lengths of the distal end part 13 and the proximal end part 11 of the steerable instrument or to accommodate amplification or attenuation ratios between bending of at least a part of the proximal end part 11 and at least a part of the distal end part 13.

In the shown embodiment, the steering wires comprise one or more sets of steering wires that form integral parts of the one or more intermediate cylindrical elements 102, 103. Preferably, the steering wires comprise remaining parts of the wall of an intermediate cylindrical element 102, 103 after the wall of the intermediate cylindrical element 102, 103 has been provided with longitudinal slits that define the remaining steering wires.

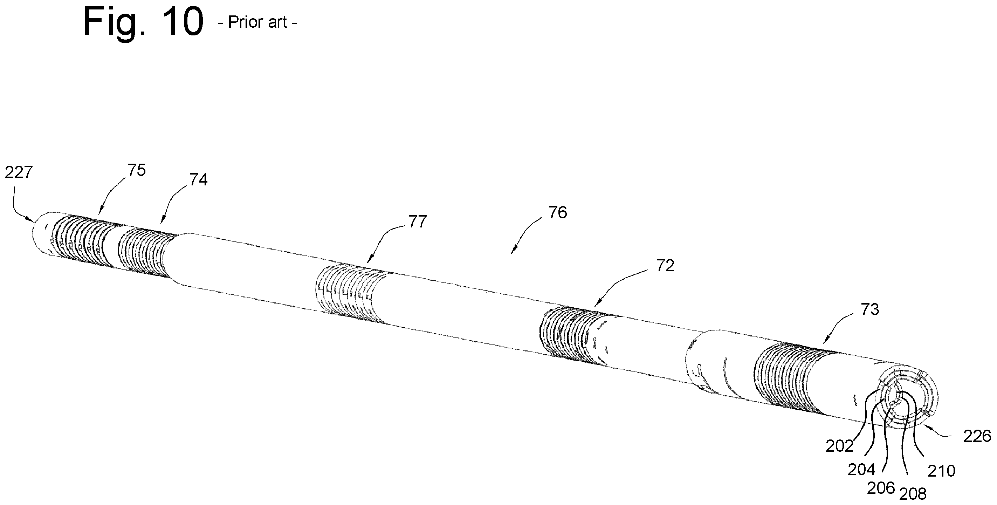

Figure 10 shows a 3D view of an example of a steerable instrument. Like reference numbers refer to the same elements as in other figures. Their explanation is not repeated here. The instruments comprises five coaxial cylindrical elements 202-210. An inner cylindrical element 210 is surrounded by intermediate cylindrical element 208 which is surrounded by intermediate cylindrical element 206 which is surrounded by intermediate cylindrical element 204 which is, finally surrounded by outer cylindrical element 202. Inner intermediate cylindrical element may be made of a flexible spiraling spring. The proximal and distal ends, respectively, of the instrument are indicated with reference numbers 226 and 227, respectively.

As shown, here, instrument 76 comprises a flexible zone 77 in its intermediate part between flexible zone 72 and flexible zone 74. I.e., intermediate cylindrical element 204 (which is located at the outer side in the area of flexible zone 77) is provided with a slotted structure to provide intermediate cylindrical element with a desired flexibility. The longitudinal length of the slotted structure in flexible zone 77 depends on the desired application. It may be as long as the entire part between flexible zones 72 and 74. All other cylindrical elements 206, 208, 210 inside intermediate cylindrical element 204 are also flexible in flexible zone 77. Those cylindrical elements that have steering wires in the flexible zone 77 are flexible by way of definition. Others are provided with suitable hinges, preferably made by suitable slotted structures.

Some locations to be operated in a body need specifically designed instruments. E.g., by making the intermediate part 12 of the instrument completely flexible, the instrument can also be used in areas in the body which are only accessible via curved natural access guides/channels, like the colon, the stomach via the oesophagus or the heart via curved blood vessels.

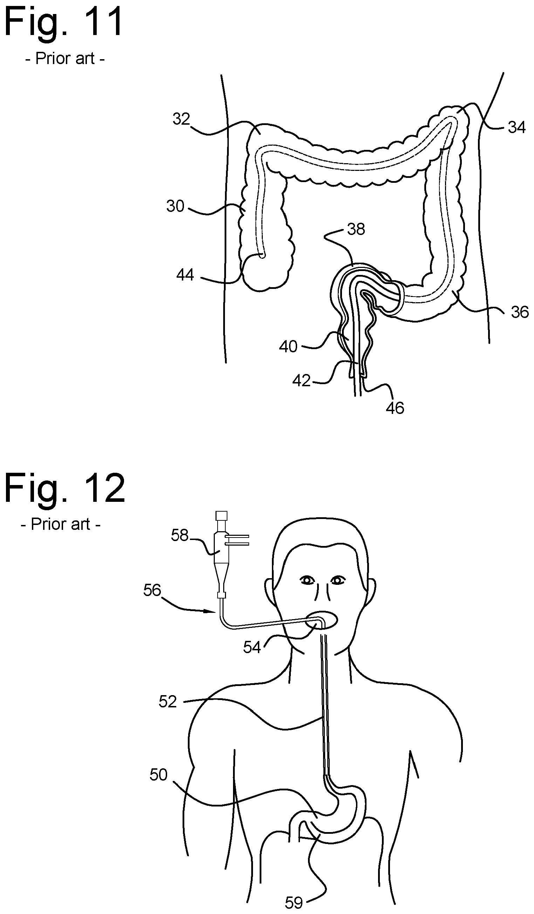

The instrument can e.g. be designed to be used as a colonoscope. Figure 11 shows a schematic view of a colonoscope 42 in use. The colonoscope 42 is inserted into a colon 30 of a human body. Typically, the colon 30 has several almost square angled sections 32, 34, 36, and 38. If a surgeon needs to operate an area of the colon 30 upstream from square angled section 32 the colonoscope 42 needs to be inserted into the colon 30 along a distance of up to 1.5 meter. Moreover, the colonoscope 42 needs to be so flexible that it can be guided from an anus through all squared angled sections 32-38 of the colon 30 easily without risks of damaging the inner wall of the colon 30.

In operation, usually, several invasive instruments are inserted through the colonoscope 42 to provide one or more tools for some function at its distal end 44. In colonoscopy, such a tool typically includes a camera lens and a lighting element. To assist the surgeon in steering the camera view to the desired location and view in colon 30, typically, the distal end is deflectable from a longitudinal axis in all angular directions. This also holds for the inserted instruments with tools 2. That can be implemented by providing such an instruments with one or more deflectable zones, like the deflectable zones 16, 17 of the instrument shown in Figures 5-10. These distal deflectable zones are controlled by suitable steering cables accommodated in the instruments connected to a suitable steering mechanism at the proximal ends of the instruments.

Figure 12 shows a schematic view of a gastroscope 56 in use. The gastroscope 56 is inserted into a stomach 50 of a human body via mouth, oral cavity / throat 54 and oesophagus 52. Especially when a surgeon needs to operate a lower portion of the stomach 50, the gastronoscope 56 needs to be guided through several curved/angled sections. Therefore, the gastroscope 56 needs to be flexible such that there is little risk of damaging inner walls of the mouth/throat 54, oesophagus 52 and stomach 50.

In operation, usually, several invasive instruments are inserted with the gastroscope 56 to provide one or more tools for some function at its distal end 59. In gastroscopy, such a tool typically includes a camera lens and a lighting element. To assist the surgeon in steering the camera view to the desired location and direction in stomach 50, typically, the distal end 59 of the gastrocope 56 is deflectable from a longitudinal axis in all angular directions. This also holds for the inserted instruments with tools too. That can be implemented by providing such an instrument with one or more deflectable zones, like the deflectable zones 16, 17 of the instrument shown in Figures 5-10. These distal deflectable zones are controlled by suitable steering cables accommodated in the instruments connected to a suitable steering mechanism of these instruments.

Instruments according to the invention can be used in such colonoscopes and gastroscopes but also in other applications like instruments designed for entering the lung bronchi. Requirements to such an instrument may be that they show a high rotational stiffness, high longitudinal stiffness, bending flexibility along its entire length and accurate and repeatable deflectability of a steerable tip even in cases of long instruments, e.g., longer than 1 m, and with a relatively small diameter that fits to the working channels within or attached to colonoscopes and gastroscopes.

As will be described hereinafter, many different new and innovative instrument configurations can be created by adding switching features that have either on/off, clamping, coupling, braking or movement limiting functions or combinations thereof. The invention describes switching mechanisms that can be used universally for the stated problems.

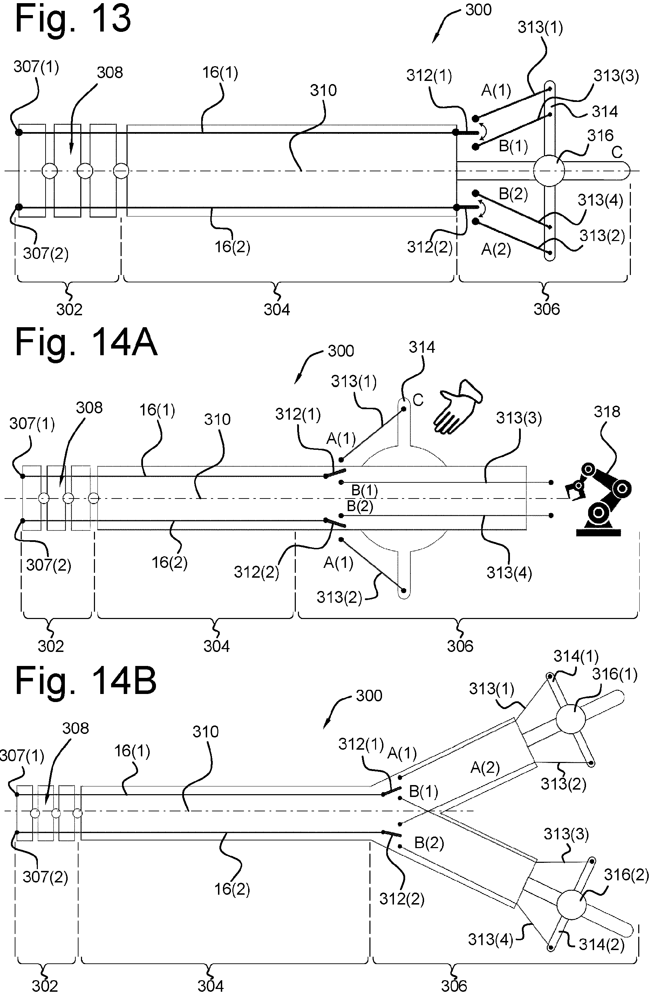

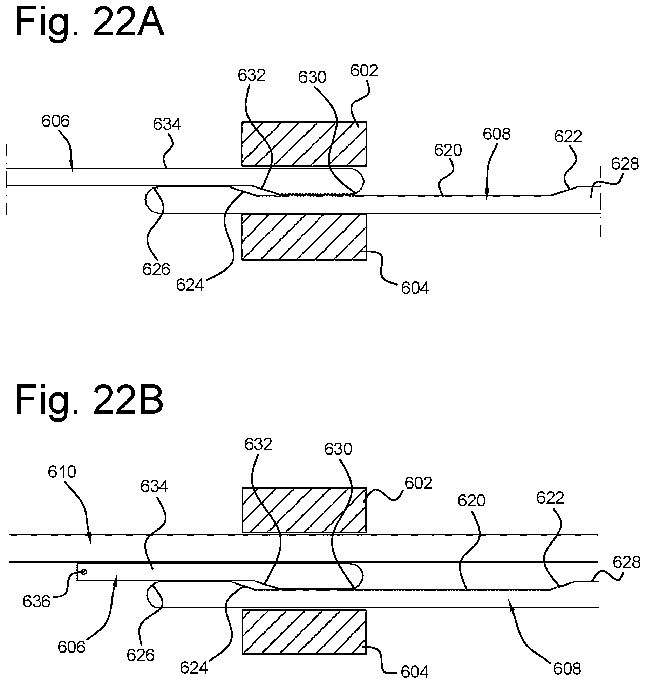

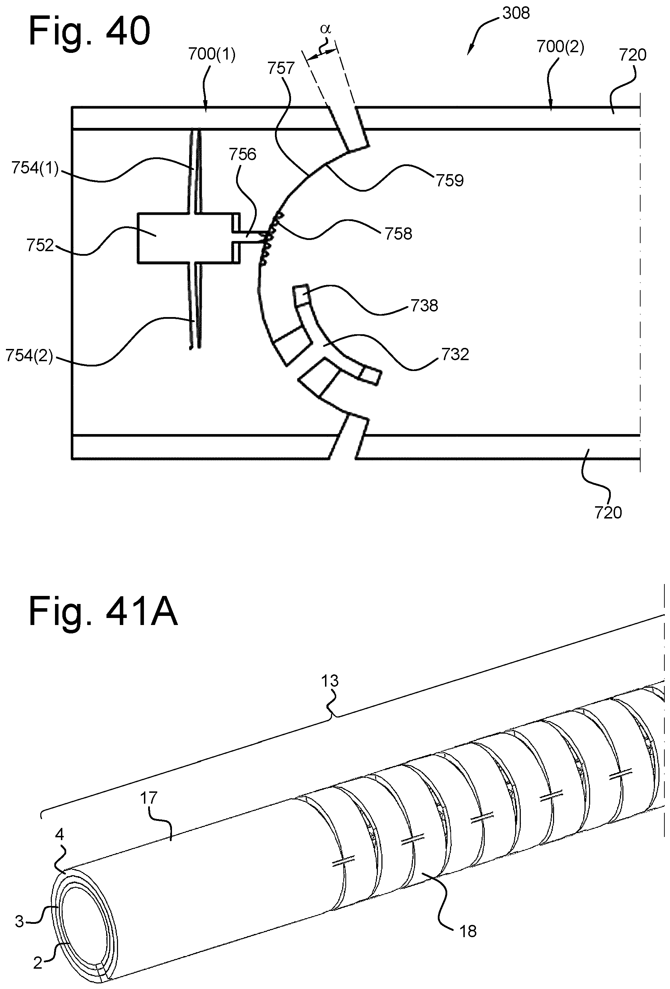

Figures 13-21 show how switching mechanisms can be used in invasive instruments, figures 22A to 40 show examples of switching elements implemented by cutting suitable patterns in a tube, and figures 41A to 47B show examples of invasive instruments based on using tubes including one or more switching elements comprising elements cut in one or more of those tubes. Figures 13-21 show tip sections that can be deflected in one surface only (i.e. the surface of the drawings). However, by applying three or more steering wires and hinges that can deflect in any direction, omni-deflectable tip sections can be made, as explained with reference to figures 1-12.

Many conventional invasive instruments have a tip section with one or more deflectable tip section portions and a steering section with one or more deflectable steering section portions. These conventional instruments have a fixed angle change ratio between the steering section portions and the tip section portions. These instruments are engineered such that a certain deflection of a steering section portion results in a certain deflection of the associated tip section portion. Usually an amplification of the steering angle change is used, e.g., a relatively small hand movement of the steering section results in a large deflection of the tip section. This is done to be able to deflect the tip section to a large deflection of up to 270 degrees with a deflection of the steering section of for example 30 degrees or whatever angle is still convenient for the operator. A strong disadvantage of this amplification of hand movement is that precision of tip section placement is adversely affected. Also unwanted tremors or accidental movement in the operator's hand are translated into larger unintended deflections of the tip section, which can adversely affect the quality of an operation or even make this dangerous.

If the operator could use a switching feature, optionally with a switch function, with which he could uncouple steering wires and couple them to another position inside the steering section that has a lower amplification, he could first make a rough movement with the tip section towards an approximate target site inside a body and then switch to a lower amplification ratio to gain precision of the tip section's steerability. In that way, also tremors or unintended hand movements are translated into much smaller unintended tip deflections, which increases the effectiveness and safety of use of such instrument. Figure 13 shows an implementation example.

Figure 13 shows a schematic overview of an invasive instrument 300 comprising one or more switching elements 312(i), i = 1, 2, ..., I. The instrument 300 has one deflectable tip section 302 at the distal end, a steering unit 306 at the proximal end, and a body section 304 between the tip section 302 and the steering section 306. Reference number 308 refers to a bendable structure inside the tip section 302, which can be implemented by any suitable structure including one or more hinges cut in a tube from which also the body section 304 is made. For sake of easiness, 308 will be referred to as hinge 308. The tube has a central axis 310.

All sections 302, 304, 306 can be implemented while using more than one tube. I.e., the instrument can be made by several tubes inserted into each other. Body section 304 may be rigid. Alternatively, body section 304 may be flexible along its entire length, or comprise alternating flexible and rigid portions.

The instrument has one or more steering wires 16(i) each one attached to a suitable attachment location 307(i), inside tip section 302, e.g., at the distal side of hinge 308. At their proximal end, i.e. at the transition between body section 304 and steering section 306, each steering wire 16(i) is attached to one side of a switching element 312(i). Another side of switching element 312(i) can be moved to be attached to an end portion A(i), B(i), ... of two or more steering section wires 313(j), j = 1, 2, ..., J. Each one of the steering section wires 313(j) is attached to a predetermined location on a lever 314. Lever 314 is arranged in instrument 300 such that it can rotate about a center of rotation 316 on central axis 310.

As shown, each steering wire 16(i) is located at a certain radial distance from central axis 310 inside body section 304. The predetermined locations on lever 314 to which steering section wires 313(j) are attached have predetermined radial distances to a center of rotation 316, which may differ from the radial distance between steering wire 16(i) and central axis 310. E.g., in the shown example, steering section wires 313(1) and 313(2) are attached to lever 314 at a same first radial distance from central axis 310, whereas steering section wires 313(3) and 313(4) are attached to lever 314 at a same second radial distance from central axis 310. First and second radial distances are different. One of them may be equal to the radial distance between steering wire 16(i) and central axis 310 inside body section 304.

The first and second radial distances can be selected such that an amplification or an attenuation effect is obtained between an angle of deflection of lever 314 relative to central axis 310 and an angle of deflection of tip section 304 relative to central axis 310, as will be understood by a person skilled in the art. In the middle position, the wires 16(i) are detached from the steering device 306 and tip 302 can move freely when the instrument is guided through a curved entrance channel.

Here, a lever 314 is shown. However, the same switching element structure can be applied with a steering section 306 using discs or balls causing lever functions.

Figure 14A shows an alternative embodiment. The difference with the embodiment of figure 13 is that steering section wires 313(3) and 313(4) are not connected, at their proximal ends, to lever 314 but to a robotic steering unit 318. Robotic steering unit 318 can be implemented in any way known in the art (current and future). So, the implementation of figure 14A allows for switching steering wires between manual and robotic control.

In the embodiment of figure 14B, steering wires 313(1) and 313(2) are still connected, at their proximal ends, to lever 314 but steering section wires 313(3) and 313(4) are connected, at their proximal ends, to another lever 314(2).

In the configuration of figures 14A and 14B, the invasive instrument 300 as described above does not have a switching element mechanism that allows switching steering wires 16(i) from one amplification to another amplification, but that allows switching steering wires 16(i) from one steering unit to another steering unit. This could be useable for an instrument with two manual controls that can be used for training purposes, as shown in figure 14B. A trainer could insert the invasive instrument 300 into a body and could perform the operation during which he could switch the control of the instrument 300 to a student at any time without retracting the instrument or letting go of the controls.

When one wants to use an instrument 300 that is remotely steered by for example a robot or other electro-mechanical, hydraulic or pneumatic means, as shown in figure 14A, often a problem occurs in the preparation phase of the operation. For example, when one wants to insert a long, steerable endoscope in a human body one can choose to perform this insertion when the instrument is hooked up to the robotic steering unit 318. Then the robotic steering unit 318 must, sometimes, be able to advance the instrument 300 over a great length whilst steering the tip section continuously to follow the entrance trajectory which may be rather curved. In this case the robotic steering unit 318 needs to have huge positioning travels and will be a huge and complex piece of equipment. In most cases it is not necessary to use robotic steering unit 318 for entering into the human body and this can be done by hand as is common practice now. For example, entering a colonoscope into the bowel is a routine procedure that can be done by hand. Therefor it would be desirable to have a steerable colonoscope that can be steered and entered into the human body, following existing and widely accepted procedures, by hand. After entrance into the body, the instrument can be hooked up to robotic steering unit 318. Robotic steering unit 318 then could take over the steering of the instrument. This is possible with the arrangement shown in figure 14A. When doing so, the robotic steering unit 318 only has to be capable of small positioning travels and can be built more compact and less complex. There are many other examples where it would be useful to use this method, for example for gastroscopes entering the esophagous, bronchoscopes for entering the larynx and further down into the lungs, etc.

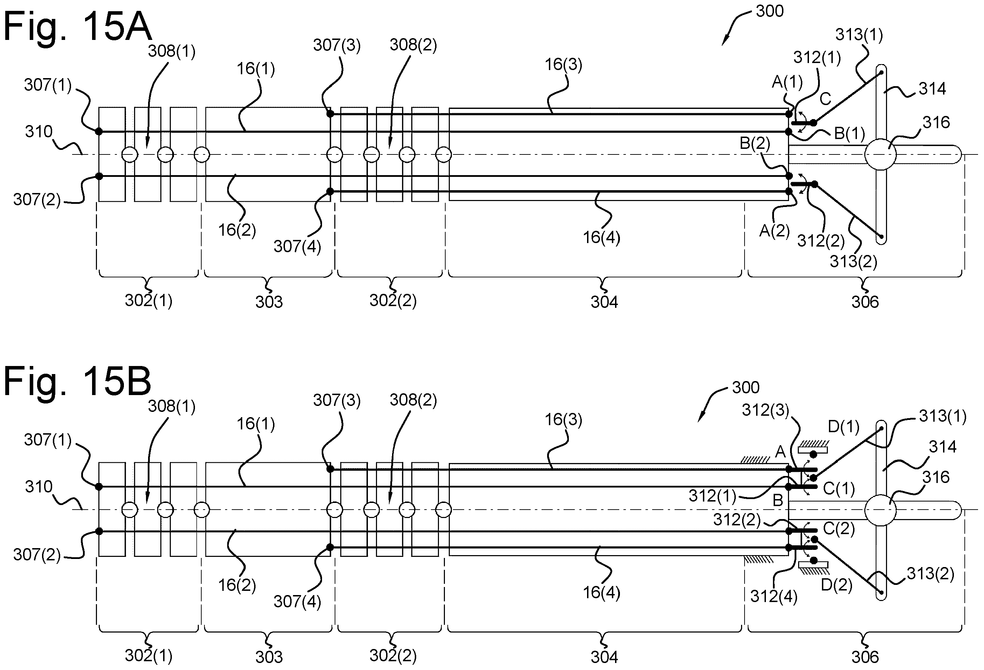

As described above, figures 5 -10 relate to invasive instruments with multiple deflectable tip section portions. An example relates to two deflectable tip section portions. The first, most proximal, tip section portion can be curved to create a so called 'triangulation' while the distal tip section portion can be steered to perform a desired operation. Invasive instruments like this need two steering devices, one for the most proximal tip section portion and one for the distal tip section portion. This complicates the instrument, more parts are needed, assembly is more complex, etc. Also the use of such an instrument might be difficult, i.e., the operator needs to manipulate two steering devices simultaneously, or he/she may need an extra operator or an external device to hold one steering device in a certain position whilst he/she manipulates the second steering device. This again makes the use of a steerable instrument with two steerable sections more difficult and expensive. Figures 15A, 15B address these issues.

Figures 15A and 15B relate to invasive instruments 300 having multiple, here two, deflectable tip section portions 302(1) and 302(2) separated by an intermediate tip section portion 303 which may be rigid. Deflectable tip section portions 302(1), 302(2) may be implemented by hinges 308(1), 308(2). Steering wires 16(1), 16(2) are attached to attachment locations 307(1), 307(2) at the distal side of hinge 308(1) and steering wires 16(3), 16(4) are attached to attachment locations 307(3), 307(4) at the distal side of hinge 308(2).

In figure 15A, steering section wire 313(1) can be switched by switching element 312(1) between being attached to either steering wire 16(1) or steering wire 16(3). Steering section wire 313(2) can be switched by switching element 312(2) between being attached to either steering wire 16(2) or steering wire 16(4). So, by deflecting lever 314 either tip section portion 302(1) or 302(2) can be deflected. This instrument allows an operator to control the deflection of both tip section portions 302(1) and 302(2) with one steering device, e.g., lever 314 as shown or with any other steering unit including a robotic device.

In figure 15B, at the proximal end of body section 304 switching element 312(1) has one end attached to steering wire 16(1) whereas its other end can be switched between being attached to steering section wire 313(1) or being in a non-attached state. At the proximal end of body section 304 a switching element 312(3) has one end attached to steering wire 16(3) whereas its other end can be switched between being attached to steering section wire 313(1) or being attached to a fixed location D(1) of instrument 300. Moreover, at the proximal end of body section 304 switching element 312(2) has one end attached to steering wire 16(2) whereas its other end can be switched between being attached to steering section wire 313(2) or being in a non-attached state. At the proximal end of body section 304 a switching element 312(4) has one end attached to steering wire 16(4) whereas its other end can be switched between being attached to steering section wire 313(2) or being attached to a fixed location D(2) of instrument 300.

The instrument according to figure 15B allows the following sequence of use. An operator sets switching element 312(3) and 312(4), respectively, such that steering wire 16(3) and 16(4), respectively, is attached to lever 314. Steering wires 16(1), 16(2) are then disconnected. The operator enters the instrument into the operation site, e.g., a human body. Then, by using lever 314 which operates steering wires 16(3) and 16(4), he/she steers tip section portion 302(2) to a desired deflected state, e.g. a desired triangulation geometry. Then, he/she switches switching element 312(3) and 312(4), respectively, to the fixed world location D(1) and D(2), respectively. So, the deflection of tip section portion 302(2), i.e., the articulation angle, is frozen in a desired position. Concurrently, switching element 312(1) and 312(2) is switched to be attached to steering section wire 313(1) and 313(2), respectively. Now, the operator can steer most distal tip section portion 302(1) as desired by using lever 314 which now controls steering wires 16(1), 16(2).

During the operation, the operator might control steering section wire 313(1) and 313(2), respectively, to be either attached to steering wire 16(1) or 16(3), and steering wire 16(2) or 16(4), respectively. Any time steering section wire 313(1) and 313(2), respectively, is attached to steering wire 16(1) and 16(2), respectively, steering wire 16(3) and 16(4), respectively, is attached to the fixed world such that the articulation angle of tip section portion 302(2) is frozen.

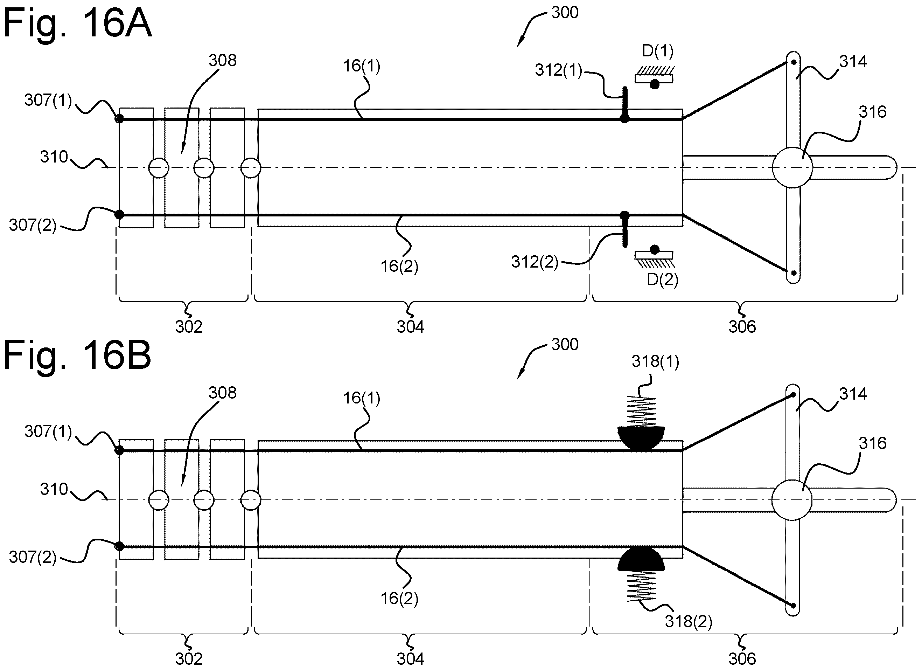

A switching feature would not only be usable in steerable invasive instruments for switching and freezing selected portions in the steerable tip section 302 as described above. A switching feature can also be used in steerable instruments for enhancement of other performance aspects. As explained above, the ability of 'freezing' of the deflection of one portion of the steerable tip section is a valuable feature for improving a steerable instrument's performance, ease of use and safety of use. But freezing of geometry can also be applied to other portions of the instrument or even to the whole instrument. The latter is important if, for example, a (support) instrument is used to grab tissue at a certain location in a body in which the instrument is inserted, then move this tissue to another location by steering the tip section of the instrument and then hold it in place whilst the operation is further performed with other instruments. In this case, it is valuable to have a switching feature in that support instrument with which the complete instrument, and the deflection thereof, can be frozen. In that way one can prevent that the operator needs a third hand or a device that holds the support instrument in a desired position. Freezing of a complete instrument's body and steerable sections can be done by applying switching features that connects steering (sets of) wires to the 'fixed world' and in this case these (sets of) steering wires can be, but do not need to be disengaged from the steering device. In this way, also the steering device is frozen in the desired position. Embodiments thereof are shown in figures 16A-16C.

Figures 16A-16C relate to a single deflectable instrument, like the one shown in figures 13, 14A, 14B. However, in multi-deflectable instruments the same principle can be applied as well. In the examples of figures 16A-16C, steering wires 16(1) and 16(2) are permanently attached to lever 314. However, once tip section 302 has been deflected in a desired state the deflected state can be frozen by switching steering wires 16(1) and 16(2) such that they cannot move in the longitudinal direction anymore.

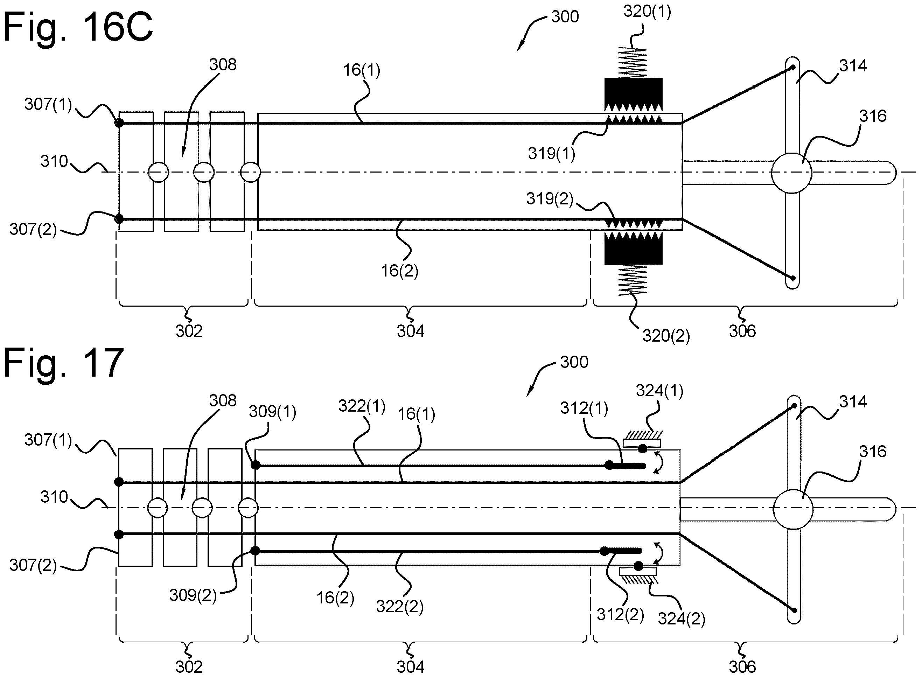

In figure 16A, an example is shown in which steering wire 16(1), 16(2) can be attached to the fixed world at location D(1), D(2) by switching element 312(1), 312(2). In figure 16C, steering wires 16(1), 16(2) are provided with a serrated structure 319(1), 319(2), e.g., at a location close to the transition between the body section 304 and the steering section 306. Moreover, a switching element 320(1), 320(2) is provided which has a serrated structure 319(1), 319(2) too at a side facing steering wire 16(1), 16(2). Both in the embodiment of figure 16A and 16C, once an operator has deflected tip section 302 to a desired state, this state can be frozen by switching steering wires 16(1) and 16(2). In the embodiment of figure 16A this would be accomplished by switching switching element 312(1), 312(2) to be attached to fixed world location D(1), D(2). In the embodiment of figure 16C, this can be achieved by moving the serrations of switching element 320(1), 320(2) towards the serrated structure 319(1), 319(2) of steering wire 16(1), 16(2) such that they are engaged and longitudinal movement of steering wire 16(1), 16(2) is blocked.

In the embodiment of figure 16B, steering wire 16(1), 16(2) cannot be attached to the fixed world in a hard, or on-off like manner. Here, a switching element 318(1), 318(2) is applied that is configured to apply friction to steering wire 16(1), 16(2) without really locking the steering wire. I.e., switching element 318(1), 318(2) can be used as a (friction) brake or a ratchet brake. The operator can manipulate the deflection of tip section 302 with lever 314 till the correct tip section deflection is achieved, during which the brake is off, i.e., clamp 318(1), 318(2) is not operative. Then he/she can apply a friction brake to steering wire 16(1), 16(2) by means of switching element 318(1), 318(2). Small adjustments of the frozen geometry can be done by simply deflecting lever 314 by forcing it through the applied brake friction forces or ratchet force.

In another configuration, shown in figure 17, body section 304 is flexible. Steerable instrument 300 has a (set of) steering wire(s) 16(i) to steer steerable tip section 302 of instrument 300. Moreover, instrument 300 has one or more control wires 322(k), k = 1, 2, ..., K each one being attached to an attachment point 309(k) in body section 304 just proximal of steerable tip section 302.

These wires 322(k) run through body section 304 to the proximal end of the instrument, e.g., to the transition area between body section 304 and steering section 306 where they are not attached and can move freely. When instrument 300 is passed through a curved entrance trajectory, e.g. in a human body, towards the operation site, body section 304 of the instrument may be bent and therefor the most proximal ends of the control wires 322(k) will move to a certain longitudinal position. A switching element 312(k) is provided for each control wire 322(k) at the proximal end of the instrument that is configured to attach these wires 322(k) in that certain position to the fixed world, here indicated with 324(k), or that can apply a brake to control wires 322(k). By switching on this attachment or brake function, one can now freeze flexible body section 304 up to the attachment points 309(k) of wires 322(k). But, in this frozen position of body section 304, tip section 302 is still steerable with the steering device in steering section 306 because steering wires 16(i) can still move longitudinally freely inside body section 304.

This has huge advantages when compared to current steerable flexible body instruments. When these prior art instruments pass the curved trajectory and their body section cannot be frozen, manipulation of the steering device usually also changes the body section curvature because, mechanically spoken, a flexible body section is also a 'steerable tip section'. This usually results in the situation that steering of the instrument tip also partially steers the body section of the instrument and this steering of the body section can result in unwanted movement or force generation on surrounding tissue. This can be very unwanted or unsafe for example when the instrument passes through delicate tissues like lung bronchi or brain tissue. With the ability of freezing the instrument body section 304, one can prevent unintended steering and unintended movement or force loading of surrounding tissue. Another advantage is that when the instrument body is frozen, the steering of the tip can be performed more precise and is more predictable because the end of the instrument body is now in a fixed 3d position instead of moving rather unpredictable as a result of tip steering.

Of course one can have any number of active (sets of) steering wires 16(i) and any number of passive (sets of) steering wires 322(k) in a rigid body or flexible body or hybrid body instrument. Also the attachment points 309(k) of the control wires can be positioned anywhere along the length of instrument 300.

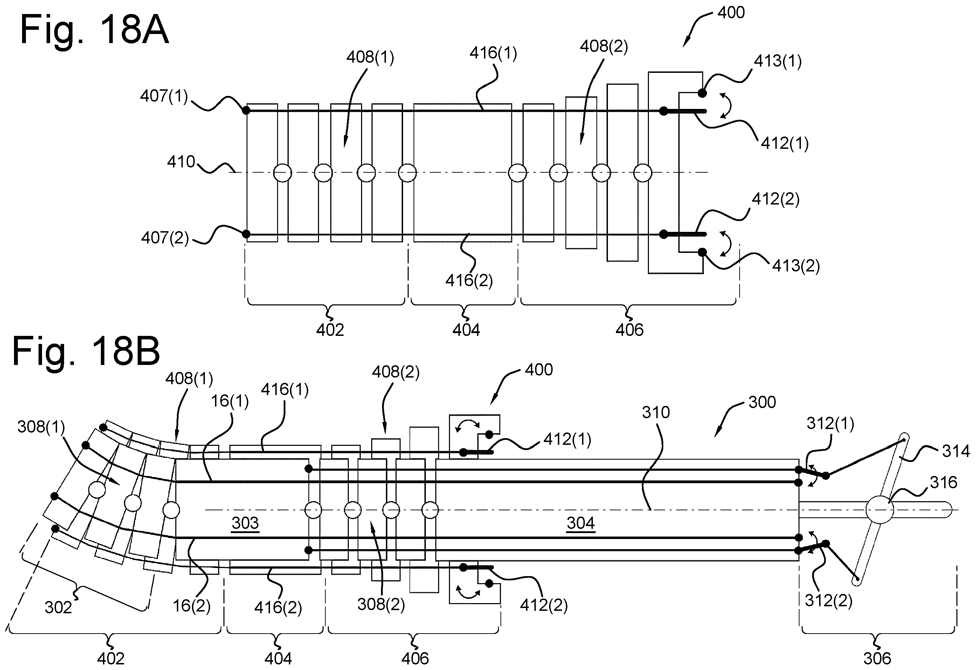

The concept of applying passive (sets of) steering wires can also be applied to an instrument without a steerable tip and this instrument could then function as an introducer or trocar for an instrument with a steerable tip, that takes the shape of the entrance trajectory and then can be frozen in that shape. This will be further explained with reference to figures 18A - 19B.

Figure 18A shows a schematic drawing of an example of such a trocar 400. It is shown to be bendable in one surface only, i.e., the surface of the drawing. However, as is evident to persons skilled in the art it can be made flexible in all directions by proper design of the hinges.

Trocar 400 comprises a distal flexible section 402 and a proximal flexible section 406, as well as an intermediate section 404 between both flexible sections 402, 406. Distal bendable section 402 may be implemented by a hinge structure 408(1) which may be made by cutting a suitable pattern of one or more slots in a tube. Intermediate section 404 may be rigid or flexible. If flexible, intermediate section 404 may be less flexible than distal flexible section 402 and proximal flexible section 406.

Proximal bendable section 406 may be implemented by a hinge structure 408(2).

Trocar 400 has a central axis 410 and may be made from any of the materials which can also be used for instrument 300 and are mentioned above. Trocar 400 defines a lumen through which another instrument, e.g. instrument 300 can pass. In the embodiment shown, this channel broadens slightly towards the proximal end inside proximal flexible section 406.

Trocar 400 is provided with one or more locking elements 416(m), m = 1, 2, ..., M which have the form of control wires. Each locking element 416(m) can be attached by a switching element 412(m) to a proximal attachment point 413(m), which may be located at the proximal end of trocar 400. Once trocar 400 is inserted into a body, distal flexible section 402 and proximal flexible section 406, as well as body section 404 if it is flexible, may have taken a bent orientation due to the inside environment of the body. That status can be frozen by activating switching elements 412(m) such that locking elements 416(m) are attached to attachment points 413(m) and cannot move longitudinally anymore. Instead of a fixed attachment a connection can be made which would provide some friction or braking to locking element 416(m).

Trocar 400 can, alternatively, be steered by a separate steerable instrument like instrument 300 that is placed in the lumen of the trocar. Initially, as shown in figure 18B, steerable tip section 302 of the steerable instrument can be lined up in the longitudinal direction with distal flexible section 402 of trocar 400. In that configuration, steering section 306 of instrument 300 can be used to steer steerable tip section 302 of steerable instrument 300 which would then actively steer tip section 406 of trocar 400. Once the desired shape is reached, tip section 402 of trocar 400 can be frozen by coupling locking elements 416(m) to a locked position as explained above.

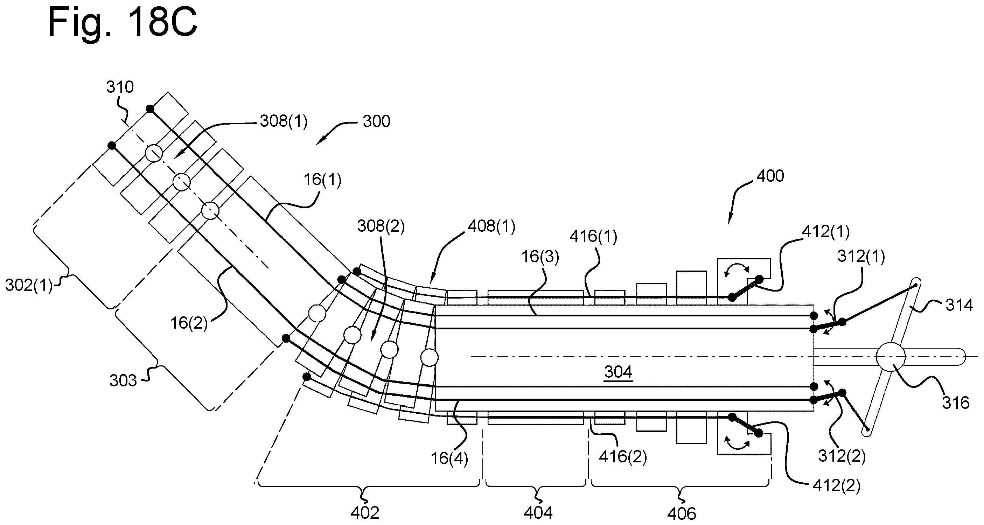

Then, as shown in figure 18C, steerable instrument 300 can be advanced further through trocar 400 that now acts as a shaped guiding tube (assuming body section 303 to be flexible). Moreover, steerable instrument 300 may have two (or more deflectable) tip sections 302(1), 302(2). In the example shown, the most proximal tip section 302(2) may be advanced until it is longitudinally aligned with distal flexible section 402 of trocar 400. In this way, its articulation angle is defined by the frozen deflection of distal flexible section 402 of trocar 400. In the status shown in figure 18C, if desired, switching elements 412(m) may be decoupled temporarily such that the articulation angle of most distal tip section 302(2) may be adapted by steering most distal tip section 302(2) by means of steering section 306. This may be done by a single steering device 314 which can be coupled to either steering wires 16(1), 16(2) or steering wires 16(3), 16(4) by switching elements 312(1), 312(2), as shown in figures 18B and 18C.

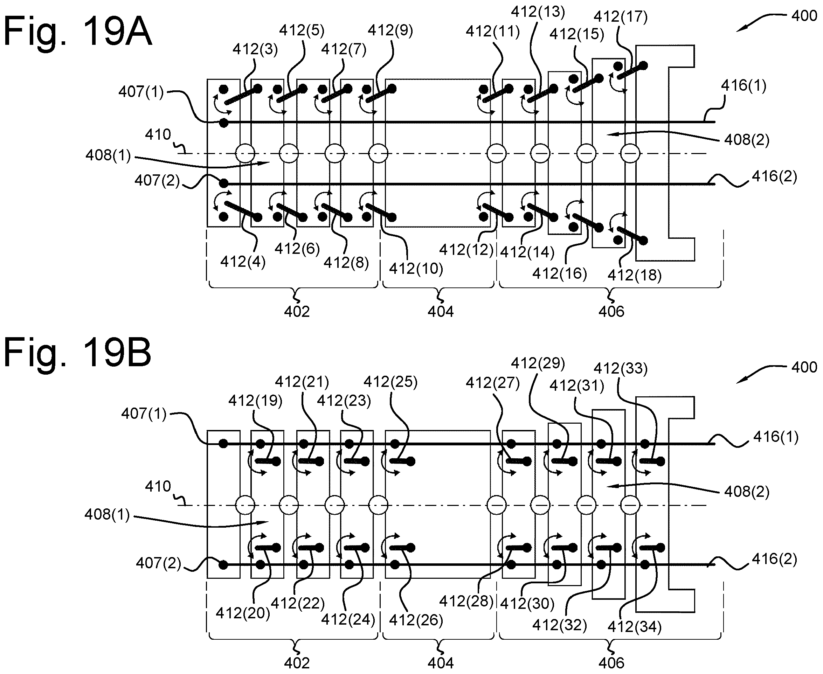

As described above, a certain curvature of a steerable instrument section or a flexible section of a non-steerable instrument can be frozen by locking active or locking elements in a fixed position. This method of freezing, however, still allows S-shape (or other) deformations of the curve itself, because only the length of the active or locking elements is fixed. Within that length different hinge deflections are still possible as long as the total length of the hinges and room between them is constant. Figures 19A, 19B show embodiments in which an entire curved shape of an instrument can be frozen. These figures show a trocar but the same principle is applicable to steerable instruments 300.

Figure 19A shows trocar 400 with a plurality of individual hinge segments inside hinge 408(1), as well as a plurality of hinge segments inside hinge 408(2). Between consecutive hinge segments inside hinge 408(1) one or more switching elements 412(3) - 412(8) are provided. One or more switching elements 412(9) and 412(10) are provided between body segment 404 and the most proximal hinge segment of hinge 408(1). Moreover, alternatively or additionally, one or more switching elements 412(11), 412(12) are provided between body section 404 and the most distal hinge segment of hinge 408(2). Between consecutive hinge segments inside hinge 408(2) one or more switching elements 412(13) - 412(18) may be provided. All switching elements 412(3) - 412(18) can be individually activated or in one or more groups to either clamp or un-clamp. Once in a desired shape, switching elements 412(3) - 412(18) can be activated to lock a current mutual orientation of adjacent hinge segments and, thus, freeze trocar 400 in that shape. The complete curvature is now frozen, because each individual hinge is frozen in its rotation position.

In the embodiment of figure 19B, switching elements 412(3) - 412(18) are substituted by switching elements 412(19) - 412(34). I.e., one or more hinge sections in distal flexible section 402 can be clamped by one of these switching elements 412(19), 412(21), 412(23) to locking element 416(1) and/or by one of these switching elements 412(20), 412(22), 412(24) to locking element 416(2). Also body section 404 may be clamped to locking element 416(1) by switching element 412(25) and/or to locking element 416(2) by switching element 412(26). Alternatively or additionally, one or more hinge sections in steering section 406 can be clamped by one of these switching elements 412(27), 412(29), 412(31), 412(33) to locking element 416(1) and/or by one of these switching elements 412(28), 412(30), 412(32), 412(34) to locking element 416(2). All switching elements 412(19) - 412(34) can be individually activated to either clamp or un-clamp. Once in a desired shape, switching elements 412(19) - 412(34) can be activated to freeze trocar 400 in that shape. Also now the complete curvature is frozen, because each individual hinge is frozen in its rotation position.

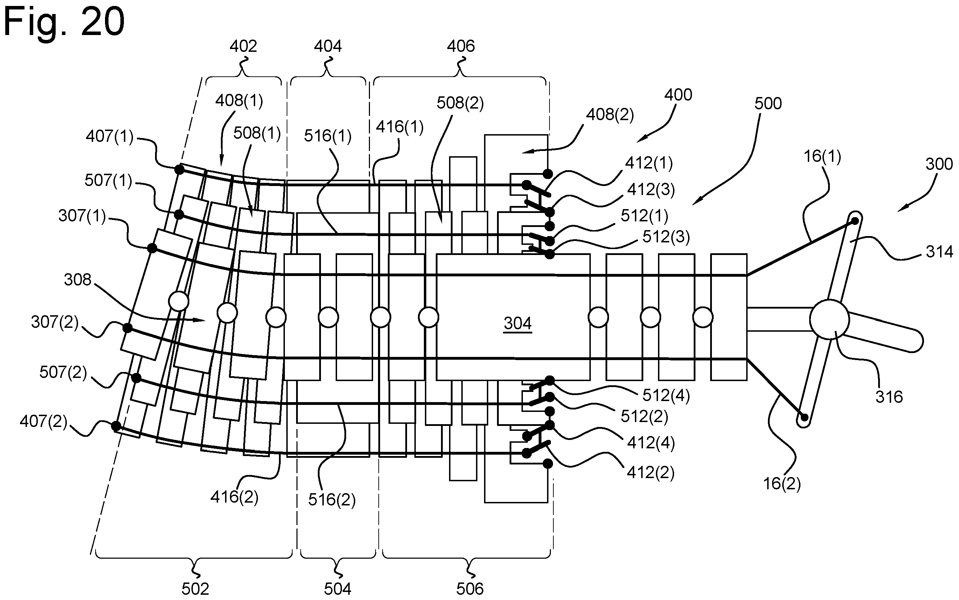

With configurations as in figures 18A-18C, and 19A, 19B, one could also build a telescopic device containing two or more non-steerable instruments or trocars, which can be steered by one steerable instrument sequentially. In this way one can create a curved guiding tube with two or more curves at any length location. This is explained with reference to figure 20.

Figure 20 shows a first trocar 400 surrounding a second trocar 500 which, in turn, surrounds steerable instrument 300. Second trocar 500 comprises a distal flexible section 502 and a proximal flexible section 506, as well as a body section 504 in between them. Both distal flexible section 502 and proximal flexible section 506 may be made by suitable hinge structures 508(1) and 508(2), e.g. implemented by suitable slot patterns in a tube. Body section 504 may be rigid or (partly) flexible. Switching elements 412(1) and 412(2) can clamp locking element 416(1), 416(2) of first trocar 400 to proximal flexible section 406 of trocar 400 whereas switching elements 512(1) and 512(2) can clamp locking element 516(1), 516(2) of second trocar 500 to proximal flexible section 506 of trocar 500. Switching elements 412(3), 412(4) can couple a most proximal segment of first trocar 400 to a most proximal segment of second trocar 500. Switching elements 512(3), 512(4) can couple a most proximal segment of second trocar 400 to instrument 300, e.g., to its body section 304. Activation and deactivation of switching elements 412(1), 412(2) is synchronized with activation and deactivation of switching elements 412(3), 412(4). Moreover, activation and deactivation of switching elements 512(1), 512(2) is synchronized with activation and deactivation of switching elements 512(3), 512(4).

Figure 20 shows the starting position. In this starting position, the steerable tip section 302 of steerable instrument 300 is longitudinally lined up with distal flexible sections 402, 502 of trocars 400, 500. Moreover, all switching elements 412(1), 412(2), 512(1), 512(2) are deactivated and do not clamp. Switching elements 512(3) and 512(4) are activated and hold instrument 300 in a fixed longitudinal position in trocar 500 and switching elements 412(3) and 412(4) are activated and hold trocar 500 in a fixed longitudinal position in trocar 400. Trocars 400, 500 together with steerable instrument 300 can now be moved in its entirety into, for example, a body lumen with a certain curvature. At a first curve in that lumen, steering device 314 of steerable instrument 300 can now be used to steer distal flexible sections 402, 502 of trocars 400, 500 in a required curvature by steering tip section 302 in a desired deflection. Now switching elements 412(1), 412(2) are activated and locking elements 416(1), 416(2) of instrument 400 are locked in place. At the same time, or at the operator's discretion, activation of switching elements 412(3), 412(4) releases the connection between instrument 400 and 500 and the assembly of steerable instrument 300 and non-steerable instrument 500 can be advanced further into the curved trajectory while trocar 400 remains at its position. For freezing in a second curve in the trajectory, switching elements 512(1), 512(2) are switched such that locking elements 516(1), 516(2) of trocar 500 are locked in place. At the same time, or at the operator's discretion, switching elements 512(3) and 512(4) are switched and release the connection between trocar 500 and steerable instrument 300 and steerable instrument 300 can now be further advanced through the curved trajectory. This can also be done with three or more layers/tubes of non-steerable instruments and with steerable instruments with two or more steerable sections. It is also observed that the number of locking elements 416(1), 416(2), 516(1), 516(2) can be different than the one shown here.

Another way of creating a multi curved guiding tube or instrument body is to apply multiple individually steerable sections in one instrument that can also be frozen individually and each individual curve could be frozen by locking the steering element at it's end or at locations per individual hinge with combining switching element configurations as in figures 16 thru 20.

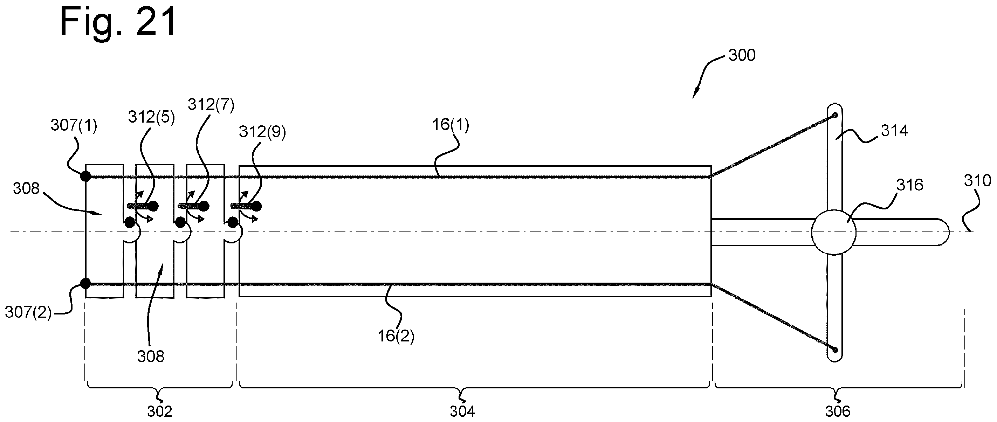

As described above, switching elements can be provided that are operated by a user to manipulate the attachment of active steering wires and locking elements to freeze the curvature of steerable and non-steerable instruments, or freeze this curvature by freezing individual hinges. Alternatively, one can use a different switching mechanism to accomplish the same. I.e., can also freeze the curvature of a steerable and non-steerable instrument by blocking the rotation of the hinge-like structures at the rotation point itself. This is schematically indicated in figure 21.

Figure 21 shows steerable instrument 300, which may be realized through the current invention as defined by the appended claims may be applied. Two consecutive hinge sections of hinge 308 can be frozen at a desired relative rotation angle by locking a first one of them to the second one of them by activating a switching element 312(5), 312(6), 312(7) located at or close to the point of rotation. Moreover, a switching element 312(9) may be activated to block a point of rotation between a most proximal hinge segment of hinge 308 and body section 304.

In the examples described above, the desired performance can be selected as many times as one would like by operating the switching mechanism back and forth between two, or more, discrete switching element positions. However, one can also make a clamp feature that can be operated only once, for example to permanently set a new neutral position of the steering handle. Also in the examples described above, the switching elements have discrete positions, but in for example switching elements that generate friction instead of a keyed or fixed connection, one can also create switching elements with variable positions with which the amount of friction is regulated proportionally. Next to that, in the examples described above it is assumed that the clamp, for example, connects a steering element to the fixed world, or that switches a steering element from one connected position to another connected position, that this position is fixed. But one can also create a switching element that connects one element to another in a way that one element can still move relative to the other element, but in which that movement is limited to a certain range. It is also obvious that the described performance features and the associated switching element functions can be used in any combination and location in a steerable or non-steerable instrument. The examples described above only describe a small selection of all possible combinations of performance aspects and functionality and locations of the associated switching element features.

However, all of the example instruments described above have in common that they can be realized with more or less generic switching element features that can be categorized, looking to their functionality, as follows:
- a switching element with an 'on-off' function,
- a switching element with a switching function, with or without a neutral position,
- a switching element with a movement limiting function,
- a switching element with a friction or ratcheted braking function, either 'on-off' or proportional,
- a switching element that can be operated one time, with or without an auto lock function,
- any combination of a) thru e) in one switching element mechanism or multiple switching element mechanisms that are activated with one user control feature.

Now, a plurality of switching element examples will be described with reference to figures 22A to 47D. It is observed that the presentation of figures 22A to figure 40 is in a 2D surface but that their implementation is 3D in reality. I.e., the components shown in these figures are, in reality, made from one or more tubes such that they are part of the curved wall of one or more of such tubes. Thus, movements of elements in the "up direction" or "down direction" are in the tangential direction in the real physical implementation. Horizontal movements in these figures are in the longitudinal (or axial) direction of the tube(s).

In figure 22A, reference numbers 602, 604 refer to "fixed world" portions, i.e., fixed portions of instrument 300. Instrument 300 comprises a longitudinal element 606, e.g. a steering wire, extending in the longitudinal direction. Longitudinal element 606 comprises a less wider portion 634 and an extension 630 which has a face 632 towards less wider portion 634. Extension 630 is located opposite to fixed world portion 602.

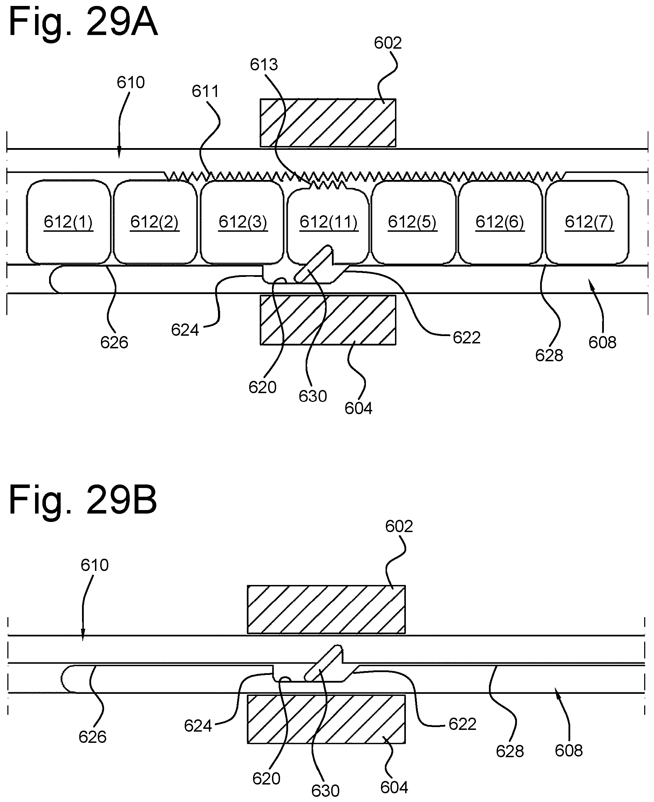

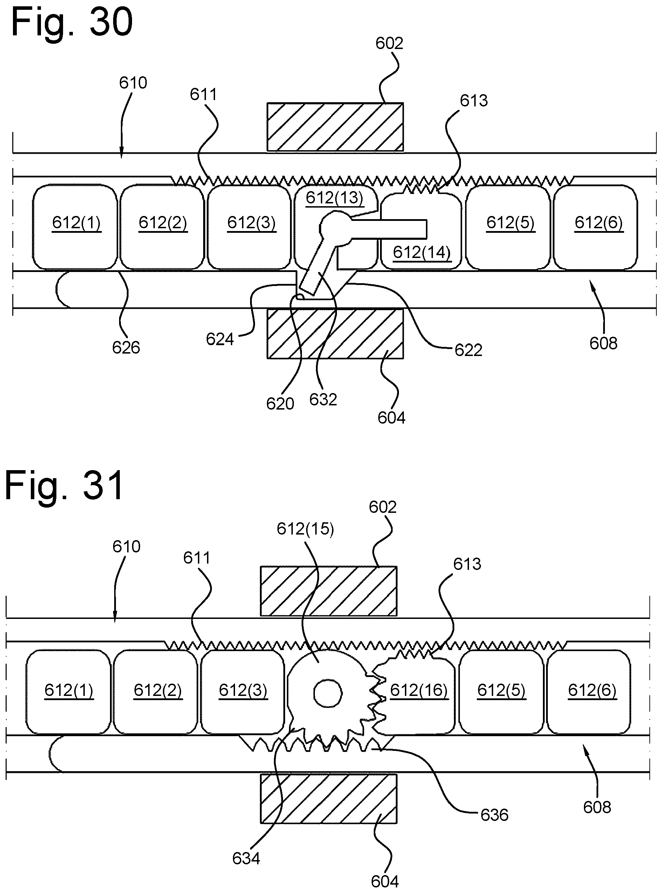

A longitudinal activation element 608 has an extension 626 which has a face 624 towards one end of a less wider portion 620. At another end, less wider portion 620 is provided with a further extension 628. There is a face 622 between less wider portion 620 and further extension 628. Faces 622 and 624 are both angled relative to the longitudinal direction of the instrument such as to form a slope to be used to transform a longitudinal movement of actuation element 608 into a tangential movement of a locking element. The angle may deviate from 90 degrees but in some embodiments it is 90 degrees (e.g. figures 29A, 29B, 30).

In the non-activated state, less wider portion 620 is located between extension 630 and fixed world portion 604. When activation element 608 is pulled or pushed in the right direction, the faces 624, 632 will push extension 630 towards fixed world portion 602 and extension 626 towards fixed world portion 604 which limit the movement in the up-down direction. As a result, longitudinal element 606 will be clamped and its longitudinal movement is prohibited.

A same clamping force will be generated if one pulls or pushes activation element 608 in the left direction because, then, face 622 of extension 628 will push extension 630 towards fixed world portion 602 and extension 628 towards fixed world portion 604 such that longitudinal element 606 is clamped against fixed world portion 602 and cannot move in the longitudinal direction anymore or only with a certain amount of friction.

This clamping can be released by moving activation element 608 in the opposite direction as the direction used for the clamping.

In this example, the longitudinal force exerted on activation element 608 is transferred to longitudinal element 606. So, longitudinal movement of longitudinal element 606 as a result of this force has to be prohibited by features or mechanisms in the instrument that hold longitudinal element 606 in position during activation of the switching element mechanism. The longitudinal force may be exerted from the proximal end of instrument 300, either generated manually or generated by a suitable robotic instrument.

Figure 22B shows an alternative to the example of figure 22A. Here, longitudinal element 606 is implemented as a lever rotatable about a rotation axis 636. Lever 606 functions as a clamp. Instead of a rotation axis, an attachment location 636 can be used e.g. made by welding lever 606 to an underlying portion of instrument 300. If so, the less wider portion 634 is, preferably, flexible such that extension 630 can move towards and away from fixed world portion 602.

A longitudinal element 610 is located between lever 606 and fixed world portion 602. Longitudinal element 610 may be a steering wire 16(i).

By pushing or pulling activation element 608 in the right or left direction, respectively, extension 630 will be moved towards fixed world portion 602 by face 624 or 622, respectively, and, as a result, clamp longitudinal element 610 against fixed world portion 602 such that longitudinal element 610 cannot move in the longitudinal direction anymore. Again, the clamping can be released by moving activation element 608 in the position where its less wider portion 620 is in between extension 630 and fixed world portion 604, as shown in figure 22B.

In the embodiment of figure 22B, longitudinal forces exerted by activation element 608 during a clamping activation will be transferred to lever 606 and not directly to longitudinal element 610.

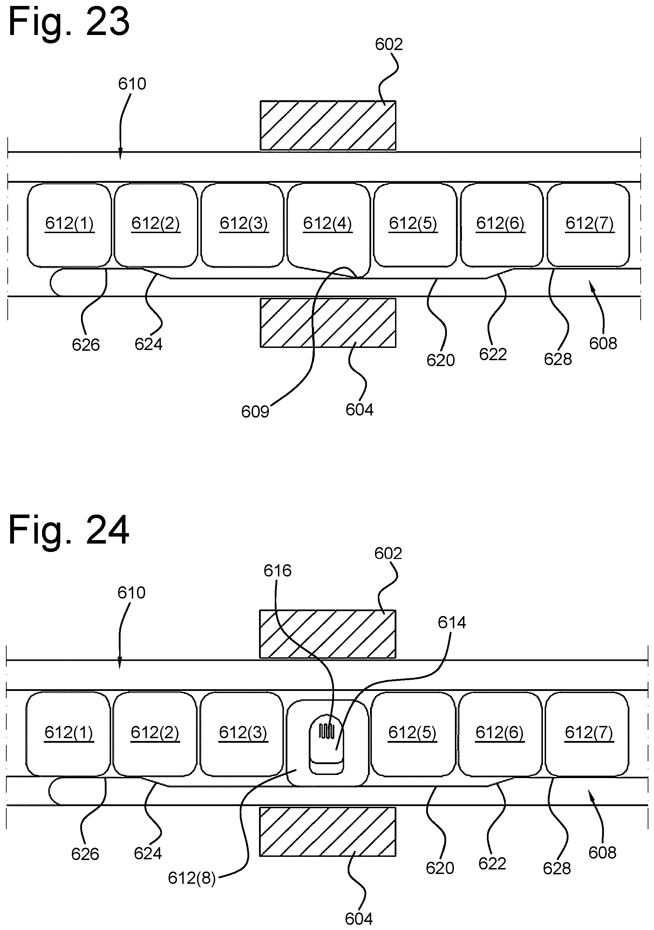

Instead of lever 606 for absorbing the longitudinal force, a sliding element can be used that is sliding in a direction perpendicular to the longitudinal direction, i.e. the tangential direction, of instrument 300. Such sliding element is configured to be movable towards longitudinal element 610 such that longitudinal element 610 can be clamped against fixed world portion 602. Moving the sliding element away from the longitudinal element 610, then, releases longitudinal element 610. Examples are explained with reference to figures 23-35. In these embodiments, a switching element is implemented as a sliding element or a lever.

Figure 23 shows an embodiment in which a sliding element 612(4) is arranged between two other elements 612(3) and 612(5). These latter elements 612(3), 612(5) are fixed relative to the fixed world such that sliding element 612(4) cannot move in the longitudinal direction of instrument 300. One or more elements 612(1), 612(2) may be present on the longitudinal side of element 612(3) opposite to the longitudinal side of element 612(3) facing sliding element 612(4). One or more elements 612(6), 612(7) may be present on the longitudinal side of element 612(5) opposite to the longitudinal side of element 612(5) facing sliding element 612(4).

Longitudinal element 610 is, again, extending in the longitudinal direction of instrument 300. Also activation element 608 is, again, extending in the longitudinal direction of instrument 300, e.g., towards the proximal end of instrument 300 from where it is operated. In the example, all elements 612(1) - 612(7) are configured to operate as spacers between longitudinal element 610 and activation element 608.

Sliding element 612(4) is provided with an extension 609 opposite to activation element 608. By pushing or pulling activation element 608 in the right or left direction, respectively, extension 609 and thereby sliding element 612(4) will be moved towards fixed world portion 602 by face 624 or 622, respectively, and, as a result, clamp longitudinal element 610 against fixed world portion 602 such that longitudinal element 610 cannot move in the longitudinal direction anymore or only with a certain amount of friction. Again, the clamping can be released by moving activation element 608 in the position where its less wider portion 620 is in between extension 609 and fixed world portion 604, as shown in figure 23.

Figure 24 shows an alternative to the embodiment of figure 23. Here, there is a sliding element 612(8) between elements 612(3) and 612(5), which is provided with an opening in which a guiding element 614 is located. Guiding element 614 is attached to the fixed world of instrument 300 at a point of attachment 616, e.g. by (laser) welding guiding element 614 to an underlying or overlaying portion - like a tube - of instrument 300. Sliding element 612(8) is configured to be sliding towards and away from fixed world portion 602 as guided by guiding element 614. I.e., by pushing or pulling activation element 608 in the right or left direction, respectively, sliding element 612(8) will be moved towards fixed world portion 602 by face 624 or 622, respectively, and, as a result, clamp longitudinal element 610 against fixed world portion 602 such that longitudinal element 610 cannot move in the longitudinal direction anymore or only with a certain amount of friction. Again, the clamping can be released by moving activation element 608 in the position where its less wider portion 620 is in between sliding element 612(8) and fixed world portion 604, as shown in figure 24.

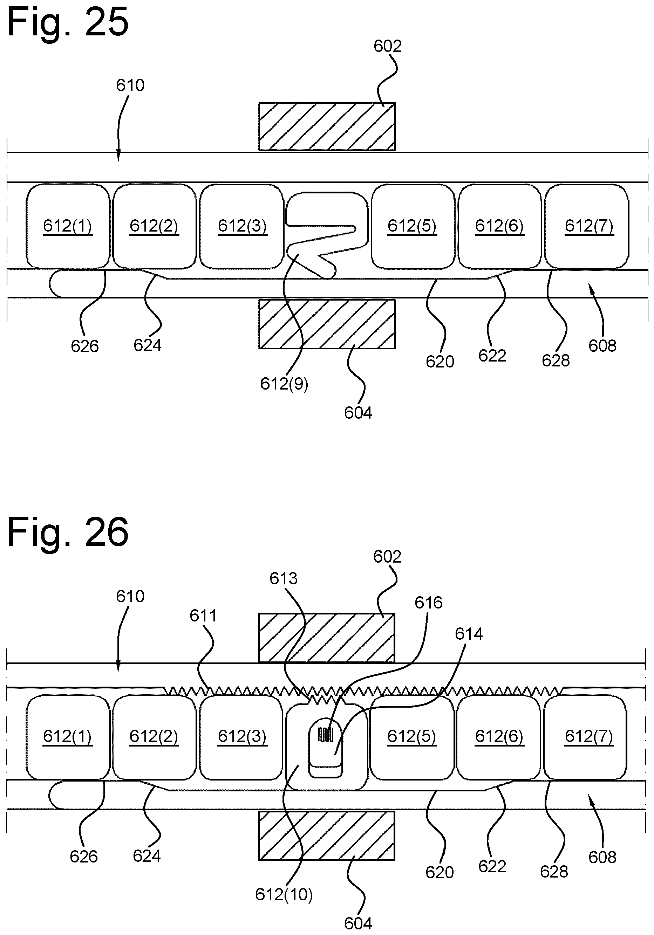

All above switching elements generate a normal force to the side of element 606/610, and therefor generate a friction force that holds element 606/610 in position. This friction force directly depends on the shape of the face 622, 624 and how strong the activation element 608 is pushed or pulled. If one would like more control over the maximum friction force and no dependency of how strong and how far the activation element 608 is displaced, one can use a mechanism as in figure 25. Now a sliding element 612(9) is provided between two elements 612(3), 612(5) which act as the fixed world. Sliding element 612(9) is equipped with an elastic portion which acts like a spring. The maximum normal force that can be generated with activation of element 608 is now only dependent on the shape and material properties of this spring element and its compression amount. One could now also use the displacement of activation element 608 to proportionally regulate the normal force. Sliding element 612(9) can have any shape, as long as it behaves in a spring like fashion.

All of the above mechanisms clamp or brake the (longitudinal) movement of element 606/610 with a friction force. For obtaining that friction force high normal forces and therefore relatively high activation forces might be needed. To obtain a clamping that holds that element firmly in place, one can use a 'form closed' or keyed connection as in figure 26. In the example of figure 26, longitudinal element 610 is provided with teeth 611 that may have the form of a triangle or a saw tooth. Also the sliding element 612(10) is provided with teeth 613. Once sliding element 612(10) is pushed towards longitudinal element 610, teeth 613 of sliding element 612(10) engage teeth 611 of longitudinal element 610 prevents any longitudinal movement of longitudinal element 610. Whilst in the above mechanisms according to figures 22A-25 longitudinal element 610 can be clamped in any random position, the use of teeth results in switching longitudinal element 610 in a limited number of discrete positions. However, if the size of the teeth or peak to peak distance between adjacent teeth is small, this results in sufficient switching position accuracy.

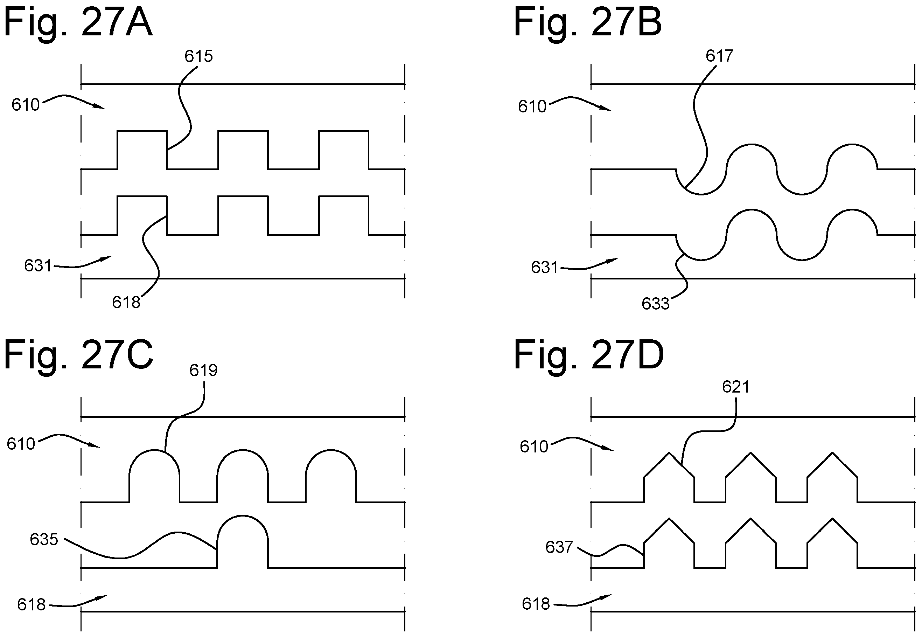

In figure 26, teeth 611, 613 are shown to have a triangle form. However, other teeth forms may be used. Figures 27A-27H show different possible serrations, though others may be envisaged.

Figure 27A shows block shaped serrations 615, 631 in both longitudinal element 610 and an actuation element 618 which have a high longitudinal fixation ability in combination with a low activation force. Trade off may be that positioning freedom is low, i.e., longitudinal element 610 can only be clamped in discrete steps with a length equal to the distance between two consecutive blocks. Note that 618 may, here, also refer to an intermediate element between an actuation element 608 and longitudinal element 610.

Figures 27B, 27C and 27D show the same as figure 27A, but now with self-centering properties. I.e., in figure 27B serrations 617 of longitudinal element 610 have a repetitive shape with semi-circular extensions and semi-circular indentions. Also serrations 633 of actuation element 618 have a repetitive shape with semi-circular extensions and semi-circular indentions. Serrations 619 in longitudinal element 610 in figure 27C comprise indentions with a curved bottom. Serrations 635 of actuation element 618 comprises one or more extensions with a curved front end and which match the profile of the indentions 619 in longitudinal element 610. In figure 27D, serrations 621 of longitudinal element 610 comprise indentions with a triangle shaped bottom, whereas serrations 637 of actuation element 618 comprise extensions with triangle shaped front surfaces which match the profile of serrations 621. In the examples of figures 27B-27D, the serrations in longitudinal element 610 do not have to be lined up with the serrations in activation element 618 upon activation.

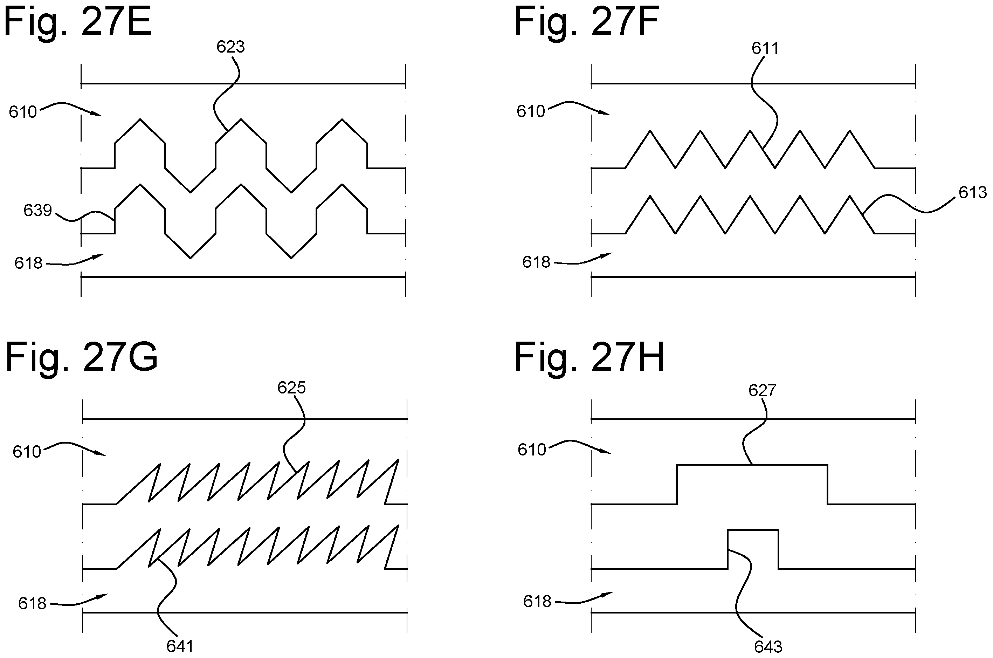

Figure 27E shows serrations 623 in longitudinal element 610 with the same indentions as in figure 27D but now also with extensions that have a triangle shaped front surface. Actuation element 618 has identically shaped serrations 639. Thus, this example is also self-centering, but when longitudinal element 610 is longitudinally loaded with a force (e.g. because it is a steering wire), no reaction force is generated perpendicular to the load. In switching elements like in figures 22A -26 this might lead to longitudinal friction forces. When a serration like in figure 27B and 27F is applied to a mechanism as shown in figure 25, one can balance the shape of the serrations and the spring force to obtain a ratcheting clamping. This mechanism would clamp longitudinal element 610 to a certain maximum longitudinal force, but beyond that force the switching block would we pushed open and longitudinal element 610 would reposition to the next serration.

Figure 27F shows serration geometry with triangle shape 611. 613. Serration 625 in longitudinal element 610 as shown in figure 27G has a saw tooth shape. Also serration 641 in actuation element 618 has a saw tooth shape. Saw tooth shapes can withstand high loads in one longitudinal direction but can only withstand a lower ratcheting load in the other longitudinal direction.

Figure 27H shows a serration 627 inside longitudinal element 610 with a block shaped indention. Serration 643 of actuation element 618 has a block shaped extension with a smaller longitudinal dimension than the longitudinal dimension of block shaped indention 627. Thus, when block shaped extension 643 is moved inside blocked shaped indention 627 longitudinal movement of longitudinal element 610 is still possible but limited to the longitudinal dimension of block shaped indention 627 minus the longitudinal dimension of block shaped extension 643.

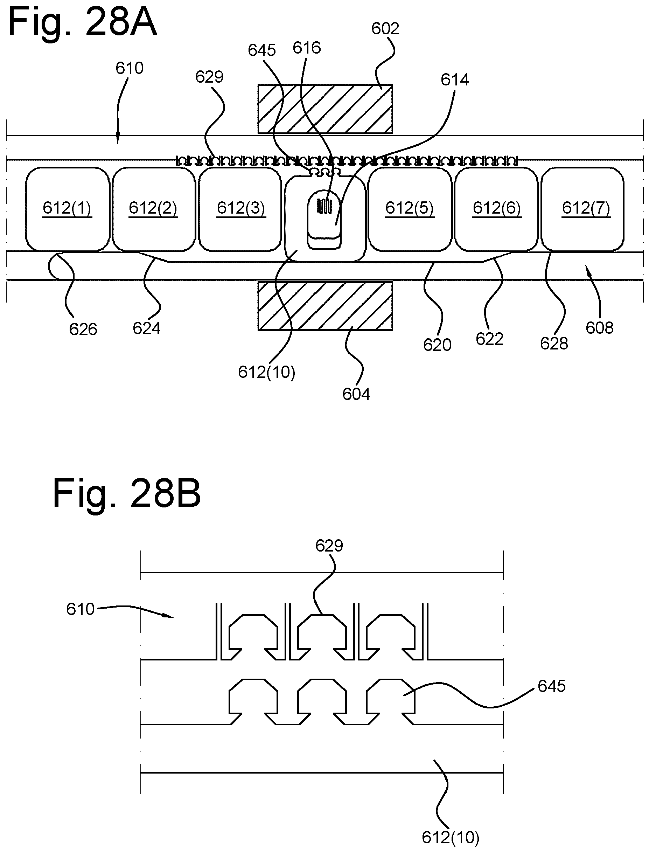

The embodiment shown in figures 28A, 28B is identical to the one shown in figure 26 apart from sliding element 612(10) and longitudinal element 610, respectively, having other serration forms 645 and 629, respectively. I.e., serrations 629, 645 are configured as snap-fit connections. Figure 28B shows an enlarged view of these serrations 629, 645. Serrations 629, 645 are shaped such that once they engage one another they form a snap-fit connection which cannot be disengaged anymore. This type of switching element and serration can be used if certain properties or dimensions in instrument 300 need to be set one time, for example at the start of a procedure to set a zero position of a steering handle or to calibrate a certain performance feature. Numerous types of self-holding or automatically locking geometries can be envisioned.

Figures 22A - 28B show switching mechanisms that are activated by actuation element 608/618 that translates a longitudinal movement into a tangential (possibly, back and forth) activation of the switching mechanism. Obviously there are many more solutions for activation of switching mechanisms.

Figure 29A, for instance, shows a floating lever 630 having one side located inside a space defined between extensions 626 and 628 of actuation element 608 and another side inside a sliding element 612(11). Sliding element 612(11) is provided with teeth 613 and longitudinal element 610 is provided with corresponding teeth 611. Lever 630 will be forced to rotate by face 624 to a wider dimension in a direction perpendicular to the longitudinal direction and towards longitudinal element 610 if actuation element 608 is moved to the right. Consequently, sliding element 612(11) will move towards longitudinal element 610 and teeth 613 will engage teeth 611 such that longitudinal element 610 is clamped, at least in its longitudinal movement. Figure 29B shows another embodiment of this mechanism, but now longitudinal element 610 is clamped directly by lever 630, without sliding element in between.

Figure 30 shows a variant to the one shown in figure 29A. A lever 632 is provided which is rotatably arranged about a rotation point inside a spacer 612(3) between longitudinal element 610 and actuation element 608. Lever 632 has two arms. One arm is operated by actuation element 608, e.g. by face 624 and/or 622. The other arm is connected to a sliding element 612(14) which upon actuation by actuation element 608 moves toward longitudinal element 610. Sliding element 612(14) is serrated with teeth 613 which then engage teeth 611 of longitudinal element 610 such that longitudinal element is blocked from any longitudinal movement.

Figure 31 shows a further variant in which actuation element 608 is provided with teeth 636 and an element 612(15) is provided between longitudinal element 610 and actuation element 608. Element 612(15) is also serrated with teeth 634 which force element 612(15) to rotate once actuation element 608 makes a longitudinal movement. Teeth 634 also engage teeth of a sliding element 612(16) such that sliding element 612(16) is moved towards longitudinal element 610. Again, teeth 613 of sliding element 612(16) engage teeth 611 of longitudinal element 610 such that it cannot move longitudinally anymore.

Figures 22A-31 show more or less 2D mechanisms that can be made completely in one plane or one wall of a tube. Although some of the elements of these mechanisms need to be attached to underlying or overlaying layers or tube walls, all parts of the mechanism are manufactured by material removal from one tube wall. To obtain more design freedom, or to create more space for parts or to optimize strength or accuracy of the switching element mechanism, one can also use multilayer mechanisms in which for example some parts of the mechanism are manufactured in a first layer/tube and some parts are manufactured in a second layer/tube. When these layers/tubes are placed on top of each other and interlayer connections, for example via laser welds, are made in the required locations, one can create more or less 3D mechanisms. Some 3D examples will be explained hereinafter.

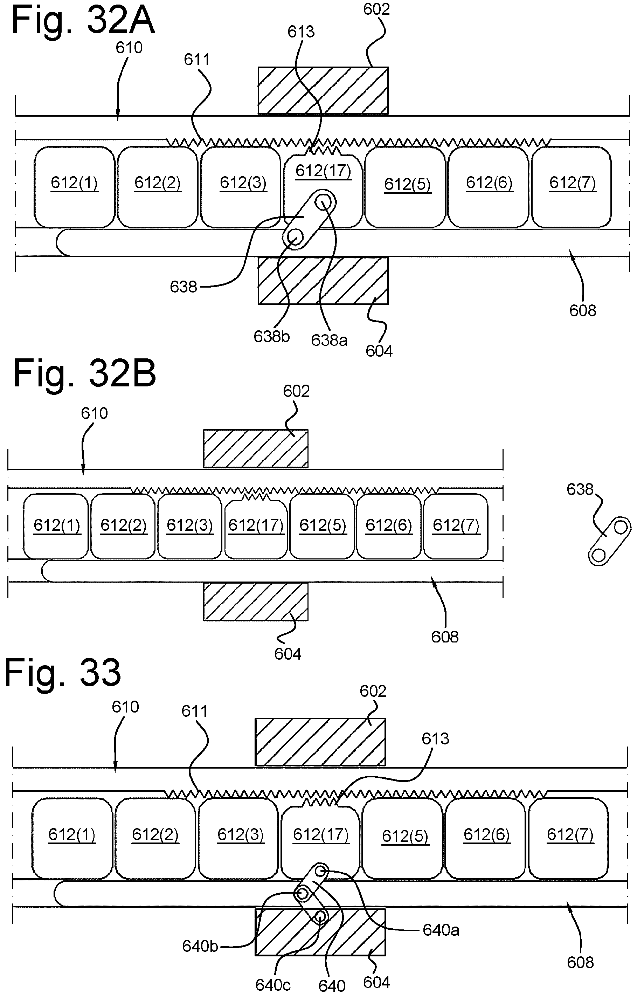

The arrangement shown in figure 32A is quite similar to the one shown in figure 29A. De difference is that sliding element 612(11) is substituted by a sliding element 612(17) which can be operated by a lever 638 which is located on top of sliding element 612(17). Lever 638 is attached to sliding element 612(17) with an axis 638a such that lever 638 can rotate about axis 638a. Moreover, lever 638 is attached to actuation element 608 with an axis 638b such that lever 638 can rotate about axis 638b. Lever 638 is configured such that by pushing/pulling actuation element 608 in the longitudinal direction it can push sliding element 612(17) in the direction of longitudinal element 610 and serrations 613 of sliding element 612(17) engages serrations 611 of longitudinal element 610, and longitudinal element 610 cannot move longitudinally anymore. Note that, since lever 638 is attached to both actuation element 608 and sliding element 612(17), moving actuation element 608 in one direction results in pushing sliding element 612(17) towards longitudinal element 610 but moving actuation element 608 in its opposite direction results in actively pulling sliding element 612(17) away from longitudinal element 610.

Figure 32B shows all components shown in figure 32A which are made in one tube at the left hand side and the lever 638 and the rotation axes 638a, 638b which are located in another layer under or on top of that tube at the right hand side. Lever 638 with rotation axes 638a, 638b can be made from another tube having a smaller or larger diameter than the tube from which the components on the left hand side are made. Rotation axes 638a, 638b can be (laser) welded to that tube. The mechanism of figures 32A, 32B may be more compact and stronger than for example a comparable mechanism as shown in figures 29A, 29B.

The arrangement of figure 33 is, apart from its lever mechanism, identical to the one of figures 32A, 32B. It has a lever 640 with two arms. One arm is attached to sliding element 612(17) with a rotation axis 640a such that it can rotate about rotation axis 640a. Moreover, lever 640 is attached to actuation element 608 with an axis 640b such that lever 638 can rotate about axis 640b. The other arm is also attached to actuation element 608 with axis 640b such that lever 638 can rotate about axis 640b. Finally, the other arm is attached to the fixed world 604 with a rotation axis 640c such that the other arm can rotate about rotation axis 640c.

In its non-actuated state, actuation element 608 can move freely in the longitudinal direction and sliding element 612(17) and longitudinal element 610 are not engaged. Moreover, then, both arms of lever 640 are oriented at an angle <90 degrees relative to the longitudinal direction, as shown. By longitudinally moving actuation element 608 (here, in the right direction) both arms are moved towards an orientation closer to 90 degrees relative to the longitudinal direction such that both arms push sliding element 612(17) towards longitudinal element 610. Serrations 613 engage serration 611 and longitudinal element 610 is blocked from any longitudinal movement. The forces that can be exerted by the lever mechanism of figure 33 are higher than with the one of figures 32A, 32B. Moreover, actuation element 608 needs less longitudinal displacement to cause a same movement of sliding element 612(17) as in figures 32A, 32B.

Note that, since lever 640 is attached to both actuation element 608 and sliding element 612(17), moving actuation element 608 in one direction results in pushing sliding element 612(17) towards longitudinal element 610 but moving actuation element 608 in its opposite direction results in actively pulling sliding element 612(17) away from longitudinal element 610.

Note that the two arms with rotation axes 640a, 640b, 640c are now located in two different layers/tubes than the tube from which the other components are made. Rotation axis 640b can be made of two portion which are welded together and made from two different tubes.

It is observed that this strategy of multilayer/multitube mechanisms (two in figures 32A, 32B, and three in figure 33) is not significantly more expensive to make than single layer/tube mechanisms. All parts are still made by material removal from a layer/tube. The only additional assembly action that is required is making the attachments, preferably with laser welds. This will add no significant cost or complexity, because the same process is already used for attaching the tube layers to each other, there are only a few additional welds to make. The attachment process may be automated and adding a few attachment points is quite simple.

Figures 22A-33 describe single layer/tube and multiple layer/tube mechanisms that act as on/off or clamp and unclamp mechanisms. But mechanisms that have more than one function or that have a switching function can also be envisioned, as will be explained now.

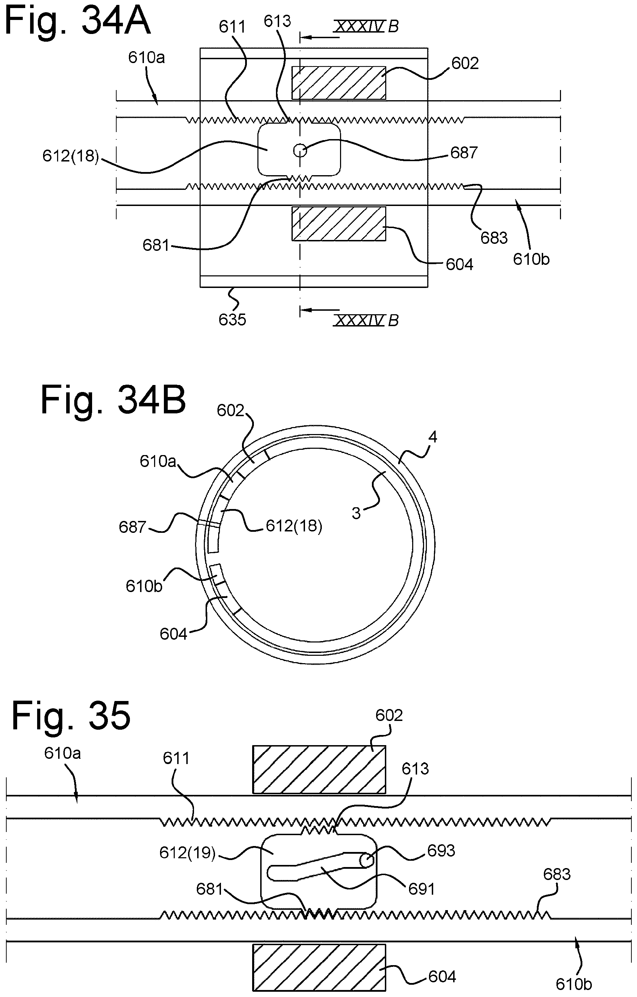

Figure 34A shows an example of a switching arrangement with two parallel longitudinal elements 610a, 610b which may both be steering wires 16(1), 16(2) in instrument 300. The components shown in figure 34A are located in a tube which may be tube 3. Tube 3 is surrounded by tube 4. Figure 34B shows a cross section through tubes 3, 4 as indicated with XXXIV-XXXIV in figure 34A. Figure 34B is a view from the inside of tube 3. A movable element 612(18) is provided between longitudinal elements 610a, 610b. Longitudinal element 610a is provided with serrations 611 facing serrations 613 of movable element 612(18) and longitudinal element 610b is provided with serrations 683 facing serrations 681 of movable element 612(18). Movable element 612(18) is attached to a pin 687 extending into tube 4, as is visible in the right hand part of the figure. Note that this pin 687 can be made by cutting a circular element in both tube 3 and a circular element in tube 4 and then (laser) weld them to one another. Finally, fixed world portions 602 and 604, respectively, are located adjacent to longitudinal elements 610a and 610b, respectively, at sides opposite to the side provided with serrations 611 and 683, respectively.

Tube 4 is rotatably arranged about tube 3 in the tangential direction. Rotating tube 4 about tube 3 results in moving movable element 612(18) in the tangential direction. So, tube 4 can be used as a control element to let either serrations 613 engage with serrations 611, such that longitudinal element 610a is blocked from longitudinal movement, or let serrations 681 engage with serrations 683, such that longitudinal element 610b is blocked from longitudinal movement. The tangential dimension of movable element 612(18) may be selected such that it can be moved in a position between longitudinal elements 610a and 610b such that both longitudinal elements can move freely in the longitudinal direction.

In the arrangement of figures 34A, 34B, tube 4 can be substituted by a ring shaped or any other shaped portion of tube 4. Moreover, a control element to control tangential movement of movable element 612(18) can, alternatively, be a suitably shaped portion of a tube inside tube 3. Instead of one pin 687 several pins can be applied.

Figure 35 shows an alternative to figures 34A, 34B. In this figure a movable element 612(19) is provided which is equal to movable element 612(18) apart from having a slot 691 in which a pin 693 is located, instead of a pin-connection to a control element in another tube. Pin 693 is attached to an actuation element (not shown) in an underlying or overlaying tube, which actuation element can be longitudinally moved but is unable to move tangentially. Slot 691 is shaped such that by longitudinally moving pin 693 by means of the actuation element, movable element 612(19) can be moved between a position in which serrations 611 engage serrations 613 such that longitudinal element 610a is blocked from longitudinal movement and a position in which serrations 681 engage serrations 683 such that longitudinal element 610b is blocked from longitudinal movement.

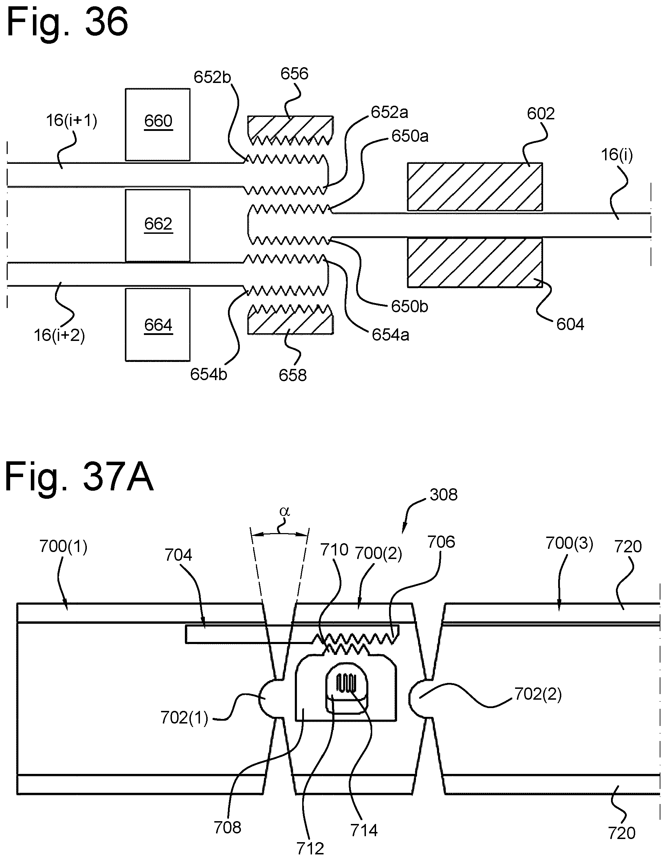

Figure 36 shows an arrangement in which a steering wire can be switched from a first status of being attached to a first steering wire to a status of being attached to a second steering wire. It may be an implementation of the example of figure 15B. The figure shows a steering wire 16(i) of which longitudinal movement is controlled by a suitable control mechanism at the proximal end of instrument 300. At its distal end, steering wire 16(i) is provided with serrations 650a and 650b at both longitudinal sides. Steering wire 16(i) is prevented from tangential movement, e.g., by adjacently located fixed world portions 602, 604.

First steering wire 16(i+1) is tangentially shifted relative to steering wire 16(i) and has serrations 652a at one longitudinal side opposite serrations 650a and serrations 652b at its other longitudinal side opposite serrations of a fixed world portion 656. Second steering wire 16(i+2) is tangentially shifted in the opposite direction relative to steering wire 16(i) and has serrations 654a at one longitudinal side opposite serrations 650b and serrations 654b at its other longitudinal side opposite serrations of a fixed world portion 658. First steering wire 16(i+1) may be configured to steer a deflection of a first deflectable instrument portion, e.g. at the distal end of instrument 300. Second steering wire 16(i+2) may be configured to steer a deflection of a second, other, deflectable instrument portion, e.g. also at the distal end of instrument 300.

A switching element is provided which is configured to move both steering wires 16(i+1) and 16(i+2) simultaneously in the same tangential direction. Here, that switching element comprises a first switching element 660, a second switching element 662 and a third switching element 664. Second switching element 662 is located between both steering wires 16(i+1), 16(i+2), whereas steering wires 16(i+1) and 16(i+2), respectively are located between second switching element 662 and first/third switching elements 660 and 664, respectively.

When switching elements 660, 662, 664 are moved in a first tangential direction (up in the drawing), two things are accomplished. First steering wire 16(i+1) is engaged with fixed world portion 656 via serrations 652b. This prevents any further longitudinal movement of first steering wire 16(i+1) and, consequently, freezes the curvature of the first deflectable instrument portion. At the same time, serrations 654a of second steering wire 16(i+2) engage serrations 650b of steering wire 16(i) such that second steering wire 16(i+2) is coupled to steering wire 16(i). So, any longitudinal movement of steering wire 16(i) is transferred to longitudinal movement of second steering wire 16(i+2) which then controls deflection of the second deflectable instrument portion.

When switching elements 660, 662, 664 are moved in the opposite tangential direction (down in the drawing), the reversed result is achieved. Serrations 652b of first steering wire 16(i+1) are disengaged with fixed world portion 656 whereas its serrations 652a engage serrations 650a of steering wire 16(i) and couple first steering wire 16(i+1) to steering wire 16(i). At the same time, serrations 654a of second steering wire 16(i+2) disengage with serrations 650b of steering wire 16(i) whereas serrations 654b now engage with serrated fixed world portion 658. So, longitudinal movement of steering wire 16(i) is now transferred to longitudinal movement of first steering wire 16(i+1) which then controls deflection of the first deflectable instrument portion. Now the second deflectable instrument portion curvature is frozen. This switching mechanism therefore has multiple functions, i.e., freezing the curvature of one deflectable instrument portion and at the same time engage the steering of the other deflectable instrument portion.

The examples 22A thru 36 only describe a few of all possible mechanisms that can have an on/off, a switching, a braking (friction or ratcheted) or a movement limiting function. The examples also merely show mechanisms that are activated tangentially and that have an effect on longitudinal movements of various elements. But, one can envision mechanisms that are activated longitudinally and that have an effect on tangential movements of an element. Also directions in between are possible. Many more examples of these mechanisms, their activation method and a mix of the above mentioned characteristics can be envisioned. One of many other possibilities of what one can do with these mechanisms is described next. Whereas, examples of figures 22A thru 36 describe mechanisms that affect linear movement or fixation of sliding elements, similar mechanisms can affect rotational movement or fixation of rotating elements, as will be explained with reference to figures 37A thru 41.

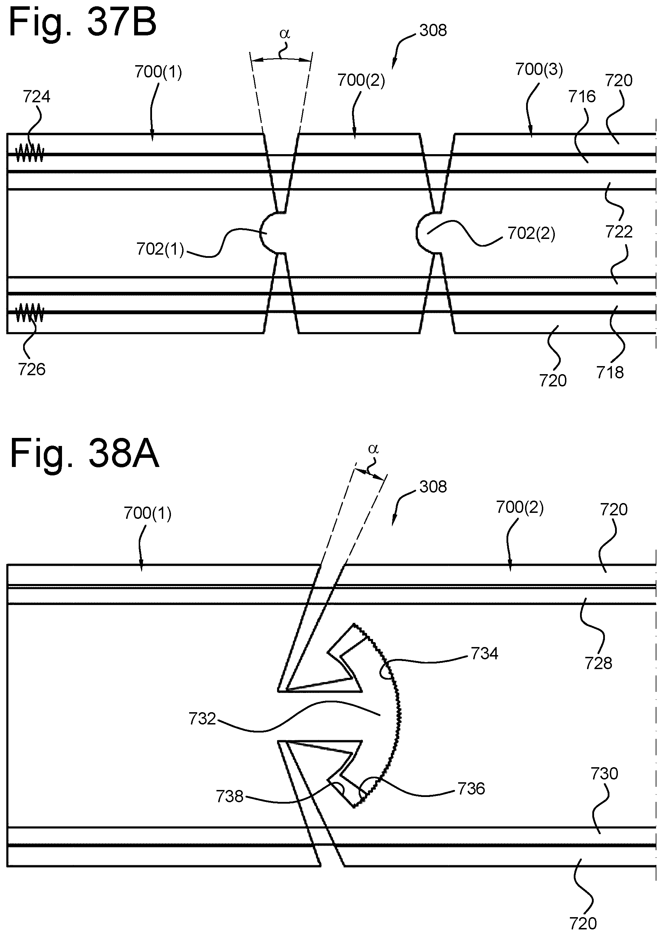

Figure 37A, which can be seen as an implementation of the example of figure 19A, shows three adjacent hinge portions 700(1), 700(2), 700(3) of a hinge 308 in a longitudinal cross section and as seen from the inside of a tube 720 such that one sees an outer wall of that tube. Hinge 308 may be part of a deflectable portion of instrument 300, e.g., located in the tip section. Hinge portion 700(1) and hinge portion 700(2) can be rotated relative to one another over an angle α about a rotation portion 702(1). Hinge portion 700(2) and hinge portion 700(3) can be rotated relative to one another over a predetermined angle, which may be equal to α, about a rotation portion 702(2). Portions 700(1), 700(2), 700(3), 702(1), 702(2) are, in an example, cut in a first tube. The embodiment shown in figures 37A, 37B deflects in one direction only, i.e., the surface of the drawing. However, the same principles apply to hinges which can be deflected in all directions.

A locking element 704 is provided which is located inside or outside and attached to hinge portion 700(1). Locking element 704 extends over hinge portion 700(2) with a serrated side 706. Moreover, a switching element implemented as a sliding element 708 is provided. Sliding element 708 is located inside or outside hinge portion 700(2) and is movable in the tangential direction of the instrument. Sliding element 708 has a serrated side 710 facing serrations 706 of locking element 704. Sliding element 708 is provided with a hole in which a guiding element 712 is located which is attached to underlying or overlaying hinge portion 700(2) at a point of attachment 714, e.g., by laser welding. Guiding element 712 allows sliding element 708 to be only movable in the tangential direction towards and away from locking element 704. Sliding of sliding element 708 can be controlled in any suitable way, e.g., by using any of the examples shown in any of the preceding figures. All elements 704, 708, 714 are arranged in a second tube underlying or overlaying the first tube.

Locking/unlocking of hinge portions 700(1), 700(2) of figure 37A is done as follows. Once hinge portions 700(1), 700(2) are rotated relative to one another at a certain desired angle α, sliding element 708 is shifted towards locking element 704 such that serrations 706 and 710 engage one another. Because locking element 704 is attached to hinge portion 700(1), sliding element 708 is longitudinally frozen relative to hinge portion 700(1), and so angle α between hinge portions 700(1) and 700(2) will be frozen.

Note that a similar locking mechanism can be used for the angle of rotation between hinge portions 700(2) and 700(3). Moreover, the same principle can be used to freeze angles of rotation between many adjacent hinge portions 700(n), 700(n+1), n = 1, ..., N. Several such rotation locking mechanisms can be controlled by several sliding elements at the same time, e.g., by using a single actuation element that can actuate all sliding elements at the same time.

Figure 37B shows an alternative embodiment. Figure 37B again shows three adjacent hinge portions 700(1), 700(2), 700(3) which can rotate relative to one another about respective rotation portions 702(1), 702(2). Again, the view is a longitudinal cross section seen from the inside of a first tube 720. A first locking element 716 is provided inside tube 720 which runs through all hinge portions 700(1), 700(2), 700(3) and may be attached to the wall of tube 720 of hinge portion 700(1) at a location 724. A second locking element 718 (or even more) may be provided inside tube 720 which also runs through all hinge portions 700(1), 700(2), 700(3) and may be attached to the wall of tube 720 of hinge portion 700(1) at a location 726. First and second locking elements 716 and 718 may both be resulting from cutting a suitable pattern in a second tube located inside the first tube having the hinge portions 700(1), 700(2), 700(3). Here, a switching element is implemented by an inner core 722 provided inside the second tube. Inner core 722 is expandable in the radial direction by a suitable actuation element not shown, e.g. located at the proximal end of the instrument 300. For instance, inner core 722 can be implemented by a spring which can be expanded by rotating portions relative to one another.

If one or more of the adjacent hinge portions 700(1), 700(2), 700(3) would rotate relative to one another, locking elements 716, 718 would longitudinally move relative to hinge portions 700(2) and 700(3). If one then expands inner core 722 locking elements 716, 718 would be pushed against the wall of first tube720, resulting in freezing the hinge 308 in its curvature.

Hinge 308 can be provided with more than three hinge portions. Moreover, other mechanisms may be used to push locking elements 716, 718 radially against tube wall 720 once the hinge portions are in a desired rotated position.

Another switching element mechanism that can be used for blocking hinged portions in an instrument is a mechanism that blocks rotation of rotation portions. Prior art reveals (for instance in "follow-the-leader" technology) that blocking of rotation of hinged elements can be accomplished by pushing or pulling the hinged elements towards each other. The generated normal forces in the hinge contact surfaces than cause a friction force between these contact surfaces that prevents rotation. Here, another mechanism is proposed, in which serrations are provided in opposite surfaces which will engage one another once actuated, thus blocking any further rotation. Such mechanism is defined in the appended claims.

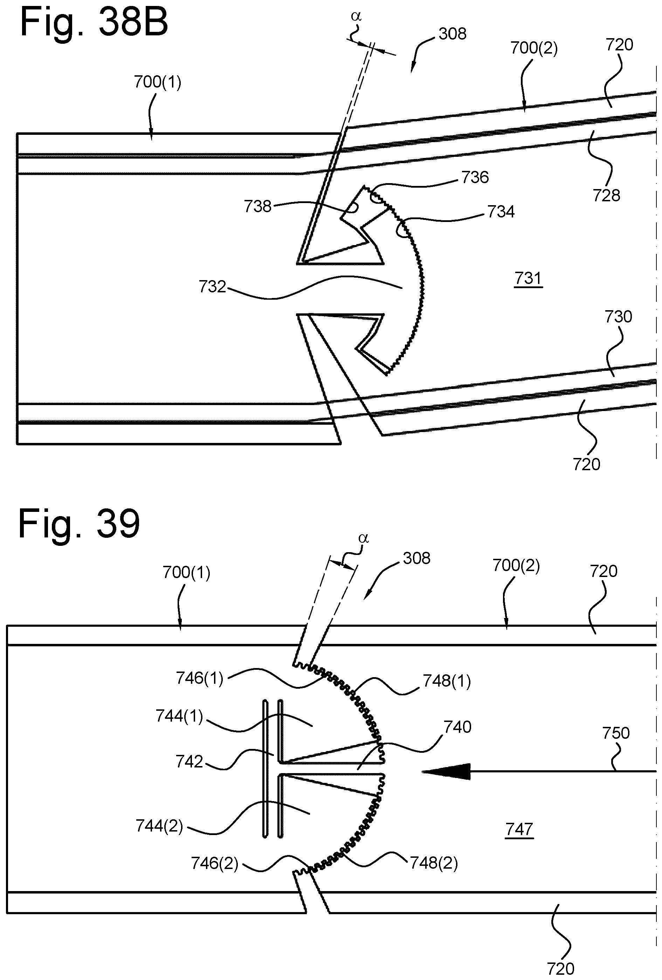

Figure 38A shows hinge 308 in which adjacent first and second hinge portions 700(1), 700(2) can rotate relative to one another over an angle α. The figure is a longitudinal, inside cross section through a first tube 720 having wall. First hinge portion 700(1) is provided with a convex portion 732 configured to rotate inside a concave portion 738 in second hinge portion 700(2). Convex portion 732 may be implemented as a T-shaped extension having an outer convex front edge and concave portion 738 may be implemented as a T-shaped opening accommodating T-shaped extension 732 configured such that T-shaped extension 732 can rotate inside T-shaped opening 738. Convex portion 732 and concave portion 738 function as a clamp. Convex portion 732 is provided with a serrated outside surface 734 facing a serrated outside surface 736 of concave portion 738. Serrated outside surface 734 and serrated outside surface 736 function as a locking element. One or more actuation elements 728, 730 run through both the first and second adjacent hinge portions 700(1), 700(2) and are attached to one of them. Similar constructions are described with reference to figures 39, 40, 45A-45D.

Actuation elements 728, 730 can be used to push first and second hinge portions 700(1), 700(2) away from each other in the longitudinal direction, as shown in figure 38A. In that condition, serrations 734, 736 do not engage and hinge portions 700(1), 700(2) can rotate freely relative to one another. However, as shown in figure 38B, once first and second hinge portions 700(1), 700(2) have a desired rotation angle α relative to one another, the one or more actuation elements 728, 730 are pulled such as to pull serrations 734, 736 into mutual engagement. Then, first and second hinge portions 700(1), 700(2) are blocked from further mutual rotation.

Figures 38A, 38B can be seen as an implementation of the example of figure 21.

Figure 39 shows a variant to figures 38A, 38B. Again, a longitudinal cross section through hinge 308 is shown, as seen from the inside. Here, first hinge portion 700(1) is provided with first and second convex portions 744(1) and 744(2). First convex portion 744(1) has a serrated outside convex edge 746(1). Second convex portion 744(2) has a serrated outside convex edge 746(2). Second hinge portion 700(2) has a concave portion 747 with a first serrated concave edge 748(1) facing serrated outside convex edge 746(1) and a second serrated concave edge 748(2) facing serrated outside convex edge 746(2). Convex portions 744(1), 744(2) and concave portion 747 function as a clamp. Serrated outside convex edges 746(1), 746(2) and serrated outside concave edges 748(1), 748(2) function as locking element.

First and second hinge portions 700(1) and 700(2) are resiliently attached to one another. In the shown example, that is implemented by a longitudinally extending bridge 740 fixedly attached to second hinge portion 700(2) and resiliently attached to first hinge portion 700(1). To that end, bridge 740 may be attached to a flexible strip 742 cut from wall 720 and extending in the tangential direction. Strip 742 has slots at both longitudinal sides which allow the point of attachment between strip 742 and bridge 740 to move resiliently in the longitudinal direction. Bridge 740 extends between first and second convex portions 744(1), 744(2) such that they are able to rotate inside first and second concave edges 748(1), 748(2) about a predetermined maximum angle α.

The arrangement shown in figure 39 is provided with an actuation mechanism that is configured to pull or push hinge portions 700(1), 700(2) away from or towards one another. This may be implemented by one or more (not shown) actuation elements like 728, 730 shown in figures 38A, 38B. Preferably, when such actuation elements are not actuated, strip 742 is in a relaxed state, wherein serrations 746(1) do not engage serrations 748(1) and serrations 746(2) do not engage serrations 748(2). Thus, then, first and second convex portions 744(1), 744(2) can rotate freely relative to second hinge portion 700(2). However, once first hinge portion 700(1) and second hinge portion 700(2) are mutually rotated at a desired angle α, the actuation mechanism can be actuated to push or pull first and second hinge portions towards one another, as indicated with an arrow 750, such that serrations 746(1) engage serrations 748(1) and serrations 746(2) engage serrations 748(2) which will freeze the mutually rotated orientation of first and second hinge portions 700(1) and 700(2). In the engaged state, strip 742 will develop a spring force trying to disengage first and second hinge portions 700(1), 700(2).

If the actuation mechanism is implemented by one or more actuation elements 728, 730 actuation can be implemented by a longitudinal pulling force. To stop the actuation one, then, needs to stop the pulling force because resilient strip 742 will take care of first and second hinge portions 700(1), 700(2) becoming disengaged again such that the freezing of the rotation angle will end. An advantage may be that, in such an implementation, no pushing force needs to be exerted on actuation elements 728, 730 for such a disengagement which pushing force could result in buckling problems in actuation elements 728, 730. The spring force of member 742 can be engineered such that it does not compress as a result of pull forces on steering wires when steering the tip of the instrument.

Figure 40 shows an alternative hinge arrangement to the one shown in figure 39. Again, a longitudinal cross section through hinge 308 is shown. In this example, first hinge portion 700(1) is provided with a concave edge. Second hinge portion 700(2) is provided with a convex portion 759 configured to rotate inside concave portion 757. Convex portion 759 is provided with serrations 758 on its outside edge. Moreover, a switching element 752 is cut from wall 720 in first hinge portion 700(1). Switching element 752 is attached to one or more flexible strips 754(1), 754(2) which are cut from wall 720 and extending in the tangential direction such that switching element 752 can be longitudinally moved back and forth, cf. 750, in a resilient way. This back and forth movement can be controlled by an actuation mechanism like actuation element 728 shown in figure 38A, 38B which is then attached to switching element 752. Switching element 752 has a locking tip 756 which is configured to engage serrations 758. Locking tip 756 and serrations 758 function as locking element. In the engaged state, concave edge 757 and convex edge 759 cannot rotate relative to one another anymore and the current angle of rotation α between first and second hinge portions 700(1), 700(2) is then frozen.

In the shown embodiment, first and second hinge portions 700(1), 700(2) are prevented from moving too far from one another in the longitudinal direction by providing first hinge portion 700(1) with a T-shaped element 732 extending inside a T-shaped slot 738 in second hinge portion 700(2), which T-shaped element 732 and T-shaped slot 738 are designed such that mutual rotation of first and second hinge portions 700(1), 700(2) is possible. Of course, other shaped mechanisms can be applied to prevent substantial longitudinal relative movement but allow rotational relative movement of first and second hinge portions 700(1), 700(2).

Examples 37A thru 40 only describe a few possible mechanisms that one can use to block rotation of one hinge portion to an adjacent hinge portion. Such hinge portions may be located anywhere in instrument 300, e.g. in a steerable tip or instrument body section. For the two basic principles of either fixing the gaps between the hinge portions or actually blocking the rotation of the hinge portions many more mechanisms can be envisioned. The next figures show some possible embodiments entirely implemented by means of cutting elements in tubes surrounding each other, e.g. by means of laser cutting.

Figures 41A - 41C relate to a three tubes embodiment. Figure 41A shows tip section 13 of the three tubes 2, 3 and 4 (cf. figures 1 and 2). In the shown example, tip section 13 has a solid portion and a deflectable portion proximal to the solid portion. In the solid portion, outer tube 4 comprises rigid end part 17 and in the deflectable portion outer tube 4 comprises flexible part 18 implemented by a hinge resulting from cutting a pattern of slots in tube 4. The shown hinge pattern is but one example of a possible implementation.

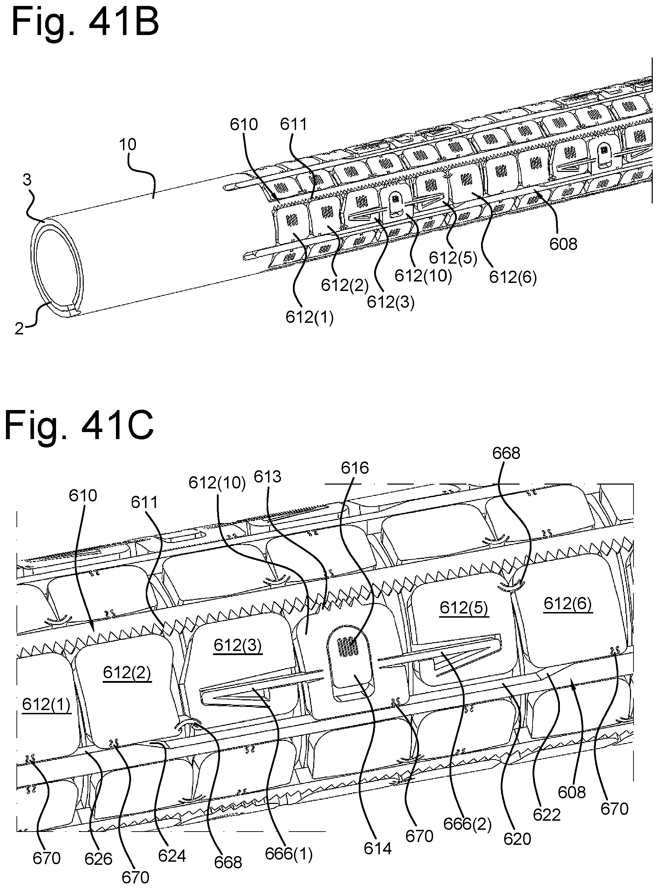

Figure 41B shows intermediate tube 3 with its rigid end part 10. Figure 41C shows a portion of the embodiment of figure 41B on an enlarged scale. In this embodiment, intermediate tube 3 has an implementation of the example of figure 26 in its flexible part in tip section 13. The figure shows some extra implementation details of adjacent spacers 612(1) - 612(3), 612(5), .... and of sliding element 612(10). I.e., sliding element 612(10) is attached to a first end of a first strip 666(1) extending to and being attached to spacer 612(3) at a second end in a resilient way. Moreover, sliding element 612(10) is attached to a first end of a second strip 666(2) extending to and being attached to spacer 612(5) at a second end in a resilient way. In this way, sliding element 612(10) is resiliently suspended between spacers 612(3) and 612(5). The suspension is such that sliding element 612(10) cannot or can hardly move in in the longitudinal direction relative to spacers 612(3), 612(5) but can move resiliently in a tangential direction relative to these spacers 612(3) and 612(5). When moving in the tangential direction towards steering element 610 first and second strips 610(1) and 666(2) develop a counter spring force.

Actuation element 608, as explained with reference to figure 26, can be actuated to force sliding element 612(10) in the tangential direction by means of its faces 622 and 624. In the actuated state, serrations 613 of sliding element 612(10) engage serrations 611 of steering wire 610 such as to block any further longitudinal movement of steering wire 610 and, thus, to freeze a deflected state of the flexible part 18.

In the non-actuated state of actuation element 608 first and second strips 666(1), 666(2) keep sliding element 612(10) in a neutral tangential position in which serrations 611 and 613 are disengaged and, thus, flexible part 18 can be deflected by steering wire 610.

As is visible in figures 41B and 41C, the shown embodiment comprises four steering wires 610 equidistantly located in the tangential direction relative to one another. Each such steering wire 610 may be locked from further longitudinal movement by one or more sliding elements located at different longitudinal locations. All sliding elements that can be actuated to lock the same steering wire 610 are actuated by a single actuation element 608. That single actuation element 608 has the form of a longitudinal strip extending from the distal end to the proximal end of instrument 300. Actuation element 608 can move freely at the distal end and is operated from the proximal end, either manually or robotically, to move longitudinally between the actuated and non-actuated state. In this embodiment there are four such actuation elements 608 in the form of longitudinal strips. They are likewise equidistantly located in the tangential direction.

As shown, before the flexible part is deflected for the first time, spacers 612(1), 612(2), sliding element 612(10), spacer 612(6), .... are still attached to adjacent actuation element 608 by means of one or more fracture elements 670. These fracture elements keep all different elements of tube 3 after the cutting process together such that tube 3 does not fall apart into separate pieces.

Fracture elements 670 should be designed in the following way. Before being fractured, each fracture element 670 is attached to one or more opposite elements cut in tube 3. These opposite elements have a geometrical shape such that the stresses in the fracture element 670 are higher than in the surrounding material and/or structure. Therefore, if a deflection or a high enough force is applied on a structure with a fracture element 670 the stress in the fracture element 670 rises above the yield stress of the tube material, causing permanent deflection of fracture element 670. Applying even more deflection or a higher force results in the stress reaching the ultimate tensile stress causing a fracture of fracture element 670. Another mechanism to break the fracture element is achieved by applying low or high cycle fatigue to fracture element 670. The stress in fracture element 670 is raised above the fatigue limit, causing a fatigue fracture. In all cases, the stresses in the surrounding structure/material stays at least below the yield stress of the tube material.

Here, fracture elements 670 are designed to fracture after instrument 300 is finished and the tip section 13 is deflected for the first time.

Moreover, spacers 612(2) and 612(3) are attached to one another by a flexible bridge in order to keep spacer 612(3) at a well-defined tangential position at a well-defined distance from actuation element 608. In the same way, spacer 612(5) and 612(6) are attached to one another by a flexible bridge 668 in order to keep spacer 612(5) at a well-defined tangential position at a well-defined distance from actuation element 608. In this way, faces 622 and 624 cannot touch and, thus, not tangentially move spacers 612(3), 612(5).

It is observed that the locking mechanism as shown in figures 41A-41C can be applied anywhere in instrument 300.

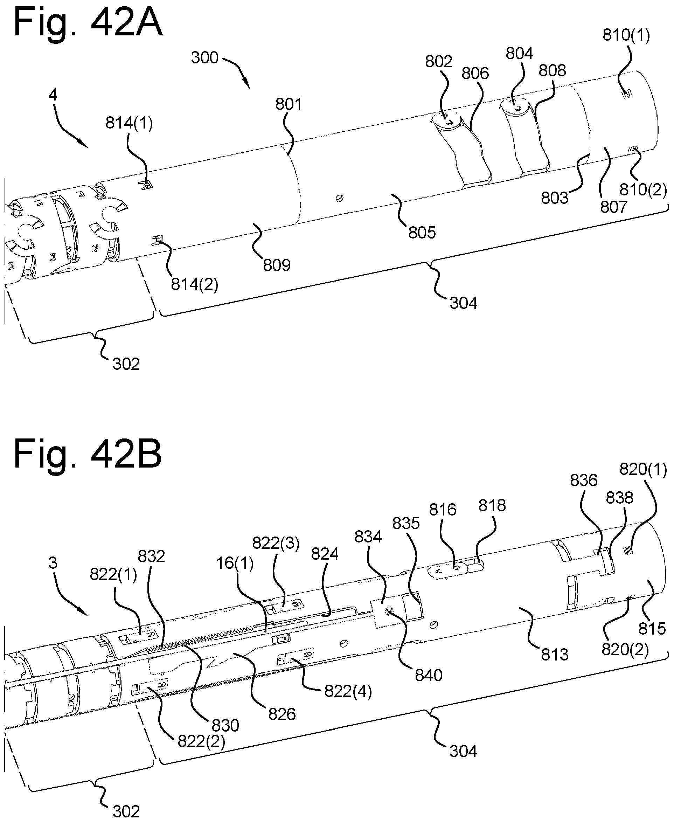

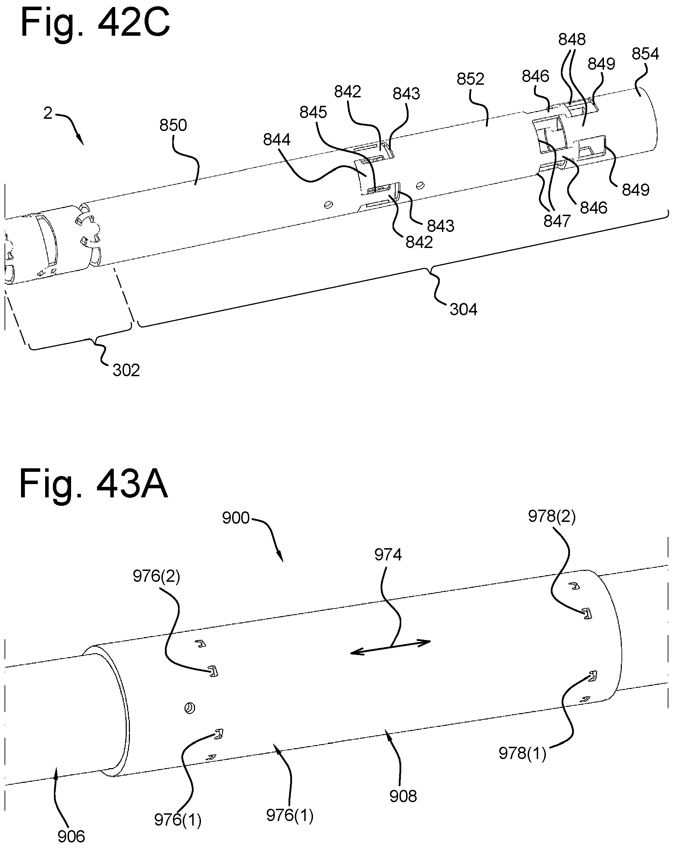

Figures 42A - 42C show a further embodiment in which steering wires can be pre-loaded. The mechanism is shown for one steering wire 16(1). However, more than one, e.g., four, steering wires may be applied. Figure 42A shows outer tube 4, figure 42B shows intermediate tube 3, and figure 42C shows inner tube 2. After finishing instrument 300, tube 2 is inserted in tube 3 and tube 3 is inserted in tube 4. All figures 42A-42B show an example of a proximal end of instrument 300 but the shown technology can be anywhere in the instrument. A rigid portion of instrument 300 is indicated with reference number 304, and a flexible portion with reference number 302. Flexible portion 302 is configured to control steering of a deflectable portion in, e.g., the tip section of instrument 300, by bending flexible portion 302. To that effect, the portion of outer tube 4 in flexible portion 302 may have any suitable hinge pattern. The one shown in figure 42A is but one example. Note that rigid portion 304 can be substituted for a flexible portion which has a lower flexibility than flexible portion 302.

In the rigid portion 304, outer tube 4 has a sleeve 805 made from outer tube 4 but separated from the remainder of outer tube 4 by two circumferential slots 801, 803 such that sleeve 805 can rotate in the tangential direction. Two actuation elements 802 and 804, respectively, are located inside tangential slots 806 and 808, respectively. Actuation elements 802, 804 may have a circular shape and are attached to elements of intermediate tube 2, e.g. by (laser) welding, as will be explained hereinafter.

At the proximal side of sleeve 805, outer tube 4 comprises a rigid ring 807 and at the distal side outer tube 4 comprises a rigid sleeve 809.

Figure 42B shows an example of intermediate tube 3. It has a suitable flexible structure in flexible portion 302 of instrument 300. In rigid portion 304 of instrument 300, intermediate tube 3 is separated into two portions which are longitudinally movable relative to one another, i.e., a rigid ring 815 at the proximal end and a sleeve 813 located distally from rigid ring 815. At one or more attachment points 810(1), 810(2), ..., respectively, rigid ring 807 in outer tube 4 is attached to rigid ring 815 in intermediate tube 3, e.g. by (laser) welding.

Sleeve 813 and rigid ring 815 are configured to be longitudinally movable relative to one another to a predetermined extent. Here, this is implemented by sleeve 813 having one or more T-shaped extensions 836 each one located in a respective T-shaped slot 838 in rigid ring 815.

Sleeve 813 is provided with a slider 816 configured such that it can longitudinally slide in a slot 818 in sleeve 813. Slider 816 is attached to actuation element 802 in outer tube 4, e.g., by (laser) welding. Sleeve 813 is attached to actuation element 804 in outer tube 4, e.g., by (laser) welding. One or more guiding elements 822(1), 822(2), ..., arranged inside respective slots are provided in sleeve 813. These one or more guiding elements 822(1), ..., are all attached to inner tube 2 as will be explained hereinafter. They are shaped such that they can slide along a predetermined longitudinal distance inside their respective slots and do not allow any tangential movement in those slots.

At its distal side, sleeve 813 is provided with one or more longitudinal slots 824 accommodating a proximal end of a steering wire 16(1). Longitudinal slot 824 is provided with a serrated portion 832 facing a serrated portion 830 of steering wire 16(1). Longitudinal slot 824 also accommodates a longitudinal actuation element 826. Longitudinal actuation element 826 has a proximal end portion 834 located inside a proximal end portion 835 of longitudinal slot 824. Proximal end portion 834 and proximal end portion 835 are configured such that proximal end portion 834 and thus the entire actuation element 826 can longitudinally move relative to sleeve 813 only to a certain predetermined extent. Moreover, apart from some manufacturing tolerances, proximal end portion 834 is designed, in this example, to be unable to move tangentially relative to sleeve 813. The distal end of actuation element 826 has one longitudinal side facing a longitudinal side of steering wire 16(1) opposite its serrated portion 830. Moreover, the distal end of actuation element 826, at its longitudinal side opposite to steering wire 16(1), is provided with one or more faces facing one or more faces in sleeve 813 such that when actuation element 826 is moved in the longitudinal direction relative to sleeve 813 towards the proximal end of instrument 300 that distal end is forced to move in the tangential direction towards steering wire 16(1) such that serrations 830 and 832 will engage and steering wire 16(1) will be blocked from any longitudinal movement relative to sleeve 813.

Figure 42C shows further details of inner tube 2. It has a suitable flexible structure in flexible portion 302 of instrument 300. In rigid portion 304 of instrument 300, inner tube 2 is separated into three portions 850, 852, 854. Reference number 854 refers to a rigid ring at the proximal end. A sleeve 852 is located distally from rigid ring 854 and a further sleeve 850 is located distally from sleeve 852. Sleeve 852 is configured to be longitudinally movable between rigid ring 854 and sleeve 850 along a predetermined distance. In the shown example, that is implemented by sleeve 852 being provided with one or more T-shaped extensions 846 located inside T-shaped openings 849 in rigid ring 854 and rigid ring 854 being provided with one or more T-shaped extensions 848 located inside T-shaped openings 847 in sleeve 852. Moreover, sleeve 852 is provided with one or more T-shaped extensions 844 located inside T-shaped openings 845 in sleeve 850 and sleeve 850 is provided with one or more T-shaped extensions 842 located inside T-shaped openings 843 in sleeve 852.

Slider 816 in intermediate tube 3 is not only attached to actuation element 802 in outer tube 4 but also to sleeve 852 in inner tube 2. All guiding elements 822(1), ..., 822(4) in intermediate tube 3 are attached to sleeve 850 in inner tube 2. Rigid ring 815 of intermediate tube 3 is not only attached to rigid ring 807 of outer tube 4 but also to rigid ring 854 in inner tube 2, as indicated with attachment locations 820(1), 820(2), ... Finally, each proximal end portion 834 of actuation element 826 is attached to sleeve 852 in inner tube 2, for instance to T-shaped extension 844. This is indicated with an attachment location 840 in proximal end portion 834. Attachments may be done with (laser) welding.

The embodiment of instrument 300 shown in figures 42A-42C operates as follows. Providing the one or more steering wires 16(1) with a pre-load starts with rotating sleeve 805 in outer tube 4 relative to the remainder of outer tube 4. Due to this rotation the actuation elements 802, 804 are forced to follow the respective paths defined by slots 806 and 808. These slots 806, 808 are designed such that, in the first part of the rotation, only actuation element 802 moves in the longitudinal direction towards the proximal end of instrument 300 due to the shape of slot 806 whereas actuation element 804 is kept at a fixed longitudinal position by slot 808. As actuation element 802 is attached to the slider 816 in intermediate tube 3, which again is attached to sleeve 852 in inner tube 2, sleeve 852 will slide in the longitudinal direction towards the proximal end of instrument 300. Since sleeve 852 is attached to proximal end portion 834 of actuation element 826, also actuation element 826 is forced to move in the longitudinal direction towards the proximal end of instrument 300.

This forces the faces in the distal end of actuation element 826 to move "upward" the counter faces in sleeve 813 such that the distal end of actuation element 826 is moved in the tangential direction which pushes the proximal end of steering wire 16(1) in the tangential direction such that its serrations 830 come into engagement with serrations 832 of sleeve 813. Now, the proximal end of steering wire 16(1) is locked to sleeve 813. Note that sleeve 813 is tangentially locked by guiding elements 822(1), ..., 822(4). By, then, rotating sleeve 805 of outer tube 4 further, the pre-load step is actuated. I.e., then, due to the shape of slots 806, 808, actuation elements 802 and 804 are simultaneously moved in the longitudinal direction towards the proximal end of instrument 300 and they pull both sleeves 813 and 852 with the attached steering wire(s) 16(1) in the same direction, i.e., towards the proximal end of instrument 300. This pre-loads the steering element(s) 16(1). This pre-loaded condition can be a stable condition by designing slots 806 and 808 such that actuation elements 802 and 804 are then in an indented portion of the slots from which they cannot return without external intervention.

Note that pre-loaded steering wire(s) 16(1) provide instrument 300 with a better response of its tip section to movements of the steering wires at the proximal end. Moreover, the shown arrangement is suitable to undo the pre-loaded condition by simply rotating sleeve 805 in outer tube 4 back again.

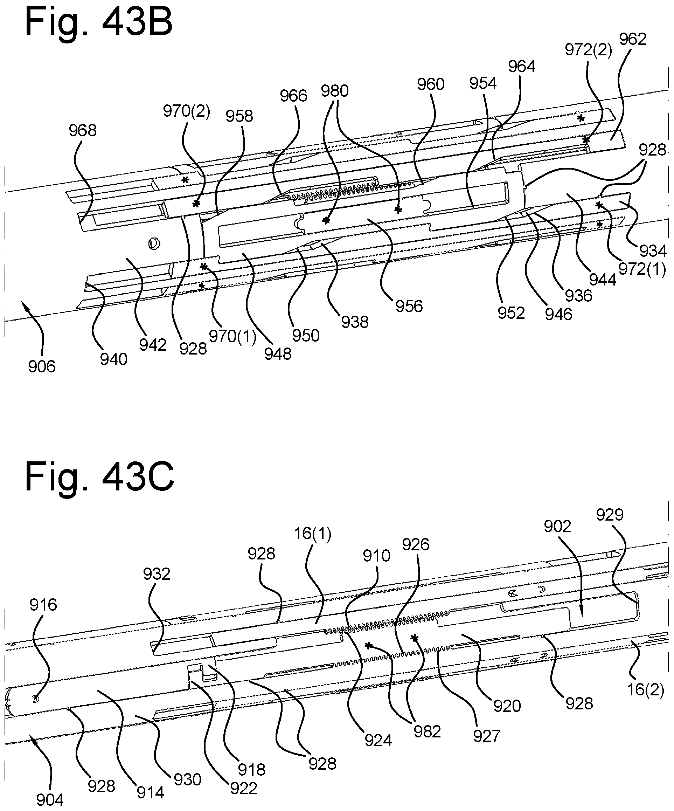

Figures 43A-43C shows an example of a switch 900 implemented by means of cutting suitable patterns in four tubes surrounding one another. However, the disclosure is not limited to this number. The switch element can be located at any desired location in instrument 300. Figure 43A shows an outer tube 906 surrounded by a sleeve 908. Figure 43B shows outer tube 906 when sleeve 908 is removed. Figure 43C shows an intermediate tube 904. An inner tube (or core) 902 is indicated in figure 43C too but not separately drawn because it has no relevant details for the current example. The shown switch may either have three or two positions in dependence on whether the switch needs a neutral position.

Outer tube 906 has a plurality of actuation elements, two of which being indicated with reference numbers 934, 962. Actuation elements 934 and 962, respectively are movable in the longitudinal direction in slots 940 and 968, respectively. A frame 948 is provided between these actuation elements 934, 962, which is movable in the tangential direction of switch 900 between two opposite portions 942, 944 of outer tube 906. Frame 948 has a rectangular slot 954 in which a slider 956 is provided which can slide in that slot 954 in the longitudinal direction and can be moved in the tangential direction together with tangential movement of frame 948. Actuation element 934 has one or more faces 936 and 938 facing frame 948. Actuation element 962 has one or more faces 964 and 966 facing frame 948. Frame 948 has one or more faces 950 and 952 facing actuation element 934. Frame 948 has one or more faces 958 and 960 facing actuation element 962. The shape of the faces 958 and 960 can be engineered such that it creates two or three or more discrete tangential positions of frame 948. Sleeve 908 has one or more points of attachment 976(1), 978(1) attached to one or more points of attachment 970(1), 972(1) on actuation element 934, and one or more points of attachment 976(2), 978(2) attached to one or more points of attachment 970(2), 972(2) on actuation element 962. Sleeve 908 can be longitudinally moved in both directions, as indicated with a double arrow 974. Thus, actuation elements 934 and 962 can be moved in the longitudinal direction by means of sleeve 908. Sleeve 908 may be attached to one or more control wires, e.g., cut from a same tube and running to the proximal end of instrument 300 such that longitudinal movement of sleeve 908 can be controlled from the proximal end.

Figure 43C shows more details of intermediate tube 904. Intermediate tube 904 has several steering wires of which two 16(1), 16(2) are shown. The implementation will be described for steering wire 16(1) but is equally applicable to other steering wires. Steering wire 16(1) can be moved in the longitudinal direction in a slot 932. At its distal end, steering wire 16(1), is provided with serrations 910 along a portion of its length.

A switching element is provided in the form of a switch element 920 adjacent to serrated portion 910 of steering wire 16(1). Switch element 920 is provided with serrations 924 at one longitudinal side which can engage serrations 910 of steering wire 16(1). At its opposite longitudinal side, switch 920 is provided with serrations 926. These serrations 926 are arranged opposite serrations 927 in a fixed portion 930 of intermediate tube 904. Switch element 920 is arranged inside a longitudinal slot 929 which allows switch element 920 to be movable in the longitudinal direction but also in the tangential direction to a certain extent. At one or more attachment points 982, switch element 920 is attached to slider 956 in outer tube 906 such that slider 956 operates as a guiding element for switch element 920. At its distal end, switch element 920 is connected to an element 914 such that switch element 920 can move in the tangential but not in the longitudinal direction relative to element 914. Element 914 may be a steering wire itself running in the distal direction, may be attached to a steering wire in a tube inside or outside intermediate tube 904 or may be attached to a fixed portion of a tube inside and/or outside intermediate tube 904. In the latter case, element 914 is attached at a distal end of a deflectable portion in instrument 300.

The arrangement of figures 43A-43C operates in the following way. Moving sleeve 908 in one longitudinal direction, e.g., to the proximal direction as shown, results in actuation elements 934 and 962 both being moved to a first longitudinal position in which faces 964, 966, 958 and 964 have forced frame 948 together with slider 956 in a first tangential direction. Because switch element 920 is attached to slider 956, switch element 920 is now moved into a position in which its serrations 926 engage serrations 927 of fixed portion 930 such that switch 920 is blocked from any further longitudinal movement. Thus, also element 914 is locked in its longitudinal position. Any deflectable portion attached to this element 914 is, thus, locked in its current deflected orientation.

Now moving sleeve 908 in the opposite longitudinal direction, e.g., to the distal direction, results in actuation elements 934 and 962 both being moved to a second longitudinal position in which faces 936, 938, 950, 952 force frame 948 together with slider 956 in a second tangential direction opposite to the first tangential direction until serrations 924 engage serrations 910. Then, switch element 920 can be moved longitudinally by longitudinally moving steering wire 16(1). Any longitudinal force in steering wire 16(1) will, thus, be transferred to element 914 which enables deflecting a deflectable portion of instrument 300.

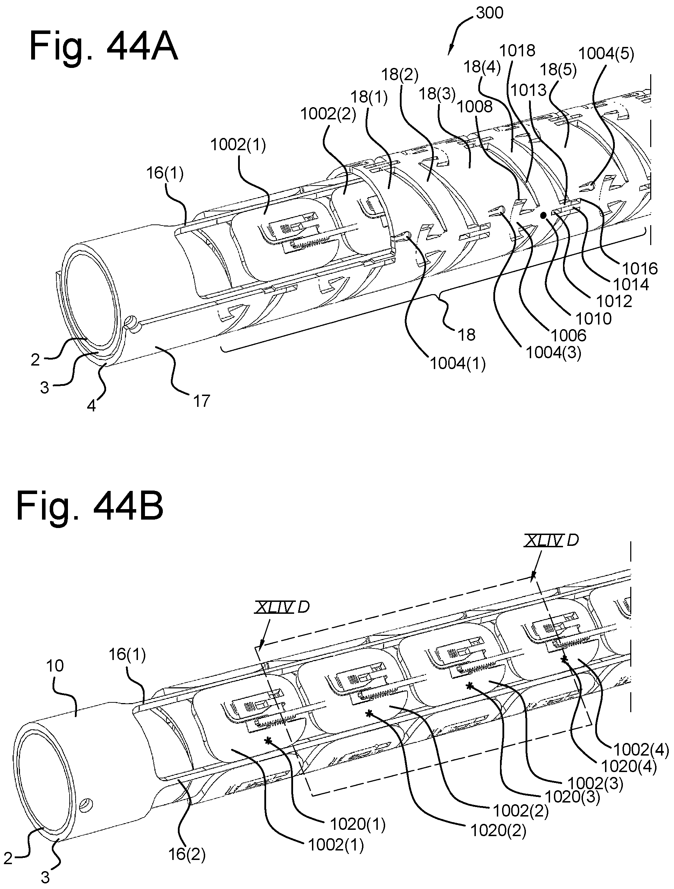

Figures 44A-44D show an embodiment in which a rotation angle between adjacent hinge segments 18(p), p = 1, 2, ..., P in deflectable portion 18 of outer tube 4 can be frozen. Figure 44A shows tip section 13 of three tubes inserted into one another, i.e., inner tube 2, intermediate tube 3 and outer tube 4. More tubes can be provided and the same mechanism can be applied in any bendable section of instrument 300. Outer tube 4 is shown in elaborated view such that a portion of intermediate tube 3 is also visible.

One or more T-shaped portions 1006 of a hinge segment 18(p) are accommodated in respective T-shaped slots 1008 of an adjacent hinge portion 18(p+1) and/or 18(p-1). In the shown embodiment there are two such T-shaped portions 1006 between two adjacent hinge segments 18(p), 18(p+1) tangentially shifted about 180 degrees. Between adjacent hinge segments 18(p+1), 18(p+2) again two such T-shaped portions 1006 are applied but then 90 degrees shifted, as shown, allowing portion 18 to be deflectable in all directions. Moreover, 90 degrees shifted relative to a set of a T-shaped portion 1006 and a T-shaped slot 1008, a set of a pin 1010 of a hinge segment extending into a slot 1012 in an adjacent hinge segment may be applied, configured to prevent mutual tangential rotation of adjacent hinge segments. Slot 1012 may be defined between two pins 1013 extending into slots 1016. Adjacent hinge segments 18(p) and 18(p+1) are separated by tangential slots 1018 configured such that these adjacent hinge segments 18(p), 18(p+1) can rotate relative to one another about point of rotation 1010 at a predetermined angle such that the hinge deflects.

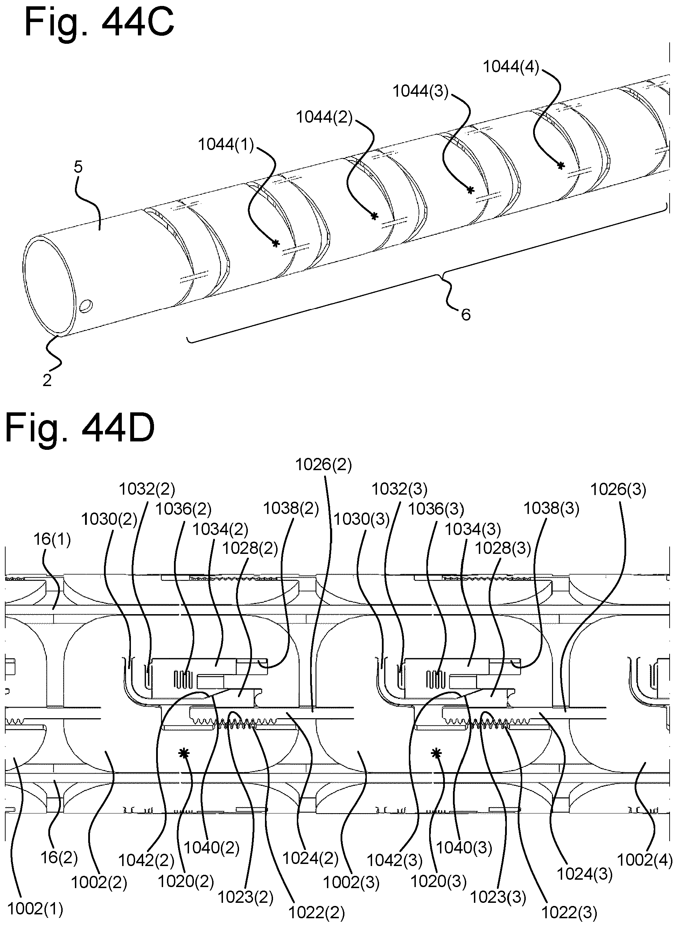

Figure 44B shows details of an embodiment of intermediate tube 3. An enlarged view of essential details is shown in figure 44D as indicated with a rectangle XLV^{D}-XL^{D} in figure 44B. Intermediate tube 3 has four steering wires, two of which 16(1), 16(2) are shown. In the shown deflectable tip section adjacent steering wires 16(1), 16(2) are separated by a series of spacers 1002(q), q = 1, 2, ..., Q. Note that in the shown example, the number of spacers 1002(q) is half the number of hinge segments 18(p). Each spacer 1002(q) is attached to a hinge segment such that if its attachment point 1020(q) is attached to an attachment point 1004(p) of hinge segment 18(p), an attachment point 1020(q+1) of adjacent spacer 1002(q+1) is attached to an attachment point 1004(p+2) of hinge segment 18(p+2). A switching element is implemented as a flexible lever 1026(q) which extends from a spacer 1002(q+1) inside adjacent spacer 1002(q) such that adjacent spacers 1002(q), 1002(q+1) can move relative to one another in the longitudinal direction.

Each spacer 1002(q) has a slot 1038(q) in which a slider 1034(q) is located such that slider 1034(q) can longitudinally move inside slot 1038(q). Slider 1034(q) may be resiliently attached to the body of spacer 1002(q) by means of a spring element 1032(q) and is attached to inner tube 2 at an attachment point 1036(q). Slider 1034(q) has a face 1042(q) touching a face 1040(q) of a lever 1028(q). Lever 1028(q) is resiliently attached to the body of spacer 1002(q) by means of a flexible arm 1030(q). Lever 1028(q) can be resiliently moved by slider 1034(q) in the tangential direction if angled faces 1040(q) and 1042(q) move relative to one another. At a longitudinal side opposite face 1040(q) lever 1028(q) touches a first longitudinal side of an end portion 1024(q) of flexible lever 1026(q) provided with serrations 1023(q) at a second longitudinal side. These serrations 1023(q) are arranged opposite serrations 1022(q) in spacer 1002(q). Spacer 1002(q) with its serrations 1022(q) function as a locking element.

Figure 44C shows an example of inner tube 2. In its flexible portion 6, inner tube 2 is provided with a hinge structure which has attachment points 1044(q). Attachment point 1044(q) is attached to attachment point 1036(q) of slider 1034(q). Inner tube 2 may have a different implementation as long as it is at least flexible in the flexible portion 18 of instrument 300 and stiff in its longitudinal direction.

The arrangement shown in figures 44A-44D operates as follows. In the shown condition, serrations 1023(q) do not engage serrations 1022(q) and, thus, adjacent spacers 1002(q), 1002(q+1) can move relative to one another in the longitudinal direction while at the same time allowing tip section 13 to be deflected by steering wires 16(i) at any desired angle in any direction. Once a desired deflection angle is obtained (which may be 0 degrees), inner tube 2 is pulled towards the proximal end causing all sliders 1034(q) to move longitudinally relative to the body of respective spacers 1002(q). This causes face 1042(q) to move relative to face 1040(q) causing lever 1028(q) to be pushed away tangentially towards lever portion 1024(q) and serrations 1023(q) engaging serrations 1022(q) resulting in blocking any further mutual longitudinal movement between adjacent spacers 1002(q), 1002(q+1) and, thus, freezing of the deflected condition of tip section 13. Thus, here, inner tube 2 and sliders 1034(q) which are attached to inner tube 2 operate as actuation elements.

By moving inner tube 2 towards the distal end of instrument 300, all sliders 1034(q) will be moved back to the position shown in figure 44D in which they do not exert any tangential force on levers 1028(q) anymore and serrations 1023(q) no longer engage serrations 1022(q). Then, adjacent hinge segments 18(p) are no longer locked.

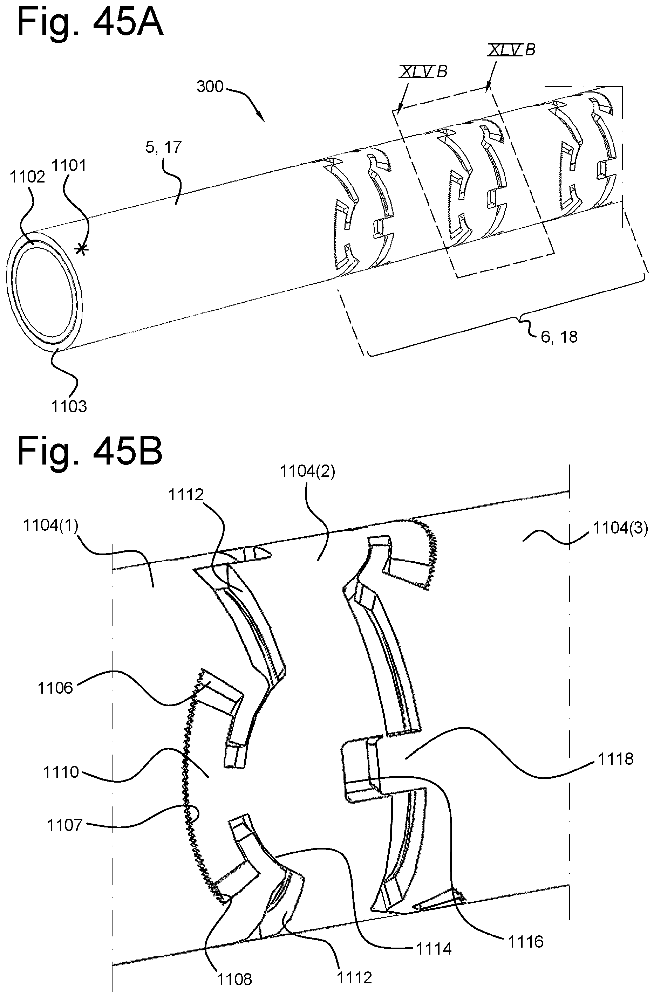

Figures 45A-45C show an implementation of how a hinge can be frozen in a deflected or non-deflected condition. Figure 45A shows two tubes 1102, 1103. Tube 1102 is inserted into tube 1103. The shown arrangement may be a portion of a tip section but it may be implemented anywhere in instrument 300. The arrangement may be steerable by suitable steering wires (not shown in figures 45A-45C). Tubes 1102 and 1103 are attached to one another at their ends, e.g., at their end edges or somewhere between the hinge and the end edges. The attachment is done at one or more locations 1101, e.g., by (laser) welding, gluing, etc. If desired, more than two tubes can be used.

Like the example shown in figures 38A, 38B adjacent hinge segments 1104(1), 1104(2), 1104(3), ... are crocheted together via one or more T-shaped extensions 1110 in one hinge segment 1104(1), 1104(2), 1104(3), ... accommodated in a T-shaped slot 1106 of an adjacent hinge segment 1104(1), 1104(2), 1104(3), .... T-shaped extension 1110 and T-shaped slot 1106 together form a clamp. Preferably two such sets of T-shaped extension 1110 and T-shaped slot 1106 are applied at two locations tangentially rotated at 180 degrees. T-shaped extension 1110 and T-shaped slot 1106 are configured such that adjacent hinge segments 1104(1), 1104(2), 1104(3), ... cannot or can hardly move in the longitudinal direction relative to one another but can rotate relative to one another by moving T-shaped extension 1110 in T-shaped slot 1106. Slots 1112 are provided between the two sets of T-shaped extension 1110 and T-shaped slot 1106. The shape of these slots 1112 determine the maximum angle of deflection between two adjacent hinge segments 1104(1), 1104(2), 1104(3), .... At its upper side T-shaped extension 1110 has a serrated portion 1107 facing a serrated portion 1108 of T-shaped slot 1106. Serrated portion 1107 and serrated portion 1108 together form a locking element.

At locations 90 degrees shifted in the tangential direction relative to the two sets of T-shaped extension 1110 and T-shaped slot 1106, a pin 1118 / slot 1116 set may be applied in opposite sides of two adjacent hinge segments 1104(1), 1104(2), 1104(3), ... configured to prevent tangential rotation of two adjacent hinge segments 1104(1), 1104(2), 1104(3), ....

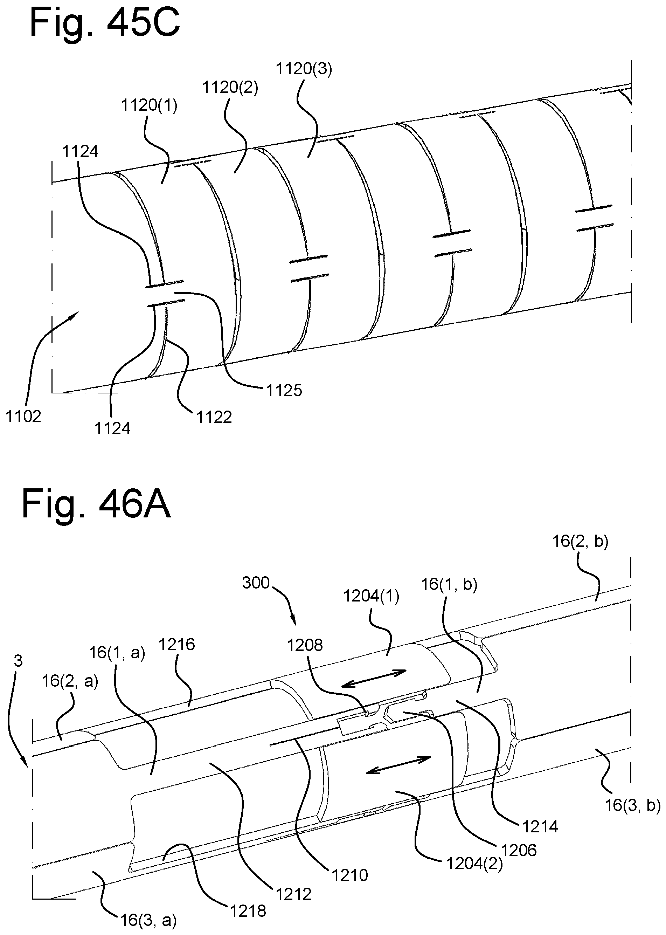

As shown in figure 45C, tube 1102 has a flexible structure, e.g., implemented by several hinge segments adjacent to one another such that tube 1102 can deflect in any direction, at least in the longitudinal area in which the hinge structure of tube 1103 is located. At the same time, tube 1102 should be designed such that a longitudinal pulling force applied on tube 1102 is transferred to a compression force on tube 1103 via one or more attachments 1101 (cf. figure 45A). Such attachments can be made by laser welding, gluing, etc. Here, tube 1102 is implemented by a hinge structure having adjacent hinge segments 1120(r), r = 1, 2, ..., R which are separated from one another by slots 1122 which are interrupted by bridges 1125 defined by two opposite longitudinally slots 1124. Preferably, there are two such bridges 1125 between two adjacent hinge segments 1120(r) located at 180 degrees tangentially shifted locations.

Tubes 1102 and 1103 are configured such that, as long as no longitudinal pulling force is applied to tube 1102, serrations 1107 and 1108 do not engage and T-shaped extension 1110 can move inside T-shaped slot 1106 without friction. However, when a pulling force is exerted on tube 1102 in the longitudinal direction away from the attached ends, a compression force is developed on hinge segments 1104(1), 1104(2), 1104(3), ... in tube 1103 causing serrations 1107 to engage serrations 1108 and, thus, freezing a current deflection condition between all adjacent hinge segments 1104(1), 1104(2), 1104(3), ... By relaxing this pulling force, the freezing condition can be made undone.

In the embodiment of figures 45A-45D, tube 1102 is used as an actuation element. However, instead of tube 1102 one or more actuation wires can be used, e.g., made by cutting them from a tube. More such sets of actuation wires can then be cut from the same tube, which can extend from the proximal end of instrument 300 to other flexible portions in instrument 300 and are configured to lock-unlock deflected conditions of such other flexible portions.

Figures 46A-46E show a locking system to lock steering wires or other elements to each other at one position. It can be cut out of a single tube, but often it is placed between an inner and outer support tube.

Figure 46A shows two steering wire portions 16(1,a) and 16(1,b) in a non-coupled condition. The two steering wire portions 16(1,a) and 16(1,b), respectively may be located between adjacent steering wires which may also consist of steering wire portions 16(2,a)/16(3,a) and 16(2,b)/16(3,b), respectively, which can be coupled to one another in the same way as steering wire portions 16(1,a) and 16(1,b).

Steering wire portion 16(1,a) is provided with a switching element 1212 having a slot 1208 at its tip portion and, possibly, a cut 1210 extending from slot 1208 into switching element 1212 in the longitudinal direction. Steering wire 16(1,b) is provided with an extension 1214 with a tip portion 1206. Sliders 1204(1) and 1204(2) are provided which, in the condition shown in figure 46A, both touch both switching element 1212 and extension 1214 such that switching element 1212 and extension 1214 cannot move in the tangential direction of instrument 300. Sliders 1204(1) and 1204(2), respectively, can move in the longitudinal direction in slots 1216 and 1218, respectively. Sliders 1204(1), 1204(2) are attached to suitable actuation elements. Such actuation elements may be implemented by actuation wires cut from the same tube as shown in figures 46A-46E or arranged inside or outside that tube. As a further alternative, an actuation tube or sleeve may be provided arranged inside or outside the tube shown in figures 46A-46E which is attached to sliders 1204(1), 1204(2) and configured to move them in the longitudinal direction.

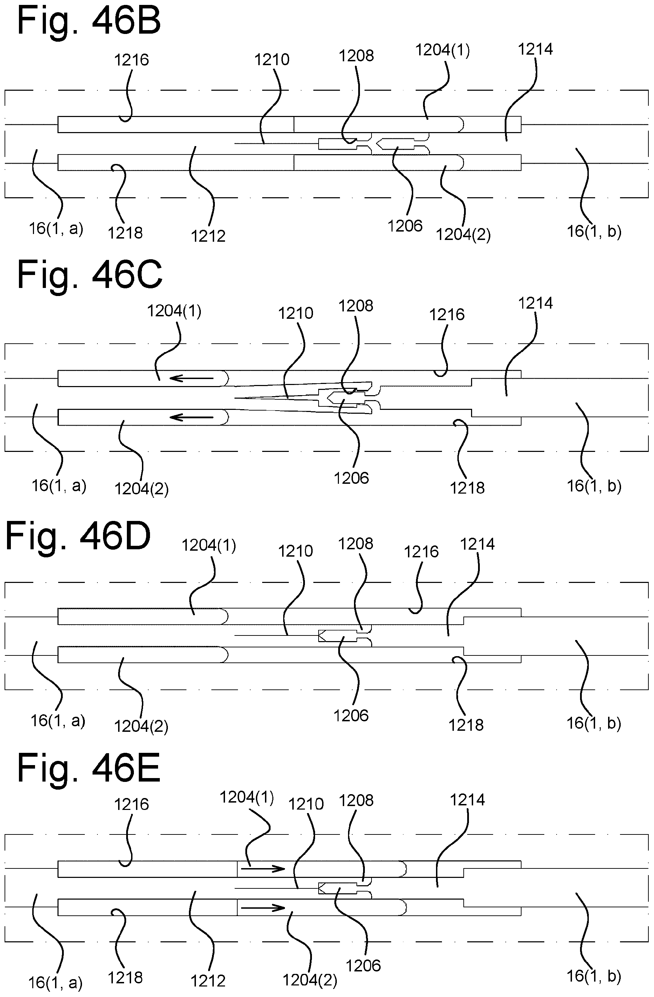

Figure 46B shows a side view of figure 46A.

Figure 46C shows a condition in which both sliders 1204(1), 1204(2) are moved in their slots 1216, 1218 such that they do no longer touch the tip portion with slot 1208 of switching element 1212. Tip portion 1206 of extension 1214 is then inserted into slot 1208 of switching element 1212 which can widen in the tangential direction, as shown in figure 46C.

As shown in figure 46D, tip portion 1206 of extension 1214 and tip portion with slot 1208 of switching element 1212 are mutually shifted such that tip portion 1206 is totally accommodated inside slot 1208 of switching element 1212 and they form a snap-fit connection. Then, as shown in figure 46E, sliders 1204(1), 1204(2) are shifted back to the positon where they both touch both switching element 1212 and extension 1214 such that slot 1208 cannot widen in the tangential direction and switching element 1212 of steering wire 16(1,a) fixedly clamps steering wire portion 16(1,b) such that if one of them moves in the longitudinal direction also the other one moves in the longitudinal direction to the same extent.

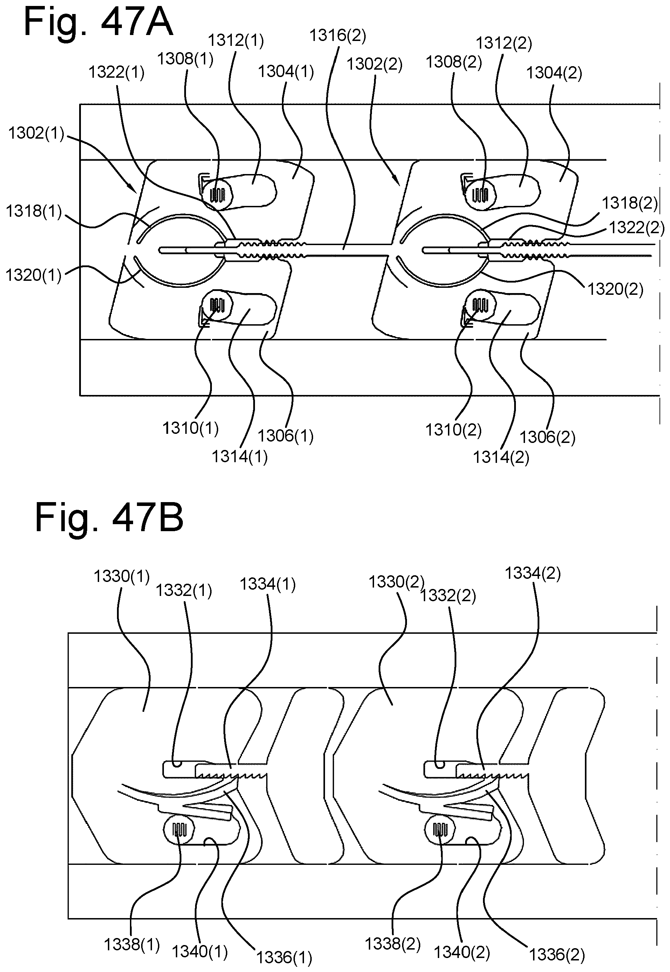

Figures 47A, 47B show an alternative to the example of figures 46A-46C. Figure 47A shows two adjacent switching elements of a series of switching elements 1302(s), s = 1, 2, ..., S. In this example they are identically shaped. Switching element 1302(s) has two switching element portions 1304(s), 1306(s). They are attached to one another at a point of attachment one longitudinal side. Opposite to this point of attachment, i.e., at its other longitudinal side, switching element 1302(s) has a slot 1322(s) separating the two slot portions 1304(s), 1306(s). In the shown embodiment, at mutual sides of slot 1322(s), both slot portions 1304(s), 1306(s) have serrated longitudinal edges facing one another. The two slot portions 1304(s), 1306(s) are configured to hinge relative to one another about the point of attachment. Extra slots 1318(s) and 1320(s) may be present inside the two switching element portions 1304(s) and 1306(s) to facilitate the hinging. Thus, slot 1322(s) can be made wider and smaller. Switching element 1302(s+1) has a longitudinal extension 1316(s+1) directed towards switching element 1302(s) and possibly provided with a serrated end portion.

Each one of the switching element portions 1304(s) and 1306(s), respectively, may be provided with a slot 1312(1) and 1314(s), respectively. Pins 1308(s) and 1310(s), respectively, are provided inside slots 1312(s) and 1314(s), respectively. These pins 1308(s) and 1310(s) are attached to one or more actuation elements (not shown) in a tube inside or outside the tube in which the switching elements 1302(s) are made. Slots 1312(s) and 1314(s) are shaped such that when pins 1308(s) and 1310(s) are moved in the longitudinal direction by these one or more actuation elements switching element portions 1304(s) and 1306(s) are forced to hinge relative to one another such as to open or close slot 1308(s). In the open condition longitudinal extension 1316(s+1) can be inserted into slot 1322(s). Once inside slot 1322(s) switching element portions 1304(s) and 1306(s) can be moved towards one another such that the serrated edges of the switching element portions 1304(s), 1306(s) engage the serrated end portion of longitudinal extension 1316(s+1), and thus switching element longitudinal extension 1316(s+1).

The serrations can have any form as explained earlier with reference to figures 27A-27H. Moreover, the shown switching element 1302(s) can also be used to clamp an end portion of a steering wire or any other longitudinal element. The one or more actuation elements can be implemented as longitudinal strips running towards the proximal end of the instrument.

Figure 47B shows a further alternative, in which a switching element 1330(s) is provided with a slot 1332(s) which has fixed dimensions. A serrated longitudinal extension 1334(s) of another element can be inserted into slot 1332(s). Switching element 1330(s) has a resilient switching element 1336(s) which can be moved into the tangential direction by longitudinally moving a pin 1338(s) inside slot 2340(s). Pin 1338(s) is attached to an actuation element (not shown) in a tube inside or outside the tube in which switching element 1330(s) is made. Once longitudinal extension 1334(s) is inserted in opening 1332(s) switching element 1336(s) is moved towards longitudinal extension 1334(s) by moving pin 1338(s) in the longitudinal direction by the actuation element such as to clamp its serrated portion and block longitudinal movement thereof.

Again, the serrations can have any form as explained earlier with reference to figures 27A-27H. Moreover, the shown switching element 1330(s) can also be used to clamp an end portion of a steering wire or any other longitudinal element. The one or more actuation elements can be implemented as longitudinal strips running towards the proximal end of the instrument.

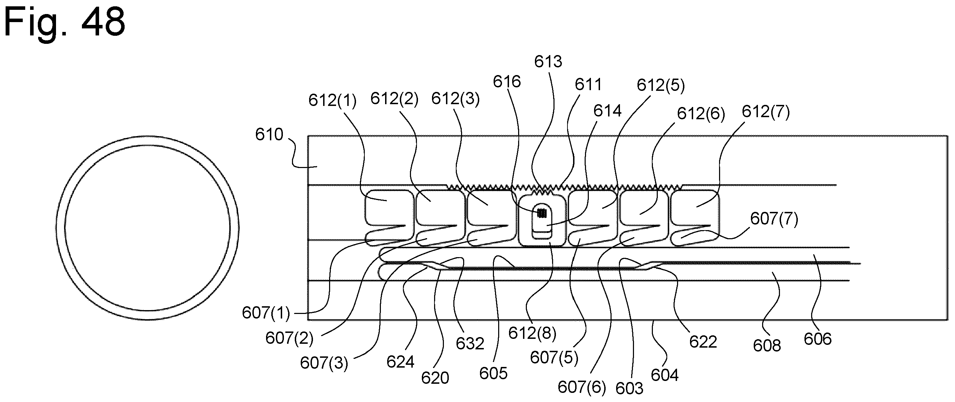

The embodiment of figure 48 contains features of other figures likes figures 22A, figure 22B and 26. the same reference numbers refer to the same or similar elements as in those figures. In the shown embodiment, all elements are portions of a tube wall, as indicated with the circular cross section of a tube at the left hand side of the figure.

The embodiment has two longitudinal elements: a longitudinal actuation element 606 and a longitudinal actuation element 608 which are both slidable in the longitudinal direction. Longitudinal actuation element 608 is guided by fixed world portion 604.

Longitudinal actuation element 608 is provided with two faces 622 and 624 such as to provide longitudinal actuation element 608 with an indented portion between them which is widening towards the outside of longitudinal actuation element 608. Longitudinal actuation element 606 is provided with two faces 603 and 632 such as to provide longitudinal actuation element 606 with an extending portion between them which is tapering towards the outside of longitudinal actuation element 606. The extending portion of longitudinal actuation element 606 is located inside the indented portion of longitudinal actuation element 608 and has a shorter length than the length of that indented portion of longitudinal actuation element 608. Thus, the extending portion of longitudinal actuation element 606 is able to longitudinally move inside the indented portion of longitudinal actuation element 608.

Moreover, the embodiment has a plurality of spacer elements 612(1), 612(2), 612(3), 612(5), 612(6), and 612(7), and sliding element 612(8) here located between spacer elements 612(3) and 612(5). All spacer elements 612(1), 612(2), 612(3), 612(5), 612(6), and 612(7), respectively, are provided with a resilient extension 607(1), 607(2), 607(3), 607(5), 607(6), and 607(7), respectively, which resiliently contact longitudinal actuation element 606. Sliding element 612(8) is slidable in the tangential direction as guided by guiding element 614 which is, again, attached - e.g. by welding - to an underlying or overlying tube at a point of attachment 616.

Sliding element 612(8) is slidable towards and away from longitudinal element 610 which may be a steering wire. At its side facing longitudinal element 610 sliding element 612(8) is provided with serrated portion 613 here shown to have teeth but any other serrated form may be applied, e.g. one of the forms shown in the figures 27A-27H. At its side facing sliding element 612(8) longitudinal element 610 is provided with serrated portion 611 here also shown to have teeth but any other serrated form may be applied, e.g. one of the forms shown in the figures 27A-27H. instead of a serration portion 611 and 613 may have a rough or non-smooth surface such that they show some friction when contacting one another.

In the embodiment of figure 48, longitudinal element 610 and fixed world portion 604 have a fixed tangential position relative to one another. Thus, if longitudinal actuation element 608 is moved in the longitudinal direction relative to longitudinal actuation element 606, at a certain moment in time, longitudinal actuation element 606 will be forced to move in the tangential direction towards sliding element 612(8) because either face 603 will slide along face 622 or face 632 will slide along face 624. This forces sliding element 612(8) to move in the tangential direction towards longitudinal element 610 until its serrated portion 613 engages serrated portion 611 of longitudinal element 610, thus, locking longitudinal element 610.

The main benefit of the embodiment of figure 48 is the higher amount of locks that can be created along a certain path length. Because the two longitudinal actuation elements 606 and 608 have their neutral (bending) axis rather close to each other the displacement of these elements with respect to each other is limited in a tortuous path. Thus, face 624 can be kept quite close to face 632 and face 603 quite close to face 622. This results in small actuation movements of at least one of the longitudinal actuation elements to create a lock. Stated differently, this embodiment has only a limited hysteresis.

This embodiment can also be used to steer the tip of the instrument if both longitudinal actuation elements 606, 608 are moved in the same longitudinal direction and the instrument can be locked if their movement is opposite. Of course, then, one of the longitudinal actuation elements needs to be connected to the distal end (tip) of the instrument. In an example, then, longitudinal element 610 is substituted by longitudinal actuation elements 606 and 608. Longitudinal actuation element 608 is e.g. in such example located adjacent the spacers 612(1), 612(2), 612(3), 612(5),... and provided with a serrated side like serrated side 611 of longitudinal element 610. The opposite sides of these spacers, then, contact a portion of the tube such that they can move in the tangential direction in a resilient way. Sliding element 612(8) remains the same.

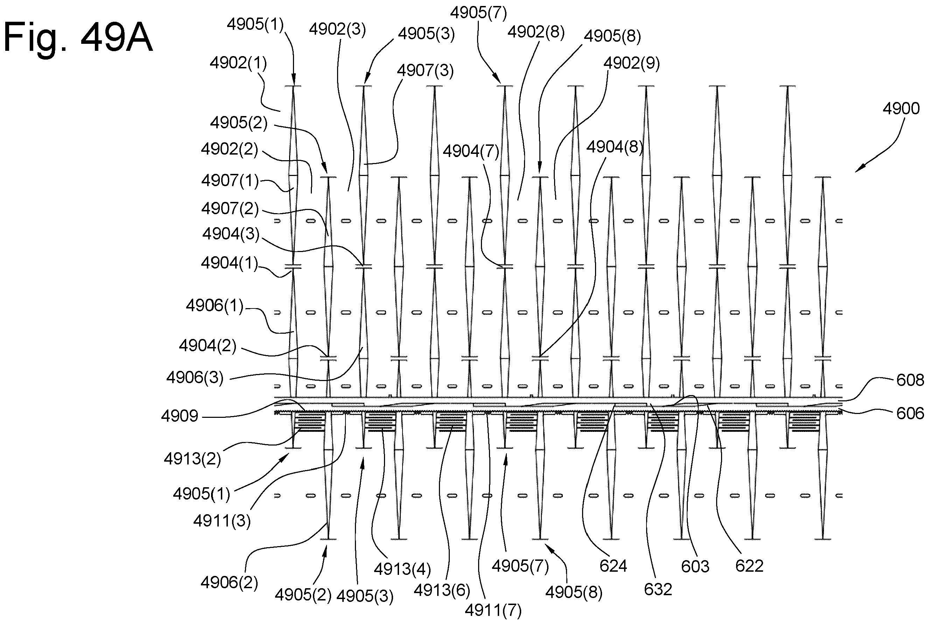

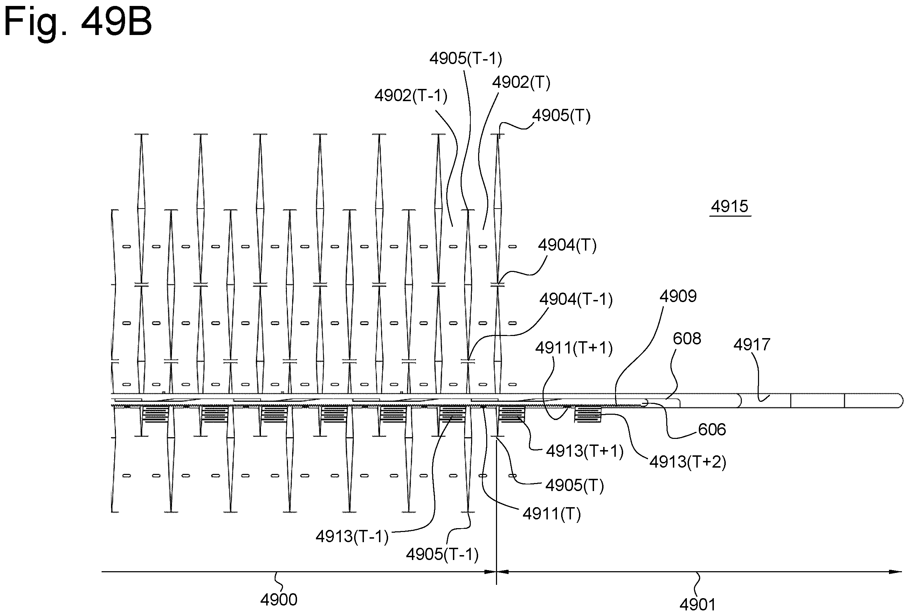

Figures 49A, 49B show an application of the idea of using two longitudinal actuation elements 606 and 608. Figure 49A shows a section 4900 of a tube which is entirely flexible and, thus, can take a curved shape while being inserted into a curved canal. The entire flexible section 4900 can be frozen or locked in in its curved (or uncurved) state. The elements shown are, again, cut in the wall of the tube and the figure shows all elements in a flattened view. The figure covers the entire 360 degrees of the tangential direction of the tube.

Longitudinal actuation elements 606 and 608 are, again, provided with faces 603, 622, 624, 632 like in figure 48. Though the reference numbers of these four faces are only shown once in figure 49A, such quartets of faces 603, 622, 624, 632 are present at regular intervals along the entire length of flexible section 4900, as one may see from figure 49A. In the shown embodiment, faces 624 and 632 are both oriented at an angle of 90 degrees relative to the axial direction. So, they cannot slide along one another. Once longitudinal actuation elements 606, 608 are in their unlocked position (as shown in figures 49A, 49B), movement of longitudinal actuation element 608 to the distal end (i.e., left direction) causes face 632 to be blocked against face 624, such that longitudinal actuation elements 606, 608 will end up in a well defined longitudinal relationship.

Flexible section 4900 has a plurality of adjacent flexible section portions 4902(1), 4902(2), 4902(t), ......4902(T). Here, 16 such flexible section portions 4902(t) are shown. However, the concept also works with any other number of two and more flexible section portions 4902(t). adjacent flexible section portions 4902(t), 4902(t+1) are, in this embodiment, attached to one another by means of two small longitudinal bridges 4904(t), 4905(t) which are located at a tangential distance of 180 degrees from one another. Note that one side of small bridges 4905(t) is shown at the upper part of figure 49A and the other side of small bridge 4905(t) is shown on the lower side of figure 49A. in reality these sides have a certain, predesigned tangential distance which is not visible in figure 49A. Between the two bridges 4904(t), 4905(t) adjacent flexible section portions 4902(t), 4902(t+1) are separated by slots 4906(t), 4907(t) which may be wider in their middle part than close to the bridges 4904(t), 4905(t).

Flexible section portion 4902(t+1) is attached to flexible section portion 4902(t+2) also by means of two small bridges 4904(t+1) and 4905(t+1). These small bridges 4904(t+1) and 4905(t+1), respectively, are present at locations 90 degrees tangentially shifted relative to the locations of small bridges 4904(t) and 4905(t), respectively. This alternating tangential locations allows the tube shaped flexible section 4900 to be flexible in all directions, as the persons skilled in the art will appreciate. It is observed that, alternatively, small bridges 4904(t+1) and 4905(t+1), respectively, may be present at locations zero degrees tangentially shifted relative to the locations of small bridges 4904(t) and 4905(t), respectively. Then, flexible section 4900 can only bend in one plane. Further alternative arrangements are possible.

Though a specific flexible section is shown, it will be apparent to persons skilled in the art that a tube can be made flexible by many other slot patterns cut in the tube. A wide variety of examples is known from the prior art and my be applied instead. Combinations of different slot patterns may be applied as well.

Longitudinal actuation element 606 is, here, provided with a serrated side 4909 facing flexible section portions 4902(t) which serrated side 4909 may have any form as discussed above. Alternatively, this side 4909 may have a roughened surface. Several of the flexible section portions 4902(t) are provided with a serrated portion 4911(t) at their longitudinal side facing serrated side 4909 of longitudinal actuation element 606 for certain values of t=t1. Other flexible section portions 4902(t) are not provided with such a serrated portion 4911(t) but with a spring like portion 4913(t) at their longitudinal side facing serrated side 4909 of longitudinal actuation element 606 for values of t=t2 where t‡t2. Here, an embodiment is shown in which serrated portions 4911(t1) alternate with spring like portions 4913(t2). However, other arrangements may be designed depending on specific requirements.

Important is that, at regular intervals, longitudinal actuation element 606 can engage and thus lock into several flexible section portions 4902(t1) along a predetermined lockable length of flexible section 4900, and that, in the unlocked state, spring like portions 4913(t2) push serrated side 4909 of longitudinal actuation element 606 away from serrated portions 4911(t1).

Figure 49B shows a proximal section 4901 as well as flexible section 4900. In the shown example, flexible section 4900 ends at the proximal section 4901, however, that is not strictly necessary. There may be one or more other sections in between. Here, proximal end 4901 is a portion of the tube with mostly solid material 4915. Both longitudinal actuation elements 606, 608 have an end portion inside a slot 4917 in proximal section 4901. Serrated side 4909 of longitudinal actuation element 606 extends into this slot 4917 in proximal section 4901. Opposite to this serrated side 4909, proximal section 4901 has a further serrated portion 4911(T+1) and a further spring like portion 4913(T+2) configured to push serrated side 4909 away from serrated portion 4911(T+1) in the unlocked condition of the tube. In this unlocked condition, both longitudinal actuation elements 606 and 608 can move in the longitudinal direction inside slot 4917.

In use, when flexible section 4900 is bent, e.g., due to being inserted in a curved (or steered) canal, longitudinal actuation elements 606, 608 will also be curved in accordance with the curved canal. However, at different locations they may be shifted in the longitudinal direction relative to one another due to the fact they are not located on the same tangential location on the curved tube. The same is true for serrate side 4909 of longitudinal actuation element 606 and serrated portions 4911(t) of flexible section portions 4902(t). When flexible section 4900 is curved there may, e.g., be less or more "teeth" of serrated side 4909 between two adjacent serrated portions 4911(t) than in a straight, non-curved state of flexible section 4900. Moreover, mutual distances between adjacent "teeth" of serrations 4911(t) may slightly change due to curvature of longitudinal actuation element 606. To prevent mismatch of these "teeth" with "teeth" of serrated portions 4911(t), longitudinal sides of flexible section portions 4902(t) may be provided with only few such "teeth" not covering the entire length of these longitudinal sides.

During inserting flexible section 4900 in a curved canal, spring like portions 4913(t) push serrated portions 4911(t) away from serrated side 4909 and the two longitudinal actuation elements 606, 608 can both longitudinally move independently from one another to a certain extent. Once flexible section 4900 is located at a desired location in the canal one may wish to lock (or freeze) flexible section 4900 in the obtained curved state such that another longitudinal instrument can easily be shifted through its lumen to a target location. Such locking can now easily be done by a relative longitudinal movement between longitudinal actuation elements 606, 608. As explained with reference to figure 48, when e.g. longitudinal actuation element 608 is moved in the right direction relative to longitudinal element 606, once face 603 contacts and is guided by face 622, longitudinal actuation element 606 is pushed in the tangential direction against the spring action of spring like portions 4913(t) causing serrated side 4909 to engage serrated portions 4911(t). This engagement at regular intervals causes longitudinal actuation element 606 to be locked in its curved state, thus, keeping the entire flexible section 4900 in its curved state.

Note that, in this locked state of flexible section 4900, serrated side 4909 has also engaged serrated portion 4911(T+1) inside proximal section 4901. This is not a strictly necessary feature but if applied it prevents flexible section 4900 from being bendable relative to proximal section 4901 in its locked state.

The proximal end of longitudinal actuation element 608 inside proximal section 4901 can be moved in the longitudinal direction by any suitable means. It may, e.g., be attached to a control mechanism in a tube surrounding the tube shown in figures 49A, 49B. Such control mechanism may, e.g., be operated manually or by suitable robotic equipment.

Though figures 49A, 49B show one set of longitudinal actuation elements 606, 608, two or more such sets may applied as well, preferably located at mutually equal tangential distances.

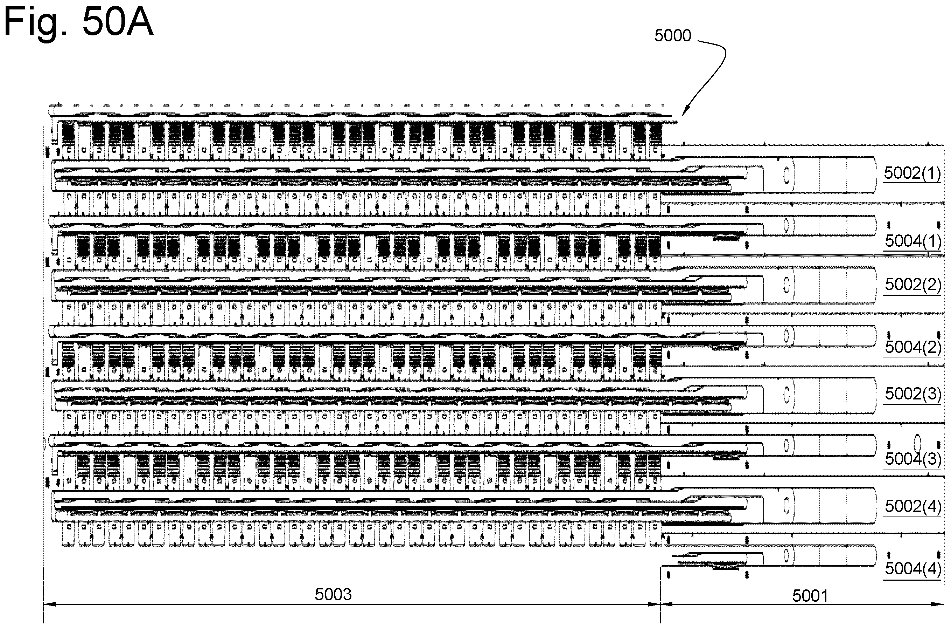

Figures 50A-50F show a further development of the embodiments shown in figures 48, 49A, 49B, which can be used in a so-called "follow-the-leader" (FTL) instrument. In the prior art such FTL instruments are implemented by an inner core and an outer core, e.g., a first tube (assembly) acting as inner core located inside a second tube (assembly) acting as outer core. For the purpose of the present specification such a prior art instrument is assumed to be known to the skilled person. Here, an embodiment is presented having all main elements implemented inside a single tube manufactured by providing the tube with a suitable slot pattern, e.g., by laser cutting. Though all elements are located inside a single tube, the prior art terms "inner core" and "outer core" will used in order to use corresponding words from the prior art. Some elements of the "inner core" will be indicated with subscript "ic" whereas some elements of the "outer core' will be indicated with subscript "oc". Figures 50A-50F show the tube elements again in flattened form. In reality they cover the entire 360 degrees circumference of the tube.

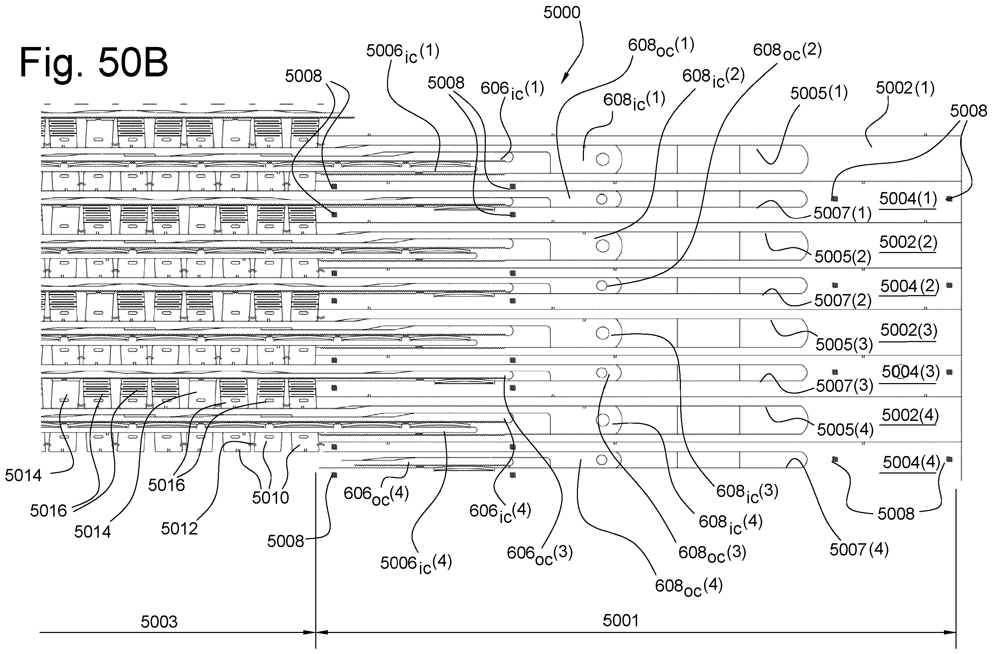

Figure 50A merely shows an overview of a FTL tube 5000. All details are shown in one or more of the other figures 50B-50F which show portions on an enlarged scale. Figure 50A shows an example of a complete FTL tube 5000. In practise, there may be one or more other tubes inside and/or outside FTL tube 5000. The size of the elements may not be on scale, e.g., the scale may be different in the vertical and horizontal directions.

In the shown embodiment, FTL tube 5000 comprises eight parallel sections: four inner core sections 5002(u), u = 1, 2, 3, 4 relating to the "inner core", and four outer core sections 5004(4) relating to the "outer core". In other embodiments, u may have another maximum value than 4. A proximal end section is indicated with reference sign 5001 and a flexible remaining section is indicated with reference sign 5003.

As better shown in figure 50B, proximal ends of the outer core sections 5004(u) are attached at several locations 5008 to one or more components inside or outside FTL tube 5000 such that the outer core sections 5004(u) form "fixed world" portions of the FTL tube 5000. Such attachments can be made by any suitable means including laser welding and small pins bent into a suitable opening in another tube. Every inner core section 5002(u) is provided with a longitudinal slot 5005(u), and every outer core section 5004(u) is provided with a longitudinal slot 5007(u).

Inside each longitudinal slot 5005(u) inner core section 5002(u) comprises three parallel longitudinal actuation elements 606_{ic}(u), 608_{ic}(u), and 5006_{ic}(u). In the shown example, longitudinal actuation element 5006_{ic}(u) is located between longitudinal actuation elements 606_{ic}(u) and 608_{ic}(u).

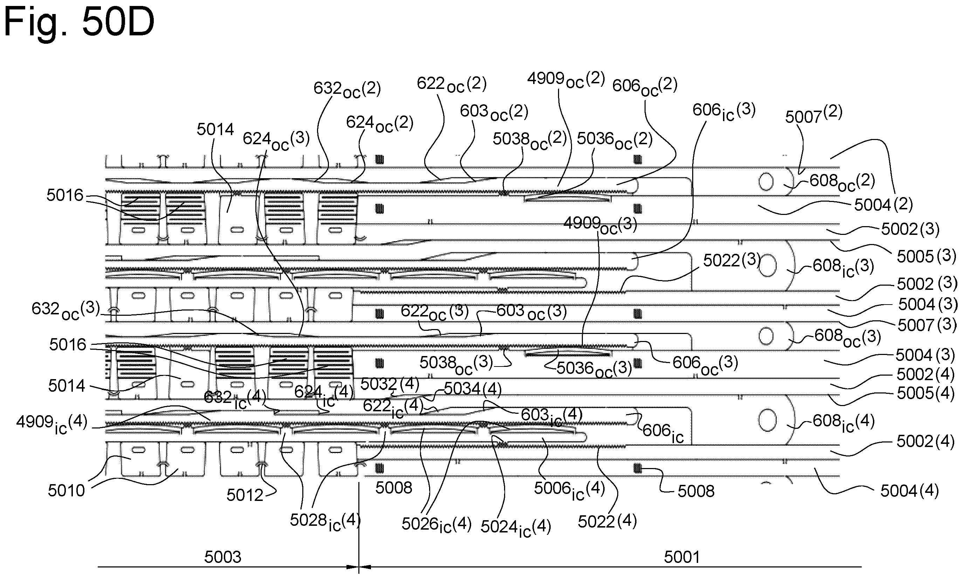

As better visible in figure 50D - which shows an enlarged view of a portion of proximal end section 5001- longitudinal actuation elements 606_{ic}(u) and 608_{ic}(u) are identically shaped as longitudinal actuation elements 606, 608 in the embodiment of figures 49A, 49B. At regular intervals they are provided with faces 603_{ic}(u), 622_{ic}(u), 624_{ic}(u), 632_{ic}(u) and longitudinal actuation element 606_{ic}(u) is provided with a serrated longitudinal side 4909_{ic}(u) facing away from longitudinal actuation element 608_{ic}(u). At its longitudinal side facing longitudinal actuation element 606_{ic}(u) longitudinal actuation element 5006_{ic}(u) is provided with serrated portions 5028_{ic}(u), as well as spring like portions 5026_{ic}(u). Spring like portions 5026_{ic}(u) are designed such that they push against serrated side 5030_{ic}(u) and prevent serrated portions 5028_{ic}(u) to engage serrated side 5030_{ic}(u) in the unlocked state of the tube.

Inside the proximal end of the tube, at its longitudinal side facing away from longitudinal actuation element 606_{ic}(u) longitudinal actuation element 5006_{ic}(u) is provided with a serrated portion 5024_{ic}(u) located opposite a serrated longitudinal side 5022(u) of the proximal end of inner core section 5002(u).

At its longitudinal side facing away from longitudinal actuation element 606_{ic}(u) longitudinal actuation element 608_{ic}(u) is provided with an angled face 5034(u). Opposite to this angled face 5034(u) the proximal end of inner core section 5002(u) is provided with an angled face 5032(u). Thus if longitudinal actuation element 608_{ic}(u) is moved in the longitudinal direction relative to this proximal end of inner core section 5002(u) such that angled faces 5032(u), 5034(u) slide along one another, a tangential force will be developed on longitudinal actuation element 608_{ic}(u) in the direction of longitudinal actuation element 606_{ic}(u).

Inside each longitudinal slot 5007(u) outer core section 5004(u) comprises two parallel longitudinal actuation elements 606_{oc}(u), 608_{oc}(u) which are, in this embodiment, identically shaped as longitudinal actuation elements 606_{ic}(u), 608_{ic}(u). The proximal end of outer core section 5004(u) has a longitudinal side facing serrated side 4909_{oc}(u) which is provided with a serrated portion 5038_{oc}(u) and a spring like portion 5036_{oc}(u). Spring like portion 5036_{oc}(u) pushed longitudinal actuation element 606_{oc}(u) away such that serrated portion 5038_{oc}(u) does not engage serrated side 4909_{oc}(u) of longitudinal actuation element 606_{oc}(u) in the unlocked state of the tube.

Inside flexible section 5003 every longitudinal actuation element 5006_{ic}(u) is separated from an adjacent longitudinal actuation element 608_{oc}(u) by means of spacers 5010, 5012 which may have any suitable form. Here, they are shown as quadrangle elements of which some adjacent ones are attached to one another by a small flexible bridge 5012.

Moreover, inside flexible section 5003 every longitudinal actuation element 606_{oc}(u) is separated from an adjacent longitudinal actuation element 608_{ic}(u+1) by means of spacers 5014, 5016. Spacers 5014 are provided with a serrated portion facing serrated side 4909_{oc}(u) and spacers 5016 are spring like such that, in the unlocked state of the tube, they push longitudinal actuation element 606_{oc}(u) away in the tangential direction such as to prevent serrated spacer 5014 from locking into serrated side 4909_{oc}(u).

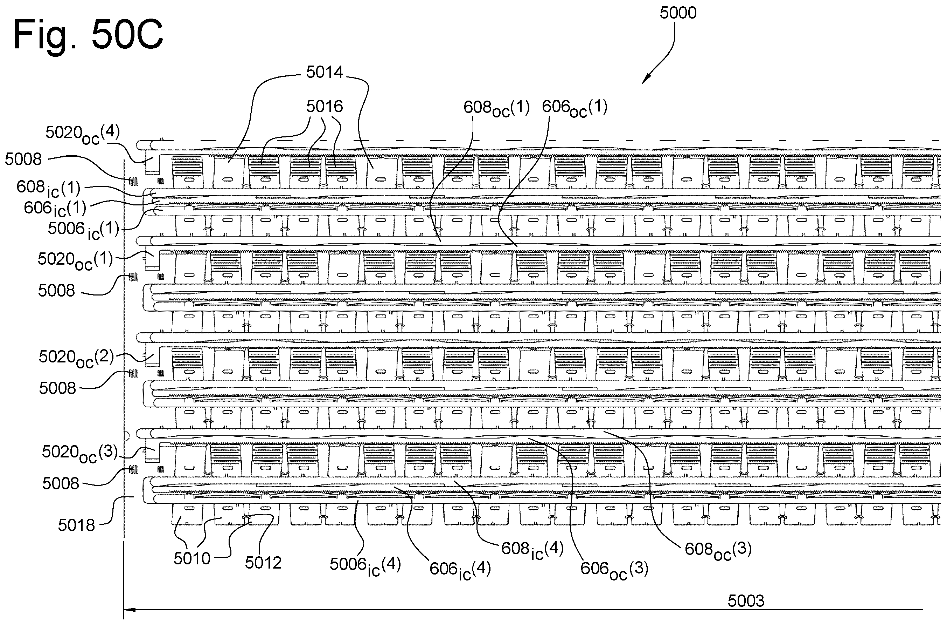

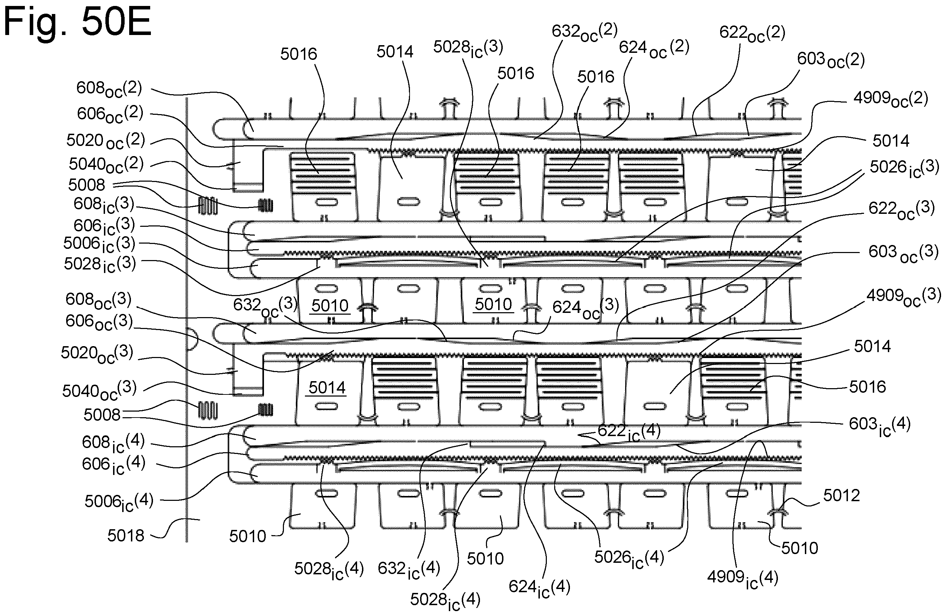

Figure 50C shows the tip section at the distal end of the tube, whereas figure 50E a portion of figure 50C on an enlarged scale. The figures show that, in this embodiment, the tip section is implemented by a ring shaped distal end 5018. As shown, the three parallel longitudinal actuation elements 606_{ic}(u), 608_{ic}(u) and 5006_{ic}(u) of the inner core sections 5002(u) are free-floating in the distal end of the tube, and so are longitudinal actuation elements 608_{oc}(u) of the outer core sections 5004(u). The only not free-floating longitudinal actuation element in this embodiment is longitudinal actuation element 606_{oc}(u) which is provided with a transverse portion 5020_{oc}(u) which is arranged inside a transverse slot 5040_{oc}(u) such as to be able to shift inside transverse slot 5040_{oc}(u).

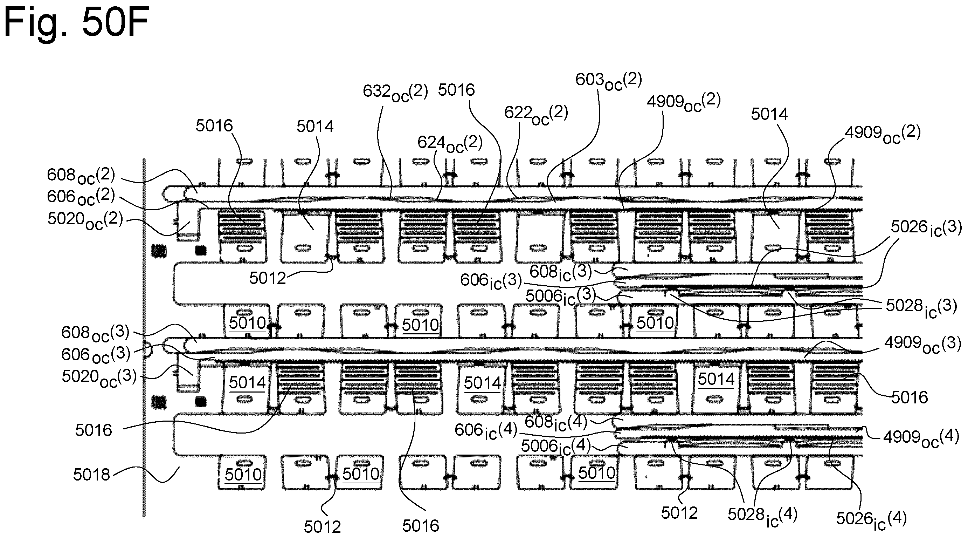

Figure 50F also shows the distal end of the tube, however, in the view of figure 50F, the outer core sections 5004(u) are moved in the distal direction of the tube relative to the three parallel longitudinal actuation elements 606_{ic}(u), 608_{ic}(u) and 5006_{ic}(u) of the inner core sections 5002(u). Thus, figures 50E and 50F show two different states of the tube while the tube is used in a FTL method, as will be further explained now.

In the starting position the tube may be straight and the positions of all elements may be as shown in figures 50A-50E. In use, the tube may be inserted into a curved canal causing flexible section 5003 of the tube to take the curved shape of the canal. While inserting the tip, here the ring 5018 may be steered by longitudinal actuation elements 606_{oc}(u) due to these longitudinal actuation elements 606_{oc}(u) being connected to the tip by means of the transverse portions 5020_{oc}(u). Here, there are four such longitudinal actuation elements 606_{oc}(u) which are equidistantly distributed in the tangential direction of the tube such as to control bending of the tip in all directions. Thus, the longitudinal actuation elements 606_{oc}(u) also act as steering wires in this embodiment. The bending of the tip may be assisted by a camera arranged on the tip.

During the first step of inserting into such a canal the longitudinal positions of the other longitudinal actuation elements 606_{ic}(u), 608_{ic}(u), 5006_{ic}(u) and 608_{oc}(u) may change due to the bending of flexible section 5003, as will be understand by the skilled person. The tube is designed such that these longitudinal actuation elements 606_{ic}(u), 608_{ic}(u), 5006_{ic}(u) and 608_{oc}(u) have some space in the longitudinal direction to move freely in the unlocked state of the tube, as one can see in figures 50A-50F. Also, due to the bending of flexible section 5003, the longitudinal actuation elements 606_{ic}(u), 608_{ic}(u), and 5006_{ic}(u) of the inner core sections may have different mutually relative longitudinal positions than the ones shown in these figures.

Once inserted to a certain depth into the canal one may decide to freeze the curved tube in its current state. That is done by operating the longitudinal actuation elements 606_{ic}(u), 608_{ic}(u), and 5006_{ic}(u). I.e., longitudinal actuation elements 606_{ic}(u), 608_{ic}(u) are shifted relative to one another in the longitudinal direction such that the set of faces 603_{ic}(u), 622_{ic}(u) slide along one another causing longitudinal actuation element 606_{ic}(u) to move in the tangential direction towards longitudinal actuation element 5006_{ic}(u) against the spring force of spring like elements 5026_{ic}(u). This causes serrated side 4909_{ic}(u) to engage serrated portions 5028_{ic}(u) which will lock longitudinal actuation element 606_{ic}(u) in its current curved state. Because there are four such, now locked, longitudinal actuation elements 606_{ic}(u) of inner core section 5002(u) distributed equidistantly in the tangential direction, the entire flexible section 5003 will now be locked in the curved state.

Note that, in the shown embodiment, also serrated portions 5024_{ic}(u) will lock into serrated sides 5022(u) preventing them from longitudinal movement relative to the proximal ends of the inner core sections 5002(u).

However, all elements of the outer core sections 5004(u) - here together with all spacers 5014, 5016 - are still able to be moved in the distal direction along the longitudinal actuation elements 606_{ic}(u), 608_{ic}(u) and 5006_{ic}(u). So, as a second step, the outer core sections 5004(u) are advanced along a certain distance in the distal direction while the longitudinal actuation elements 606_{ic}(u), 608_{ic}(u) and 5006_{ic}(u) keep their position and keep the flexible section 5003 in their curved state in the axial position of these longitudinal actuation elements 606_{ic}(u), 608_{ic}(u) and 5006_{ic}(u). During this advancement, the tip may be steered again as controlled by longitudinal actuation elements 606_{oc}(u) and , e.g., assisted by a camera arranged on the tip.

Then, as a third step, longitudinal actuation element 608_{oc}(u) is shifted relative to longitudinal actuation element 606_{oc}(u) in the longitudinal direction such that the set of faces 603_{oc}(u), 622_{oc}(u) slide along one another causing longitudinal actuation element 606_{oc}(u) to move in the tangential direction towards spacers 5014 against the spring force of spring like spacers 5016. This causes serrated side 4909_{oc}(u) to engage serrated portions of spacers 5014 which will lock longitudinal actuation element 606_{oc}(u) in its current curved state. Because there are four such, now locked, longitudinal actuation elements 606_{oc}(u) of the outer section 5004(u) distributed equidistantly in the tangential direction, the entire flexible section 5003 is now also locked in the curved state by elements of the outer core section 5004(u).

Then, in a fourth step longitudinal actuation elements 606_{ic}(u), 608_{ic}(u) are shifted relative to one another in the longitudinal direction again such that the set of faces 603_{ic}(u), 622_{ic}(u) slide along one another but now in a direction to return to the unlocked state in which serrated side 4909_{ic}(u) no longer locks into serrated portions 5028_{ic}(u).

In a fifth step, all the longitudinal actuation elements 606_{ic}(u), 608_{ic}(u) and 5006_{ic}(u) are advanced in the distal direction to a desired new axial position while all other elements remain in place.

Steps 1 until 5 are repeated as many times one wishes and as possible.

Note that to perform the mutually relative shifts between longitudinal actuation elements 606_{ic}(u), 608_{ic}(u) and longitudinal actuation elements 606_{oc}(u), 608_{oc}(u), the longitudinal actuation elements 608_{oc}(u) and 608_{ic}(u) may all be attached to components outside the tube which may be configured for manual or robotic control.

Now, some final remarks are made.

The material removal means can be a laser beam that melts and evaporates material or a water jet cutting beam and this beam can have a width of 0.01 to 2.00 mm, more typically for this application, between 0.015 and 0.04mm.

The wall thickness of tubes depend on their application. For medical applications the wall thickness may be in a range of 0.03-2.0 mm, preferably 0.03-1.0 mm, more preferably 0.05-0.5 mm, and most preferably 0.08-0.4 mm. The diameter of the tubes depend on their application. For medical applications the diameter may be in a range of 0.5-20 mm, preferably 0.5-10 mm, more preferably 0.5-6 mm. The radial play between adjacent tubes may be in range of 0.01 - 0.3 mm.

Steering wires in one tube can be attached to longitudinal and other elements in adjacent tubes such that they are together operable to transfer a longitudinal motion from a steering wire at the proximal end of the instrument to a bendable portion of the instrument at the distal end of the instrument such that the bendable portion bends. This is explained in detail in WO2017/213491 (cf. e.g. figures 12, 13a and 13b in that PCT application) of the present applicant.

It will be clear to a person skilled in the art that the scope of the invention is not limited to the examples discussed in the foregoing, but that several amendments and modifications thereof are possible without deviating from the scope of the invention as defined in the attached claims. While the invention has been illustrated and described in detail in the figures and the description, such illustration and description are to be considered illustrative or exemplary only, and not restrictive. The present invention is not limited to the disclosed embodiments but comprises any combination of the disclosed embodiments that can come to an advantage.

Variations to the disclosed embodiments can be understood and effected by a person skilled in the art in practicing the claimed invention, from a study of the figures, the description and the attached claims. In the description and claims, the word "comprising" does not exclude other elements, and the indefinite article "a" or "an" does not exclude a plurality. In fact it is to be construed as meaning "at least one". The mere fact that certain features are recited in mutually different dependent claims does not indicate that a combination of these features cannot be used to advantage. Any reference signs in the claims should not be construed as limiting the scope of the invention. Features of the above described embodiments and aspects can be combined unless their combining results in evident technical conflicts.

## Claims

1. An invasive instrument having a tip section (302) at a distal end, a steering section (306) at a proximal end and a body section (304) between the tip section (302) and the steering section (306),
the invasive instrument comprising one or more locking elements, one or more mechanical switches and one or more actuation elements (728; 730),
the invasive instrument comprising at least one hinge (308) comprising at least a first hinge segment (700(1)) and second hinge segment (700(2)), the first hinge segment (700(1)) and the second hinge segment (700(2)) being deflectable relative to one another and being lockable in a deflected condition of a flexible portion of the invasive instrument by the one or more mechanical switches,
the one or more mechanical switches being implemented as a convex portion (732) in one of the first hinge segment (700(1)) and the second hinge segment (700(2)) and a concave portion (738) in the other one of the first hinge segment and the second hinge segment,
the one or more locking elements (734, 736) each being implemented as a serrated convex portion edge (734) of the convex portion (732) and a serrated concave portion edge (736) of the concave portion (738) facing the serrated convex portion edge (734),
the mechanical switches being configured to move the serrated convex portion edge (734) and the serrated concave portion edge (736) towards one another such that the serrated convex portion edge (734) engages the serrated concave portion edge (736),
the one or more actuation elements (728; 730) running through both the first and second adjacent hinge portions (700(1), 700(2)), being attached to one of the first and second adjacent hinge portions (700(1), 700(2)), and configured to operate the one or more mechanical switches from the proximal end such that they can perform locking one or more locking element in order to freeze a deflected or non-deflected condition of a flexible portion of the invasive instrument, the locking being performed by pulling the one or more actuation elements (728; 730), whereas unlocking is performed by pushing the one or more actuation elements (728; 730) and thus push first and second hinge portions (700(1), 700(2) away from each other in the longitudinal direction,
the one or more locking elements (734, 736), the one or more mechanical switches and one or more actuation elements (728; 730) being portions of a tube (720) or of a plurality of tubes inserted into one another.

2. The invasive instrument according to claim 1, wherein the convex portion (732) is implemented as a T-shaped extension having an outer convex front edge and the concave portion (738) is implemented as a T-shaped opening accommodating the T-shaped extension (732) configured such that the T-shaped extension (732) can rotate inside the T-shaped opening (738).

3. The invasive instrument according to claim 1 or 2, wherein the body section (304) is flexible and the one or more locking elements are configured to freeze a bent or non-bent portion of the body section (304).

4. The invasive instrument according to claim 1, 2 or 3, wherein the first hinge segment (700(1)) and the second hinge segment (700(2)) are portions from a first tube (720) and the one or more locking elements are portions from a second tube arranged inside or outside the first tube.

5. The invasive instrument according to any of the preceding claims, wherein the invasive instrument is either a trocar, a surgical instrument incorporating a trocar to guide a surgical tool, or an endoscope configured to guide zero or more surgical instruments.

6. The invasive instrument according to any of the preceding claims, wherein the tube has or the plurality of tubes have a wall thickness in a range of 0.03-2.0 mm, preferably 0.03-1.0 mm, more preferably 0.05-0.5 mm, and most preferably 0.08-0.4 mm.

7. The invasive instrument according to any of the preceding claims, wherein the tube has or the plurality of tubes have a diameter in a range of 0.5-20 mm, preferably 0.5-10 mm, more preferably 0.5-6 mm.

8. The invasive instrument according to any of the preceding claims, wherein the tube has or the plurality of tubes have a radial play between adjacent tubes in a range of 0.01 - 0.3 mm.

## Patentansprüche

1. Invasives Instrument mit einem Spitzenabschnitt (302) an einem distalen Ende, einem Steuerabschnitt (306) an einem proximalen Ende und einem Körperabschnitt (304) zwischen dem Spitzenabschnitt (302) und dem Steuerabschnitt (306),
das invasive Instrument umfassend ein oder mehrere Verriegelungselemente, einen oder mehrere mechanische Schalter und ein oder mehrere Betätigungselemente (728; 730),
das invasive Instrument umfassend mindestens ein Scharnier (308), das mindestens ein erstes Scharniersegment (700(1)) und ein zweites Scharniersegment (700(2)) umfasst, wobei das erste Scharniersegment (700(1)) und das zweite Scharniersegment (700(2)) relativ zueinander auslenkbar sind und in einem ausgelenkten Zustand eines flexiblen Abschnitts des invasiven Instruments durch den einen oder die mehreren mechanischen Schalter verriegelbar sind,
wobei der eine oder die mehreren mechanischen Schalter als ein konvexer Abschnitt (732) in einem von dem ersten Scharniersegment (700(1)) und dem zweiten Scharniersegment (700(2)) und ein konkaver Abschnitt (738) in dem anderen von dem ersten Scharniersegment und dem zweiten Scharniersegment ausgebildet sind,
wobei das eine oder die mehreren Verriegelungselemente (734, 736) jeweils als eine gezahnte konvexe Abschnittskante (734) des konvexen Abschnitts (732) und eine gezahnte konkave Abschnittskante (736) des konkaven Abschnitts (738), die der gezahnten konvexen Abschnittskante (734) zugewandt ist, ausgebildet sind,
wobei die mechanischen Schalter konfiguriert sind, um die gezackte konvexe Abschnittskante (734) und die gezackte konkave Abschnittskante (736) aufeinander zu zu bewegen, sodass die gezackte konvexe Abschnittskante (734) mit der gezackten konkaven Abschnittskante (736) in Eingriff kommt,
das eine oder die mehreren Betätigungselemente (728; 730), die sowohl durch den ersten als auch den zweiten benachbarten Scharnierabschnitt (700(1), 700(2)) verlaufen, an einem des ersten und zweiten benachbarten Scharnierabschnitts (700(1), 700(2)) befestigt sind und konfiguriert sind, um den einen oder die mehreren mechanischen Schalter vom proximalen Ende aus zu betätigen, sodass sie das Verriegeln eines oder mehrerer Verriegelungselemente durchführen können, um einen abgewinkelten oder nicht abgewinkelten Zustand eines flexiblen Abschnitts des invasiven Instruments zu fixieren, wobei das Verriegeln durch Ziehen an dem einen oder den mehreren Betätigungselementen (728; 730) durchgeführt wird, während das Entriegeln durch Schieben des einen oder der mehreren Betätigungselemente (728; 730) durchgeführt wird und somit der erste und zweite Scharnierabschnitt (700(1), 700(2) in der Längsrichtung voneinander weg geschoben werden,
wobei das eine oder die mehreren Verriegelungselemente (734, 736), der eine oder die mehreren mechanischen Schalter und das eine oder die mehreren Betätigungselemente (728; 730) Abschnitte eines Rohrs (720) oder einer Vielzahl von ineinander eingesetzten Rohren sind.

2. Invasives Instrument nach Anspruch 1, wobei der konvexe Abschnitt (732) als ein T-förmiger Fortsatz mit einer äußeren konvexen Vorderkante ausgeführt ist und der konkave Abschnitt (738) als eine T-förmige Öffnung ausgeführt ist, die den T-förmigen Fortsatz (732) aufnimmt und konfiguriert ist, sodass sich der T-förmige Fortsatz (732) innerhalb der T-förmigen Öffnung (738) drehen kann.

3. Invasives Instrument nach Anspruch 1 oder 2, wobei der Körperabschnitt (304) flexibel ist und das eine oder die mehreren Verriegelungselemente konfiguriert sind, um einen gebogenen oder nicht gebogenen Abschnitt des Körperabschnitts (304) zu arretieren.

4. Invasives Instrument nach Anspruch 1,2 oder 3, wobei das erste Scharniersegment (700(1)) und das zweite Scharniersegment (700(2)) Abschnitte von einem ersten Rohr (720) sind und das eine oder die mehreren Verriegelungselemente Abschnitte von einem zweiten Rohr sind, das innerhalb oder außerhalb des ersten Rohrs angeordnet ist.

5. Invasives Instrument nach einem der vorstehenden Ansprüche, wobei das invasive Instrument entweder ein Trokar, ein chirurgisches Instrument, das einen Trokar zur Führung eines chirurgischen Werkzeugs umfasst, oder ein Endoskop ist, das zur Führung von null oder mehr chirurgischen Instrumenten konfiguriert ist.

6. Invasive Instrument nach einem der vorstehenden Ansprüche, wobei das Rohr oder die Vielzahl von Rohren eine Wandstärke in einem Bereich von 0,03-2,0 mm, vorzugsweise 0,03-1,0 mm, mehr bevorzugt 0,05-0,5 mm und am meisten bevorzugt 0,08-0,4 mm aufweist oder aufweisen.

7. Invasives Instrument nach einem der vorstehenden Ansprüche, wobei das Rohr oder die Vielzahl von Rohren einen Durchmesser in einem Bereich von 0,5-20 mm, vorzugsweise 0,5-10 mm, mehr bevorzugt 0,5-6 mm aufweist oder aufweisen.

8. Invasives Instrument nach einem der vorstehenden Ansprüche, wobei das Rohr oder die Vielzahl von Rohren ein radiales Spiel zwischen benachbarten Rohren in einem Bereich von 0,01-0,3 mm aufweist oder aufweisen.

## Revendications

1. Instrument invasif ayant une section de pointe (302) au niveau d'une extrémité distale, une section d'orientation (306) au niveau d'une extrémité proximale et une section de corps (304) entre la section de pointe (302) et la section d'orientation (306),
l'instrument invasif comprenant un ou plusieurs éléments de verrouillage, un ou plusieurs commutateurs mécaniques et un ou plusieurs éléments d'actionnement (728 ; 730),
l'instrument invasif comprenant au moins une articulation (308) comprenant au moins un premier segment d'articulation (700(1)) et un second segment d'articulation (700(2)), le premier segment d'articulation (700(1)) et le second segment d'articulation (700(2)) pouvant être déviés l'un par rapport à l'autre et étant verrouillable dans une condition déviée d'une partie flexible de l'instrument invasif par le ou les commutateurs mécaniques,
le ou les commutateurs mécaniques étant implémentés en tant que partie convexe (732) dans l'un du premier segment d'articulation (700(1)) et du second segment d'articulation (700(2)) et en tant que partie concave (738) dans l'autre du premier segment d'articulation et du second segment d'articulation,
le ou les éléments de verrouillage (734, 736) étant implémentés chacun en tant que bord dentelé de partie convexe (734) de la partie convexe (732) et en tant que bord dentelé de partie concave (736) de la partie concave (738) faisant face vers le bord dentelé de partie convexe (734),
les commutateurs mécaniques étant conçus pour déplacer le bord dentelé de partie convexe (734) et le bord dentelé de partie concave (736) l'un vers l'autre de telle sorte que le bord dentelé de partie convexe (734) vient en prise avec le bord dentelé de partie concave (736),
le ou les éléments d'actionnement (728 ; 730) s'étendant à travers l'une et l'autre des première et seconde parties d'articulation (700(1), 700(2)) adjacentes, étant fixés à l'une des première et seconde parties d'articulation (700(1), 700(2)) adjacentes, et conçus pour faire fonctionner le ou les commutateurs mécaniques à partir de l'extrémité proximale de telle sorte qu'ils peuvent mettre en œuvre un verrouillage d'un ou plusieurs éléments de verrouillage afin de bloquer une condition déviée ou non déviée d'une partie flexible de l'instrument invasif, un verrouillage étant mis en œuvre en tirant le ou les éléments d'actionnement (728 ; 730), alors qu'un déverrouillage est mis en œuvre en poussant le ou les éléments d'actionnement (728 ; 730) et pour pousser ainsi les première et seconde parties d'articulation (700(1), 700(2) à l'écart l'une de l'autre dans la direction longitudinale,
le ou les éléments de verrouillage (734, 736), le ou les commutateurs mécaniques et un ou plusieurs éléments d'actionnement (728 ; 730) étant des parties d'un tube (720) ou d'une pluralité de tubes insérés les uns dans les autres.

2. Instrument invasif selon la revendication 1, dans lequel la partie convexe (732) est implémentée en tant qu'extension en forme de T ayant un bord avant convexe externe et la partie concave (738) est implémentée en tant qu'ouverture en forme de T recevant l'extension en forme de T (732) conçue de telle sorte que l'extension en forme de T (732) peut effectuer une rotation à l'intérieur de l'ouverture en forme de T (738).

3. Instrument invasif selon la revendication 1 ou 2, dans lequel la section de corps (304) est flexible et le ou les éléments de verrouillage sont conçus pour bloquer une partie cintrée ou non cintrée de la section de corps (304).

4. Instrument invasif selon la revendication 1, 2 ou 3, dans lequel le premier segment d'articulation (700(1)) et le second segment d'articulation (700(2)) sont des parties provenant d'un premier tube (720) et le ou les éléments de verrouillage sont des parties provenant d'un second tube agencé à l'intérieur ou à l'extérieur du premier tube.

5. Instrument invasif selon l'une quelconque des revendications précédentes, dans lequel l'instrument invasif est soit un trocart, soit un instrument chirurgical incorporant un trocart pour guider un outil chirurgical, soit un endoscope conçu pour guider zéro ou plus d'instruments chirurgicaux.

6. Instrument invasif selon l'une quelconque des revendications précédentes, dans lequel le tube a, ou la pluralité de tubes ont, une épaisseur de paroi dans une plage de 0,03 à 2,0 mm, de préférence 0,03 à 1,0 mm, plus préférablement 0,05 à 0,5 mm, et le plus préférablement 0,08 à 0,4 mm.

7. Instrument invasif selon l'une quelconque des revendications précédentes, dans lequel le tube a, ou la pluralité de tubes ont, un diamètre dans une plage de 0,5 à 20 mm, de préférence 0,5 à 10 mm, plus préférablement 0,5 à 6 mm.

8. Instrument invasif selon l'une quelconque des revendications précédentes, dans lequel le tube a, ou la pluralité de tubes ont, un jeu radial entre des tubes adjacents dans une plage de 0,01 à 0,3 mm.
